(19) Europäisches Patentamt / European Patent Office / Office européen des brevets

(11) **EP 2 885 429 B1**

(12) **EUROPEAN PATENT SPECIFICATION**

(45) Date of publication and mention of the grant of the patent:
**13.03.2019 Bulletin 2019/11**

(21) Application number: **13829362.6**

(22) Date of filing: **16.08.2013**

(51) Int Cl.:
*C12Q 1/6886* (2018.01)     *G01N 33/574* (2006.01)

(86) International application number:
**PCT/US2013/055469**

(87) International publication number:
**WO 2014/028907 (20.02.2014 Gazette 2014/08)**

(54) **DIAGNOSTIC MARKERS OF INDOLENT PROSTATE CANCER**

DIAGNOSTISCHE MARKER FÜR INDOLENTEN PROSTATAKREBS

MARQUEURS DIAGNOSTIQUES DU CANCER DE LA PROSTATE INDOLENT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **16.08.2012 US 201261684029 P**
**25.10.2012 US 201261718468 P**
**21.12.2012 US 201261745207 P**

(43) Date of publication of application:
**24.06.2015 Bulletin 2015/26**

(73) Proprietor: **The Trustees of Columbia University in the City of**
**New York**
**New York, NY 10027 (US)**

(72) Inventors:
• **ABATE-SHEN, Corrine**
**New York, NY 10031 (US)**
• **SHEN, Michael**
**New York, NY 10031 (US)**
• **CALIFANO, Andrea**
**New York, NY 10011 (US)**
• **KANTH, Shazia, Irshad**
**Orpington BR6 9QD (GB)**
• **BANSAL, Mukesh**
**New York, NY 10032 (US)**

(74) Representative: **Chapman, Paul Gilmour et al**
**Marks & Clerk LLP**
**40 Torphichen Street**
**Edinburgh EH3 8JB (GB)**

(56) References cited:
**WO-A2-03/053223     WO-A2-2006/110264**
WO-A2-2011/082198     US-A1- 2011 136 683
US-A1- 2011 236 903

• L. A. MUCCI ET AL: "Testing a Multigene Signature of Prostate Cancer Death in the Swedish Watchful Waiting Cohort", CANCER EPIDEMIOLOGY, BIOMARKERS & PREVENTION, vol. 17, no. 7, 1 July 2008 (2008-07-01), pages 1682-1688, XP055076563, ISSN: 1055-9965, DOI: 10.1158/1055-9965.EPI-08-0044
• Marc Overmyer: "Molecular predictor differentiates indolent, advanced prostate cancer", Urology Times, 1 May 2011 (2011-05-01), XP055253866, Retrieved from the Internet: URL:http://urologytimes.modernmedicine.com /urology-times/news/modernmedicine/modern-medicine-news/molecular-predictor-differen tiates-indolent-a?page=full [retrieved on 2016-02-29]
• KARSTEN GRAVDAL ET AL: "Expression of bFGF/FGFR-1 and vascular proliferation related to clinicopathologic features and tumor progress in localized prostate cancer", VIRCHOWS ARCHIV, SPRINGER, BERLIN, DE, vol. 448, no. 1, 1 January 2006 (2006-01-01), pages 68-74, XP019344859, ISSN: 1432-2307, DOI: 10.1007/S00428-005-0075-3
• TROJAN L ET AL: "Identification of metastasis-associated genes in prostate cancer by genetic profiling of human prostate cancer cell lines", ANTICANCER RESEARCH - INTERNATIONAL JOURNAL OF CANCER RESEARCH AND TREATMENT, INTERNATIONAL INSTITUTE OF ANTICANCER RESEARCH, GR, vol. 25, no. 1A, 1 January 2005 (2005-01-01), pages 183-191, XP008113959, ISSN: 0250-7005

EP 2 885 429 B1

- **TONG DAN ET AL: "Gene expression of PMP22 is an independent prognostic factor for disease-free and overall survival in breast cancer patients", BMC CANCER, BIOMED CENTRAL, LONDON, GB, vol. 10, no. 1, 15 December 2010 (2010-12-15), page 682, XP021087199, ISSN: 1471-2407, DOI: 10.1186/1471-2407-10-682**

Description

BACKGROUND OF THE INVENTION

With over 200,000 new diagnoses per year (*1*), prostate cancer is one of the most prevalent forms of cancer in aged men. Several factors, including an increase in the aging population and widespread screening for prostate specific antigen (PSA), have contributed to a substantial rise in diagnoses of early-stage prostate tumors, many of which require no immediate therapeutic intervention (*2-4*). Indeed, the primary means of determining the appropriate treatment course for men diagnosed with prostate cancer still relies on Gleason grading, a histopathological evaluation that lacks a precise molecular correlate (*5*). While patients presenting with biopsies of high Gleason score (Gleason ≥8) tumors are recommended to undergo immediate treatment, determining the appropriate treatment for those with biopsies of low (Gleason 6) or even intermediate (Gleason 7) Gleason score tumors can be more ambiguous.

Currently, there is the potential for overtreatment of patients who have indolent prostate cancer (e.g., low-risk, non-aggressive or non-invasive cancers) who would not have died of the disease if left untreated (*4*, *6-8*). Consequently, the practice of "watchful waiting" (*9*) or, more recently, "active surveillance" (*10-12*) has emerged as an alternative for monitoring men with potentially low risk prostate cancer, with the intention of avoiding treatment unless there is evidence of disease progression. The advantage is to minimize overtreatment; however, the obvious risk is that active surveillance may miss the opportunity for early intervention of tumors that are seemingly low risk but are actually aggressive. Thus, there is a critical need to identify biomarker panels that distinguish the majority of low Gleason score tumors that will remain indolent from the few that are truly aggressive. Unfortunately, so far prostate cancer, unlike many other cancer types, has proven remarkably resilient to classification into molecular subtypes associated with distinct disease outcomes (*13*, *14*). Additionally, an inherent lack of understanding of the biological processes that distinguish indolence from aggressiveness has represented a considerable limitation for identifying relevant biomarkers.

Additional prior art of background relevance includes:

1. L. A. MUCCI ET AL, "Testing a Multigene Signature of Prostate Cancer Death in the Swedish Watchful Waiting Cohort", CANCER EPIDEMIOLOGY, BIOMARKERS & PREVENTION, (20080701), vol. 17, no. 7, doi:10.1158/1055-9965.EPI-08-0044, ISSN 1055-9965, pages 1682 - 1688;

2. Marc Overmyer, "Molecular predictor differentiates indolent, advanced prostate cancer", Urology Times, (20110501), URL: http://urologytimes.modernmedicine.com/urology-times/news/modernmedicine/modern-medicine-news/molecular-predictor-differentiates-indolent-a?page=full, (20160229);

3. WO2011082198;

4. WO2006110264;

5. WO03053223;

6. KARSTEN GRAVDAL ET AL, "Expression of bFGF/FGFR-1 and vascular proliferation related to clinicopathologic features and tumor progress in localized prostate cancer", VIRCHOWS ARCHIV, SPRINGER, BERLIN, DE, (20060101), vol. 448, no. 1, doi:10.1007/S00428-005-0075-3, ISSN 1432-2307, pages 68 - 74;

7. TROJAN L ET AL, "Identification of metastasis-associated genes in prostate cancer by genetic profiling of human prostate cancer cell lines", ANTICANCER RESEARCH - INTERNATIONAL JOURNAL OF CANCER RESEARCH AND TREATMENT, INTERNATIONAL INSTITUTE OF ANTICANCER RESEARCH, GR, (20050101), vol. 25, no. 1A, ISSN 0250-7005, pages 183 - 191;

8. TONG DAN ET AL, "Gene expression of PMP22 is an independent prognostic factor for disease-free and overall survival in breast cancer patients", BMC CANCER, BIOMED CENTRAL, LONDON, GB, (20101215), vol. 10, no. 1, doi:10.1186/1471-2407-10-682, ISSN 1471-2407, page 682.

SUMMARY OF THE INVENTION

**The present invention is defined in accordance with the claims which are appended hereto. Any of the following description which is not directed to the claims as appended hereto is provided by way of background or for reference purposes.**

Certain embodiments are directed to methods for determining if a prostate cancer is at a high risk of progressing to an aggressive cancer. More specifically, the method may comprise (a) identifying a subject having prostate cancer, (b) providing a test biological sample of the prostate cancer from the subject and a control sample of benign noncancerous prostate tissue from the subject or from a normal subject, (c) detecting a level of expression of a prognostic mRNA or protein encoded by each of three prognostic genes selected from the group consisting of *FGFR1, PMP22,* and *CDKN1A* in the test sample, as compared to the level of expression in the control sample, and (d) if the level of expression of the mRNA or a protein or both is the same or higher than the corresponding level in the control, then determining that the prostate cancer is indolent, and if there is about a two-fold or greater decrease in the level of expression of the mRNA or protein compared to the control then determining that the prostate cancer is at high risk of progressing to an aggressive form. In some embodiments the prostate cancer has a Gleason score of 7 or lessThe subject may be treated if it is determined that the prostate cancer is at a high risk of progressing toward an aggressive form. the biological sample is blood, plasma, urine or cerebrospinal fluid

Another embodiment is directed to a method for determining if a subject who has a prostatecancer has progressed or is progressing to an aggressive form of cancer by (a) identifying a subject having prostatecancer, (b) providing a first biological sample of the prostate cancer from the subject at a first time point and a second biological sample at a second time point; (c) determining a level of expression of a prognostic mRNA or protein or both encoded by each of three prognostic genes selected from the group consisting of *FGFR1, PMP22,* and *CDKN1A* in the first and second samples at the respective first and second time points, (d) comparing the expression levels of the prognostic mRNA or protein at the first time point to the expression levels at the second time point, and (e) determining that the prostate cancer is not progressing to an aggressive form if the level of expression of the prognostic mRNA or the protein or both at the second time point is the same or greater than at the first time point, and determining that the indolent cancer is at a high risk of progressing toward an aggressive form if there is about a two-fold or greater decrease in the level of expression of the prognostic mRNA or a protein at the second time point compared to the levels at the first time point. The subject may be treated if it is determined that the prostate cancer is at a high risk of progressing toward an aggressive form.

Other teachings are directed to various diagnostic kits for detecting the expression levels of a prognostic mRNA or a protein encoded or both by each of three prognostic genes selected from the group consisting of *FGFR1*, *PMP22,* and *CDKN1A* in a biological sample, the kit comprising oligonucleotides that specifically hybridize to each of the respective mRNAs or one or more agents that specifically bind to each of the respective proteins, or both, optionally having a forward primer and a reverse primer specific for each mRNA encoded by each of the prognostic genes for use n a qRT-PCR assay to specifically quantify the expression level of each mRNA. In another embodiment this diagnostic further includes one or more antibodies or antibody fragments that specifically bind to each of the respective proteins.

Other teachings are directed to a-microarray comprising a plurality of oligonucleotides that specifically hybridize to an mRNA encoded by each of three prognostic genes selected from the group consisting of *FGFR1*, *PMP22*, and *CDKN1A*, which cDNAs or oligonucleotides are fixed on the microarray; in which the oligonucleotides are optionally labeled to facilitate detection of hybridization to the mRNAs. In some embodiments the oligonucleotides are RNA or DNAs. In other embodiments the microarrays have a plurality of antibodies or antibody fragments that specifically bind to a prognostic protein or variant or fragment thereof encoded by each of three prognostic genes selected from the group consisting of *FGFR1*, *PMP22*, and *CDKN1A*, which antibodies or antibody fragments are fixed on the microarray. An immunoassay for detecting whether prostate cancer in a biological sample taken for a subject is indolent or is at high risk of progressing to an aggressive form, wherein the immunoassay comprises a plurality of antibodies or antibody fragments that specifically bind to prognostic proteins encoded by each of three prognostic genes selected from the group consisting of *FGFR1, PMP22,* and *CDKN1A*.

Another teaching is directed to the method where determining expression level of a prognostic protein comprises immunohistochemistry using one or more antibodies or fragments thereof that *specifically* binds to the proteins or Western Blot. In some teachings mRNA expression is quantified by qRT-PCR.

**BRIEF DESCRIPTION OF THE DRAWINGS**

The present invention is illustrated by way of example, and not by way of limitation, in the figures of the accompanying drawings.

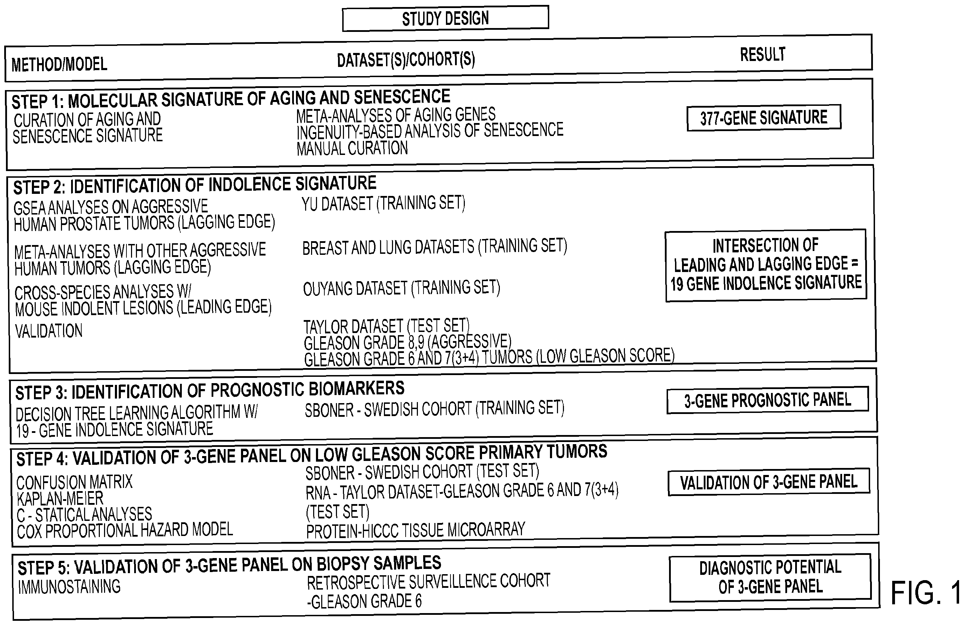

**Figure 1: Study design**

Step 1: Assembly of a 377-gene signature enriched for cellular processes associated with aging and senescence (Table 1).

Step 2: Gene set enrichment analyses (GSEA) using the 377-gene signature to query: (i) aggressive human prostate tumors from Yu et al. *(ii)* aggressive cancers from lung and breast followed by meta-analyses with the human prostate dataset. *(iii)* cross-species analysis on indolent mouse prostate lesions from Ouyang et al. The intersection of the *leading* edge from mouse prostate lesions and the *lagging* edge from the meta-analyses of human aggressive cancers led to identification of 19-gene "indolence signature" (Table 5). The indolence signature was validated on human prostate tumors from Taylor et al.

Step 3: Decision tree-learning to classify the 19-gene indolence signature to identify a 3-gene prognostic panel of indolent prostate cancer using Sboner et al.

Step 4: Validation of the 3-gene prognostic panel at the mRNA and protein levels.

Step 5: Validation of the 3-gene prognostic panel on biopsies from Gleason Grade 6 patients.

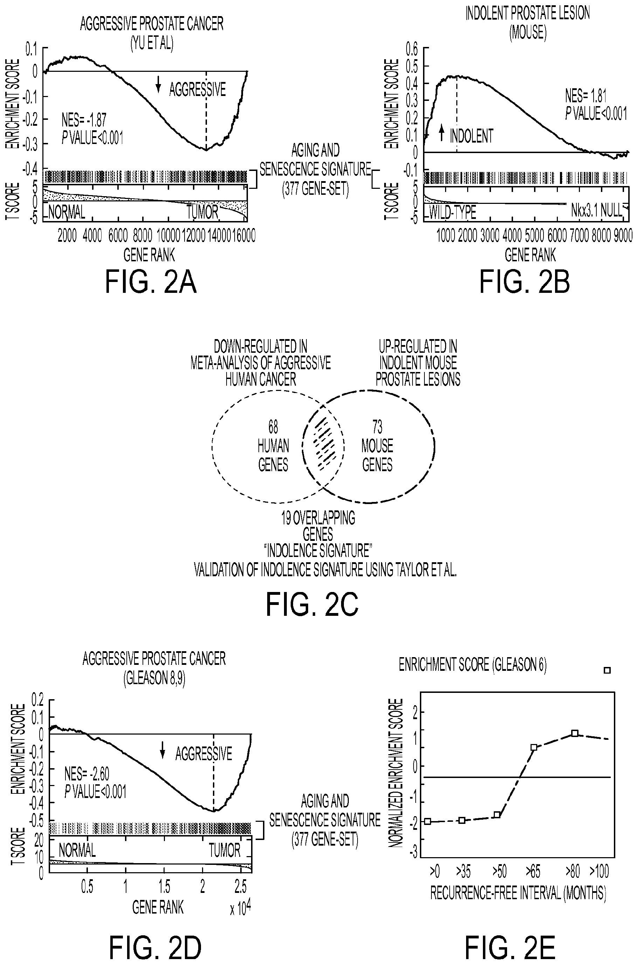

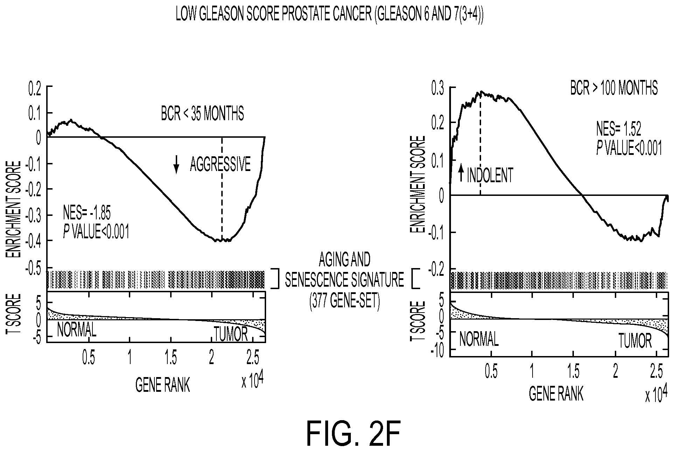

**Figure 2: A gene signature of aging and senescence stratifies human prostate cancer** (A-C) Identification of an indolence signature: (A, B) GSEA analyses using the 377-gene signature to query expression profiles from aggressive prostate tumors (*in A;* from Yu et al.) and mouse indolent prostate cancer (*in B;* from Ouyang et al.). (C) Intersection from the *lagging* edge in the meta-analyses of aggressive tumors and the *leading* edge in the mouse indolent lesions to identify the 19-gene indolence signature. (D-F) Validation of an indolence signature: (D, F) GSEA analyses on aggressive (*i.e.,* Gleason score 8,9) or low Gleason score (Gleason score 6 and 7(3 + 4)) prostate tumors Taylor et al. separated by short time to biochemical recurrence (BCR < 35 months; n = 5) or a long time with no evidence of recurrence (BCR > 100 months; n = 5). (E) Summary of the enrichment score from GSEA analyses done on all Gleason 6 prostate tumors (n = 44) partitioned by interval free of biochemical recurrence. Leading and lagging edge genes from each of GSEA plot are provided in Table 3; genes in indolence signature are provided in Table 5.

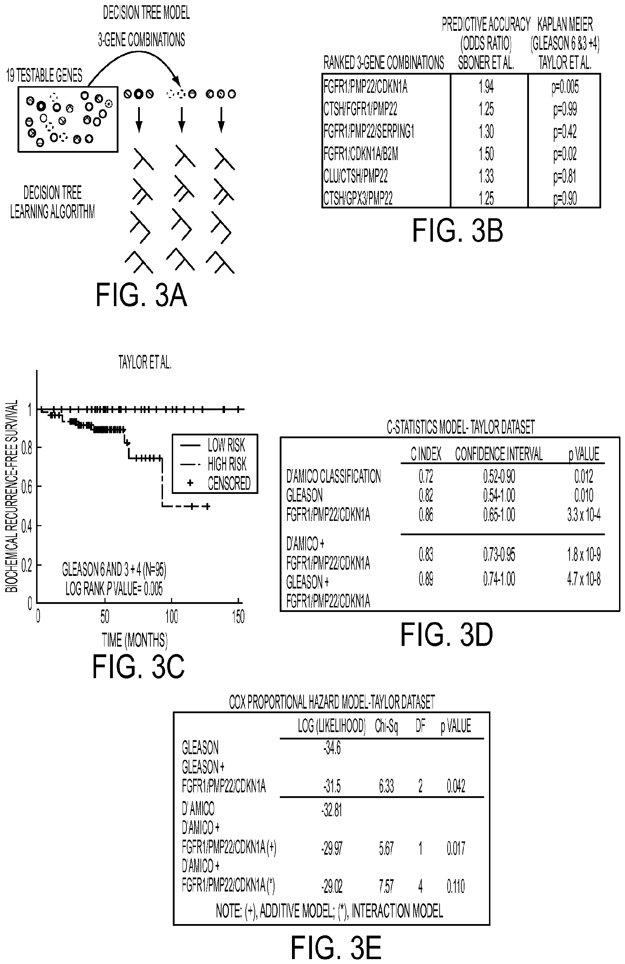

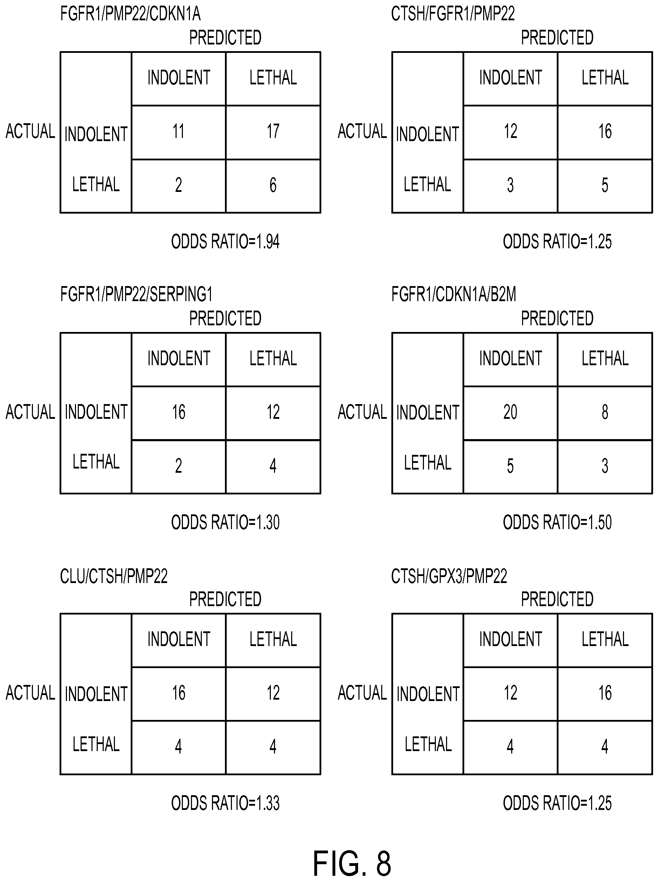

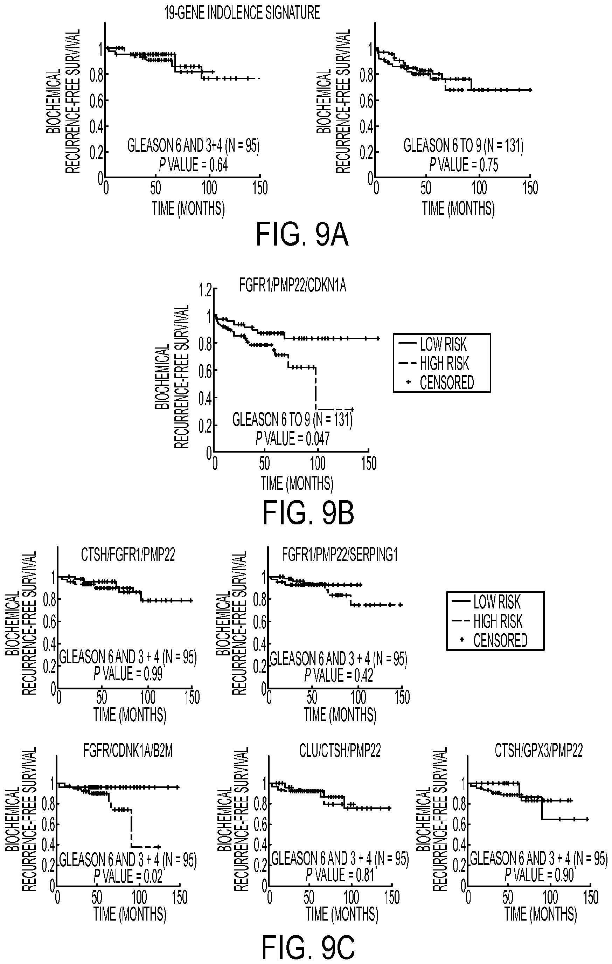

**Figure 3: A decision tree-learning model identifies a 3-gene prognostic panel** (A) Schematic representation of the decision tree-learning model. The decision tree algorithm systematically samples the expression states of all combinations of the 19-gene indolence signature to identify combinations most effective in segregating patients into indolent and lethal groups. The decision tree-learning model was performed using Sboner et al (Table 22). (B) Summary of the top 3-gene combinations from the decision tree-learning model. The first column shows combinations ranked by cross validation error (Table 6). The next two columns show independent validation using: (*1*) the odds ratio for each of the 3-gene combinations to accurately predict patient outcome (i.e., indolence or lethality) using confusion matrices (Figure 8); and (*2*) Kaplan Meier analyses of low Gleason score patients using the Taylor dataset; log-rank *p* values are summarized here and Kaplan Meier plots shown in Panel C and Figure 9. (C) Kaplan-Meier analysis of patients with low Gleason score (Gleason 6 and 7(3+4); n = 95) from Taylor et al. showing stratification of *FGFR1, PMP22,* and *CDKN1A* for fast-recurring versus slow-recurring patients. The Log-Rank *p* value is indicated. (D, E) C-statistical analysis and Cox proportional hazard model on Gleason 6 and 7(3+4) patients comparing the performance of *FGFR1, PMP22 and CDKN1A* expression levels with the D'Amico classification or with Gleason score alone.

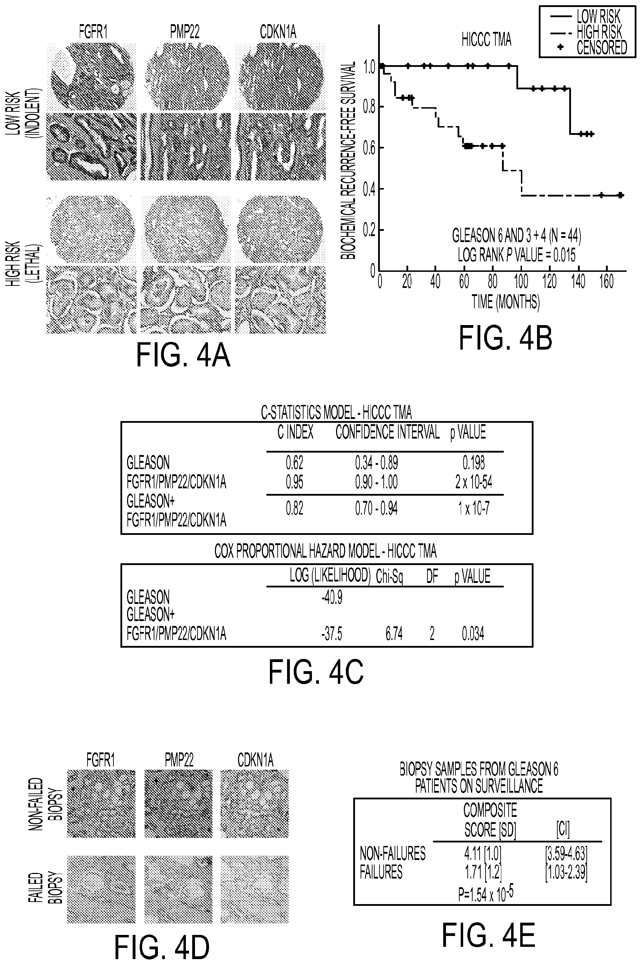

**Figure 4: Predictive accuracy of the 3-gene predictive panel at the protein expression level** (A) Analyses of a tissue microarrays immunostained for FGFR1, PMP22 and CDKN1A showing representative cases of Gleason grade 6 tumors that were indolent or lethal. (B) Kaplan-Meier analysis for patients with Gleason 6 and 7(3+4) included in the TMA (n = 44) separated into high-risk versus low-risk cancers by protein expression of FGFR1, PMP22 and CDKN1A can. The Log-Rank *P* value is indicated. (C) C-statistical analysis and Cox proportional hazard models for Gleason 6 and 7(3 + 4) patients from the TMA comparing the performance of protein expression levels of FGFR1, PMP22 and CDKN1A with Gleason score. (D) Representative immunohistochemical results from the "non-failed" and "failed" biopsy groups of Gleason 6 patients monitored by surveillance (see Table 1) showing expression levels of FGFR1, PMP22 and CDKN1A. (E) Summary of analyses of initial biopsy samples using all the "failed" cases (n = 14) in the cohort compared to non-failed cases (n = 19) and validated with a second group of non-failed cases (n = 10).

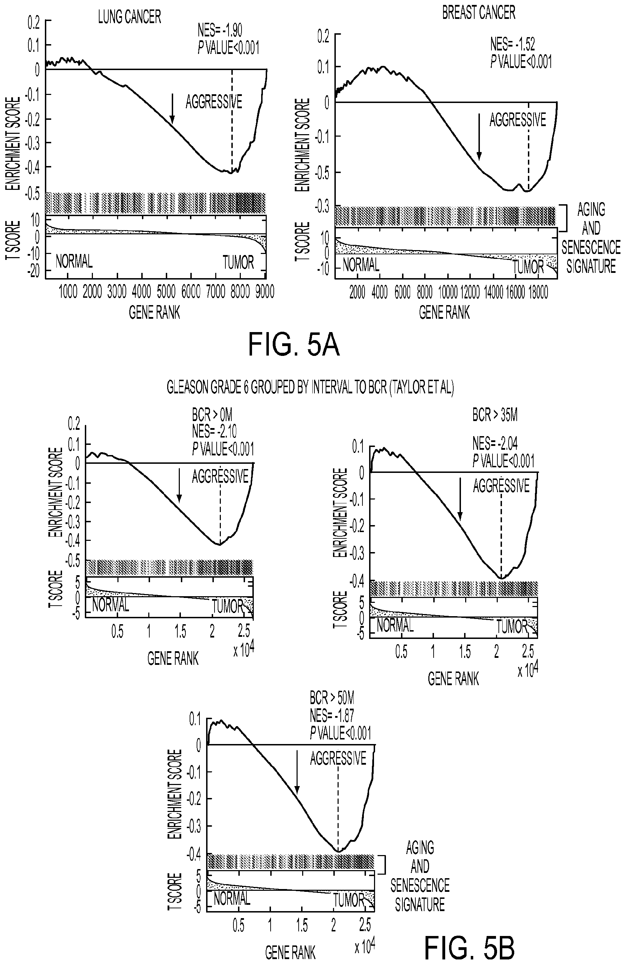

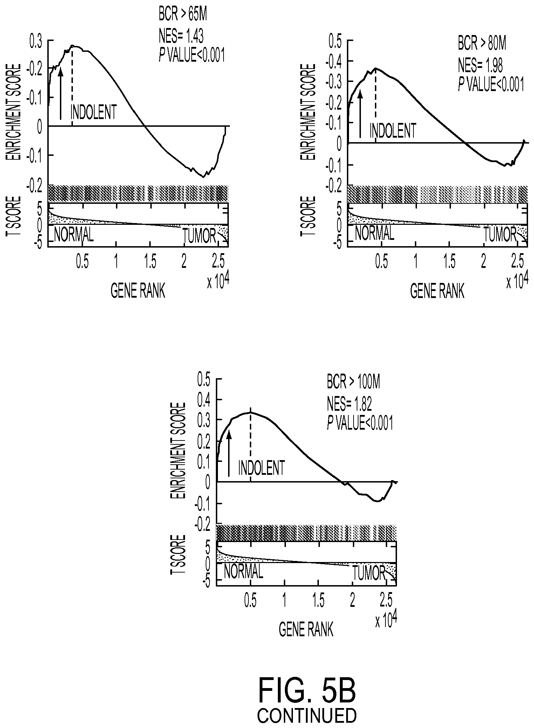

**Figure 5: Supplementary GSEA data for human cancer** (A) GSEA analyses showing results using the 377 gene-set of aging and senescence to query gene expression data from a lung and breast cancer dataset. (B) GSEA analyses using the 377 gene-set of aging and senescence to query gene expression data from Gleason grade 6 cancers from Taylor et al. partitioned according to time to biochemical recurrence. Leading and lagging edge genes are listed in Table 3.

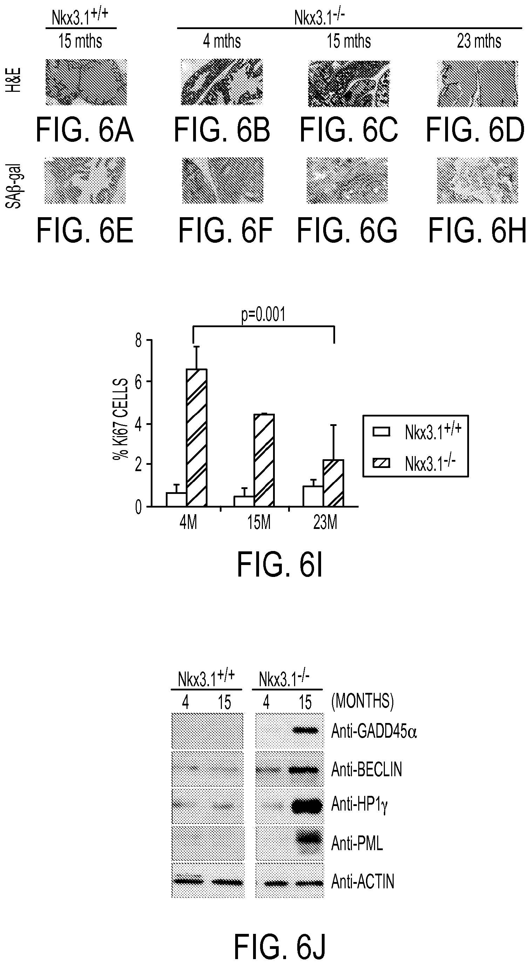

**Figure 6: Phenotypic analysis of a mouse model of indolent prostate cancer** A-D. Representative H&E images the anterior prostate of *Nkx3.1* null mutant mice at the indicated ages. Note that the mice develop prostatic intraepithelial neoplasia (PIN) by 15 months of age. **E-H.** Analyses of senescence associated β-galactasidase (SA β-gal) activity in the mouse prostate tissues. **I.** Summary of proliferation rate in the mouse prostate tissues as measured by expression of Ki67 staining. **J.** Western blot analyses of mouse prostate tissues for analyses of growth arrest (Gadd45alpha), autophagy (Beclin) and senescence-associated (HP1gamma and PML) proteins using the indicated antibodies.

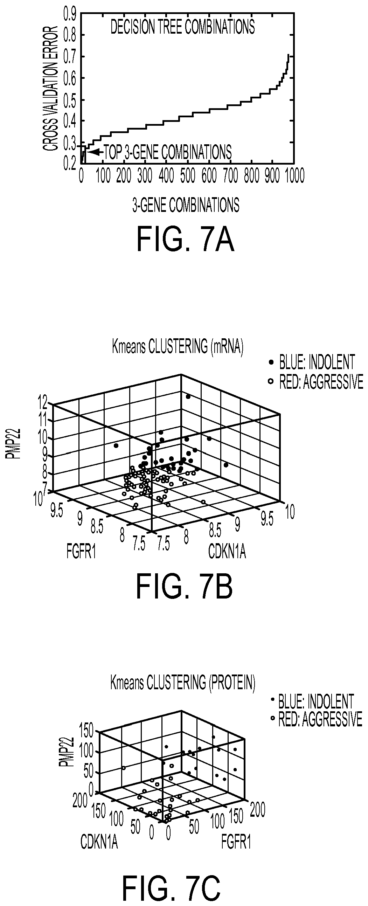

**Figure 7: Supplementary data for the decision tree-learning model and K-means clustering** (A) Summary of the range of cross validation error for all possible 3-gene combinations identified from the decision tree-learning model. A list of 3-gene combinations from the decision tree-learning model ranked by their cross validation error is provided in 6. (B) K-means clustering analyses showing fast-recurring (aggressive, red) and slow-recurring (indolent; blue) Gleason grade 6 and 7(3+4) prostate tumors from Taylor et al segregated by expression levels of *FGFR1, PMP22* and *CDKN1A.* (C) K-means clustering analyses showing segregation of predicted aggressive (red) and indolent (blue) patients from the Gleason grade 6 and 7(3 + 4) prostate tumors from the TMA by protein expression levels of FGFR1, PMP22 and CDKN1A.

**Figure 8: Confusion matrices for top-ranked 3-gene combinations from the decision tree-learning model** Confusion matrices showing the predicted versus actual calls for indolence versus lethality for the indicated 3-gene combinations using the gene expression and clinical outcome data from Sboner et al. (N=8 lethal and N=28 indolent); only cases excluded the training set used for the decision tree analyses (Table 2D). Odds ratios indicate the predictive accuracy for each 3-gene combination.

**Figure 9: Supplementary Kaplan-Meier analyses comparing the 19-gene indolence signature and the top 3-gene combinations from the decision tree-learning model** Kaplan Meier analyses were calculated using gene expression values in K-means clustering and correlated to clinical outcome data provided in the Taylor dataset using all Gleason Grade primary tumors (n = 131) or only the Gleason 6 and 7(3 + 4) (n = 95) patients as indicated.

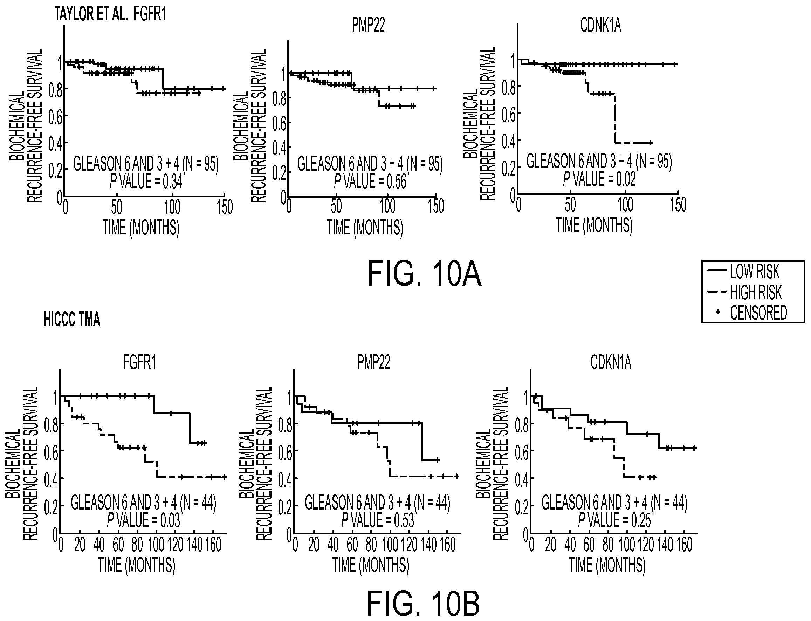

**Figure 10: Supplementary Kaplan-Meier analyses for the single genes in the 3-gene prognostic panel** Kaplan Meier analyses were calculated using gene expression values in K-means clustering and correlated to clinical outcome data provided in (A) the Taylor dataset using the Gleason 6 and 7(3 + 4) (n = 95) patients and in (B) the HICCC TMA using the Gleason 6 and 7(3 + 4) (n = 44) patients.

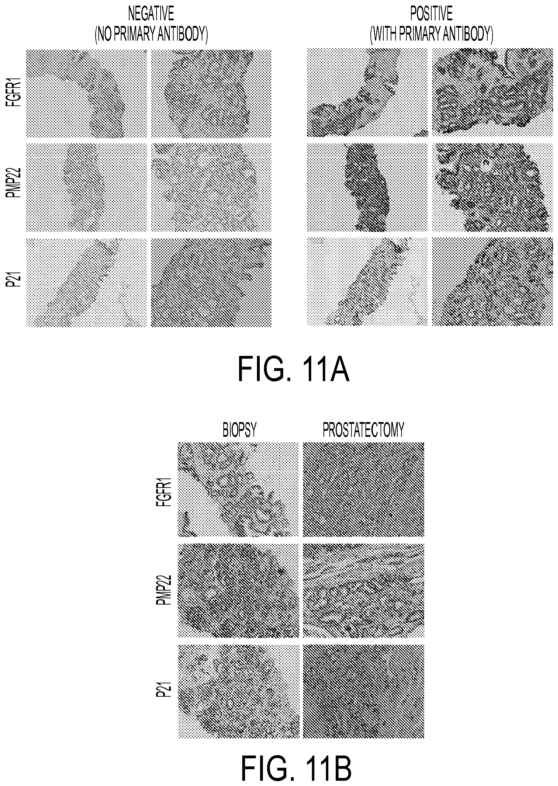

**Figure 11: Immunostaining of 3-gene prognostic panel comparing biopsies and primary tumors** A. Negative and positive controls for immunostaining with each antibody on biopsy samples showing low and high power images. B. Controls showing analogous staining on biopsy and whole prostate tissue.

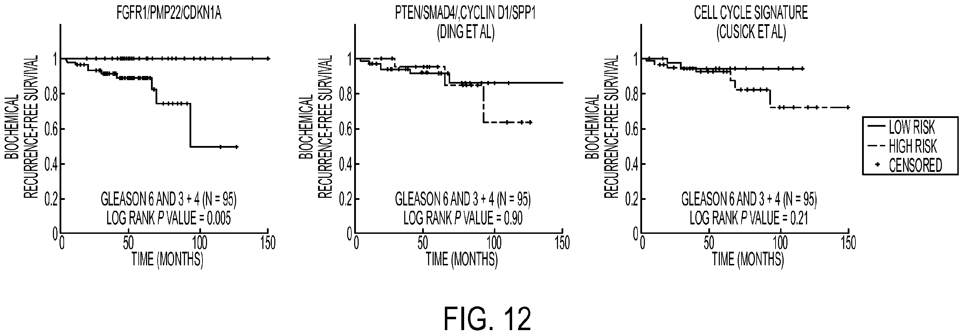

**Figure 12:** Kaplan-Meier analyses comparing the 3-gene prognostic panel with biomarkers from Ding et al. and Cuzick et al. Kaplan Meier analyses were calculated using gene expression values in K-means clustering and correlated to clinical outcome data provided in the Taylor dataset using the Gleason 6 and 7(3 + 4) (n = 95) patients.

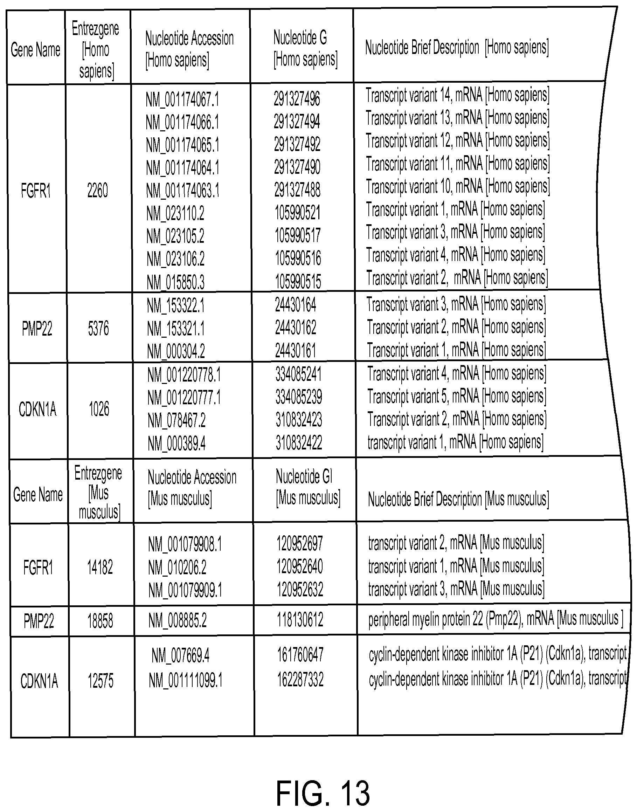

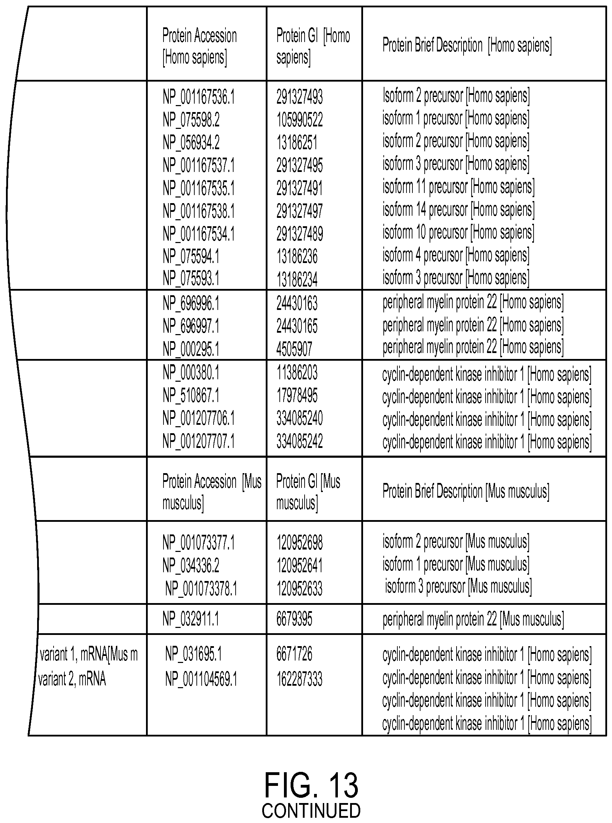

**Figure 13:** Provides the sequence information for certain genes, protein, and mRNAs, which are all publically available.

**TABLES**

**Table 1:** Description of the 377 gene-set of aging and senescence

**Table 2:** Description of patient samples used in this study

    A. Yu et al. Training set
    B. Taylor et al. Test set
    C. Sboner et al., Training set
    D. Sboner et al., Test set

**Table 3:** Leading/lagging edge genes from the GSEA analyses

A. Yu et al (human prostate cancer) lagging edge genes, Figure 2A.
B. Lung cancer (human) lagging edge genes, Figure 5A
C. Breast Cancer (human) lagging edge genes, Figure 51A
D. Ouyang et al (mouse) leading edge genes, Figure 2B
E. Taylor et al (human) Gleason grade 6 and 7(3 + 4) (BCR < 35) lagging edge genes, Figure 2F
F. Taylor et al (human) Gleason grade 6 and 7(3 + 4) (BCR > 100) leading edge genes, Figure 2F

**Table 4:** Integrative analyses of the 377 gene-set

A. Meta-analyses of human prostate, lung and breast, Integrative analyses of extreme Gleason Grade 6 groups from Taylor et al
B. Integrative analyses of all Gleason Score 6 patients from Taylor et al. Fig. 5b.

**Table 5:** Description of the 19-gene indolence signature

**Table 6:** 3-gene combinations from the decision tree learning model

A. Top 3-gene combinations with 25% cross-validation error
B. All 3-gene combinations

## DETAILED DESCRIPTION

Definitions

Unless defined otherwise, all technical and scientific terms used herein have the same meaning as commonly understood by one of ordinary skill in the art to which this invention belongs. Although any methods and materials similar or equivalent to those described herein can be used in the practice or testing of the invention, the preferred methods and materials are now described.

Generally, nomenclatures used in connection with, and techniques of, cell and tissue culture, molecular biology, immunology, microbiology, genetics, protein, and nucleic acid chemistry and hybridization described herein are those well-known and commonly used in the art. The methods and techniques of the present invention are generally performed according to conventional methods well known in the art and as described in various general and more specific references that are cited and discussed throughout the present specification unless otherwise indicated. *See*, *e.g.*, Sambrook et al. Molecular Cloning: A Laboratory Manual, 2d ed., Cold Spring Harbor Laboratory Press, Cold Spring Harbor, N.Y. (1989); Ausubel et al., Current Protocols in Molecular Biology, Greene Publishing Associates (1992, and Supplements to 2002); Harlow and Lan, Antibodies: A Laboratory Manual, Cold Spring Harbor Laboratory Press, Cold Spring Harbor, N.Y. (1990); Principles of Neural Science, 4th ed., Eric R. Kandel, James H. Schwart, Thomas M. Jessell editors. McGraw-Hill/Appleton & Lange: New York, N. Y. (2000). Unless defined otherwise, all technical and scientific terms used herein have the same meaning as commonly understood by one of ordinary skill in the art.

The following terms as used herein have the corresponding meanings given here. Unless defined otherwise, all technical and scientific terms used herein have the same meaning as commonly understood by one of ordinary skill in the art to which this invention belongs. Although any methods and materials similar or equivalent to those described herein can be used in the practice or testing of the invention, the example methods and materials are now described, including the currently preferred embodiments.

"Biological sample" refers to a body sample in which the prognostic biomarkers can be detected. In some embodiments, the sample refers to biopsy tissues collected from an individual having epithelial cancer and to benign or noncancerous control tissue from the subject or a normal control. In other embodiments the biological sample is urine, blood, csf or any other tissue where the prognostic protein and mRNA biomarkers can be detected. Biological samples of cancerous cells can also come from urine of the subject, and the prognostic biomarker mRNA and protein can be found in blood, plasma and cerebrospinal fluid.

"Indolent, or low-risk, or non-aggressive or non-invasive cancer" means cancer that is unlikely to become symptomatic during life.

"Aggressive cancer" means prostate cancer or other epithelial cancer that is symptomatic and likely to be lethal. For prostate cancer, aggressive forms typically have a Gleason score $\geq 8$.

"High Gleason score " means a Gleason score $\geq 8$ on the prostate cancer biopsy. Such patients are recommended to undergo immediate treatment.

"Intermediate Gleason score" means a Gleason score $\geq 7$ on the prostate cancer biopsy.

"Low Gleason score" means a Gleason score less than or equal to 6 on the prostate cancer biopsy.

"At High Risk of Progressing to Aggressive Prostate Cancer" means prostate cancer that is not indolent as is determined by a two-fold decrease in expression of mRNA or protein encoded by the 3-gene prognostic panel compared to normal controls.

"3-gene prognostic panel" means the genes: FGFR1, PMP22 and CDKN1A, the simultaneous expression of which identifies prostate cancer tumors that are indolent as opposed to at risk of becoming aggressive.

"Proteins encoded by the 3 gene prognostic panel" and "prognostic biomarker proteins" are used interchangeably and mean the proteins encoded by the 3-gene prognostic panel and their variants and fragments.

"mRNA encoded by the 3 gene prognostic panel" means mRNA transcribed from each of the genes in the 3 gene panel, which mRNAs are translated the prognostic biomarker proteins.

"Prognostic biomarker mRNA" means mRNA encoded by the genes in the 3 gene prognostic panel.

"Detect" "detection" or "detecting" refer to the quantification of a given prognostic biomarker mRNA or protein.

"Treatment" includes any process, action, application, therapy, or the like, wherein a subject (or patient), including a human being, is provided medical aid with the object of improving the subject's condition, directly or indirectly, or slowing the progression of a condition or disorder in the subject, or ameliorating at least one symptom of the disease or disorder under treatment.

"Indolence signature" means a group of 19 "PCIG" genes associated with cellular processes of aging and senescence that are enriched in indolent prostate tumors identified using Gene Set Enrichment Analysis (GSEA). The 19 genes are either enriched in *down-regulated* in aggressive human prostate cancer or conversely *up-regulated* in indolent prostate cancer (*i.e.,* the leading edge).

"PCIG" is an abbreviation for "Prostate Cancer Indolence Genes" (used interchangeably) and refers to any single one or any combination of the following 19 genes: B2M, CAT, CDKN1A, CFH, CLIC4, CLU, CTSH, CX3CL1, FGFR1, GPX3, IGF1, ITM2A, LGALS3, MECP2, MSN, NFE2L2, PMP22, SERPING1, TXNIP: which genes are spelled out below. Beta-2 microglobulin (B2M), Cyclin-dependent kinase inhibitor 1A (p21 or Cip1) (CDKN1A), Chloride intracellular channel 4 (CLIC4), Clusterin (CLU), Cathepsin H (CTSH), Chemokine (C-X3-C motif) ligand 1 (CX3CL1), Fibroblast growth factor receptor 1 (FGFR1), Glutathione peroxidase 3 (plasma) (GPX3), Insulin-like growth factor 1 (somatomedin C) (IGF1), integral membrane protein 2A (ITM2A), Lectin, galactose-binding, soluble, 3 (LGALS3), Methyl CpG binding protein 2 (Rett syndrome) (MECP2), Moesin (MSN), Nuclear factor (erythroid-derived 2)-like 2 (NFE2L2), Peripheral myelin protein 22 (PMP22), Serpin peptidase inhibitor, clade G (C1 inhibitor), member 1 (SERPING1) and Thioredoxin interacting protein (TXNIP).

The term "probe" refers to any molecule which is capable of selectively binding to a specifically intended target molecule, for example, an oligonucleotide probe that specifically hybridizes to a prognostic biomarker mRNA, or an antibody that specifically binds a biomarker protein encoded by the 3 gene prognostic panel. Probes can be either synthesized by one skilled in the art, or derived from appropriate biological preparations. For purposes of detection of the target molecule, probes may be specifically designed to be labeled, as described herein. Examples of molecules that can be utilized as probes include, but are not limited to, RNA, DNA, proteins, antibodies, and organic molecules.

"Epithelial, prostate, breast and lung cancer" refer to a cancerous tumor. For purposes of this application, cancer is restricted to prostate cancer.

As used herein, the term "expression level" refers to expression of protein as measured quantitatively by methods such as Western blot, immunohistochemistry or ELISA and expression of mRNA encoding the three prognostic biomarkers as measured quantitatively by methods including but not limited to, for example, qRT-PCR. Methods for quantifying expression levels of mRNA are further described below in references.

As used herein, the term "detect an expression level" refers to measuring or quantifying either protein expression or mRNA expression.

"An increased or decreased expression level" refers to increased or decreased protein expression level or mRNA expression level relative to a normal or control value. For purposes of this application, increased or decreased protein or mRNA expression refers to expression in the cancerous biological sample compared to either the corresponding level in a control subject (free of cancer) or in normal tissue adjacent to the cancer.

Unless otherwise specified, the terms "antibody" and "antibodies" broadly encompass naturally-occurring forms of antibodies (e.g., IgG, IgA, IgM, IgE) and recombinant antibodies such as single-chain antibodies, chimeric and humanized antibodies and multi-specific antibodies, as well as fragments and derivatives of all of the foregoing, which fragments and derivatives have at least an antigenic binding site. Antibody derivatives may comprise a protein or chemical moiety conjugated to an antibody moiety.

An "isolated" nucleic acid molecule is one which is separated from other nucleic acid molecules which are present in the natural source of the nucleic acid molecule, namely cancerous or noncancerous biological samples. Preferably, an "isolated" nucleic acid molecule is free of sequences (preferably protein-encoding sequences) which naturally flank the nucleic acid (i.e., sequences located at the 5' and 3' ends of the nucleic acid) in the genomic DNA of the organism from which the nucleic acid is derived. Moreover, an "isolated" nucleic acid molecule, such as a cDNA molecule, can be substantially free of other cellular material or culture medium when produced by recombinant techniques, or substantially free of chemical precursors or other chemicals when chemically synthesized. All prognostic biomarkers and mRNA in the present embodiments are isolated.

As used herein, the term "about" is used to mean approximately, roughly, around, or in the region of. When the term "about" is used in conjunction with a numerical range, it modifies that range by extending the boundaries above and below the numerical values set forth. In general, the term "about" is used herein to modify a numerical value above and below the stated value by a variance of 20 percent up or down (higher or lower).

*Summary of Results*

Many newly diagnosed prostate cancers present as low (G 6 or less) or high (G 8 or higher) Gleason score tumors that require no treatment intervention. However, distinguishing the many indolent tumors from the minority of lethal ones remains a major clinical challenge. It has now been discovered that Gleason score 7 or less prostate tumors can be distinguished as truly indolent or aggressive subgroups based on their expression of a 3-gene prognostic panel: *FGFR1, PMP22,* and *CDKN1A.* The embodiments described here also apply to epithelial tumor classification and prognosis, including lung and breast cancers.

One of the most significant risk factors associated with prostate cancer is aging (*13*), which represents a balance of anti- tumorigenic and pro-tumorigenic signals. One of the principal anti-tumorigenic signals is cellular senescence (*15-18*). Indeed, it is now widely appreciated that senescence plays a critical role in tumor suppression in general, and has been associated with benign prostate lesions in humans (*19, 20*), as well as mouse models (*21*). Thus, it was hypothesized that prostate tumors destined to remain indolent versus aggressive could be distinguished based on their enrichment for cellular processes associated with aging and senescence.

1. To test the hypothesis, a bioinformatics approach was used to identify a 19-gene group (hereafter "an indolence signature" See Table 5) that is enriched in indolent prostate tumors compared to aggressive tumors was identified using Gene Set Enrichment Analysis.

2. The 19-gene group indolence signature was further classified using a decision tree learning model leading to the identification of a 3-gene prognostic panel: *FGFR1, PMP22,* and *CDKN1A,* which together accurately predicted the outcome of low Gleason score tumors as either truly indolent or at a high risk of becoming aggressive. Validation of this 3-gene prognostic panel on independent cohorts confirmed its independent prognostic value, as well as its ability to improve prognosis with currently used clinical nomograms. Expression of the 3-gene prognostic panel was determined by quantifying mRNA or protein encoded by the panel (collectively referred to as "prognostic biomarkers"). The prognostic biomarkers were discovered to be *up-regulated* in indolent tumors and down-regulated in aggressive forms of prostate cancer (Fig. 1).

### 19 "PCIG" gene Indolence Panel also the Indolence Signature

| Entrez ID | Gene Symbol | Hyperlink | Gene Description |
|---|---|---|---|
| 567 | B2M | B2M | beta-2-microglobulin |
| 847 | CAT | CAT | catalase |
| 1026 | CDKN1A | CDKN1A | cyclin-dependent kinase inhibitor 1A (p21, Cip1) |
| 3075 | CFH | CFH | complement factor H |
| 25932 | CLIC4 | CLIC4 | chloride intracellular channel 4 |
| 1191 | CLU | CLU | clusterin |
| 1512 | CTSH | CTSH | cathepsin H |
| 6376 | CX3CL1 | CX3CL1 | chemokine (C-X3-C motif) ligand 1 |
| 2260 | FGFR1 | FGFR1 | fibroblast growth factor receptor 1 |
| 2878 | GPX3 | GPX3 | glutathione peroxidase 3 (plasma) |
| 3479 | IGF1 | IGF1 | insulin-like growth factor 1 (somatomedin C) |
| 9452 | ITM2A | ITM2A | integral membrane protein 2A |
| 3958 | LGALS3 | LGALS3 | lectin, galactoside-binding, soluble, 3 |
| 4204 | MECP2 | MECP2 | methyl CpG binding protein 2 (Rett syndrome) |
| 4478 | MSN | MSN | moesin |

(continued)

| Entrez ID | Gene Symbol | Hyperlink | Gene Description |
|---|---|---|---|
| 4780 | NFE2L2 | NFE2L2 | nuclear factor (erythroid-derived 2)-like 2 |
| 5376 | PMP22 | PMP22 | peripheral myelin protein 22 |
| 710 | SERPING1 | SERPING1 | serpin peptidase inhibitor, clade G (C1 inhibitor), member 1 |
| 10628 | TXNIP | TXNIP | thioredoxin interacting protein |

3. In various embodiments, the level of expression of the prognostic biomarkers in biopsy samples is used to identify and distinguish truly indolent forms of prostate cancer (and other epithelial cancers) from aggressive forms. In particular it has been discovered that prostate cancer from Gleason 7 or less patients will not progress to malignant disease if their prostate cancers show normal or elevated levels of expression of the 3-gene prognostic panel (mRNA or protein) compared to benign or noncancerous prostate tissue can be identified as indolent prostate cancer. By contrast, prostate cancer of Gleason 7 or less that shows significantly reduced levels (about a 2-fold reduction) of expression of the 3-gene prognostic panel compared to benign or noncancerous prostate tissue can be identified as aggressive Prostate cancer. Other embodiments are directed to the same methods as applied to epithelial cancers generally, and lung and breast cancers specifically.

4. The prognostic accuracy expression of this 3-gene panel was tested on biopsies from patients monitored by active surveillance and therefore has clinical utility. A previously identified 4-gene signature of aggressive tumors that includes *Pten*, *Smad4*, *Cyclin D1* and *SPP1*, do not overlap with the present new 3-gene panel of indolence. Notably, this 4-gene biomarker panel, which was identified on the basis of its ability to stratify advanced prostate tumors, was not effective for stratifying low Gleason score prostate tumors (Fig. 12).

5. Lung and breast cancer also showed significant enrichment of the indolence signature among genes down-regulated in aggressive tumors (NES = -1.90 and -1.52, respectively; p < 0.001 in both cases) (Fig. 5A; Table 3B,C). Meta-analysis of the down-regulated (*i.e.,* lagging-edge) genes from the prostate, lung, and breast tumors led to the refinement of the original 377 gene signature to a subset of 68 genes that were most significantly enriched in aggressive tumors (Table 4A). In some embodiments expression of prognostic biomarkers is extended to distinguish forms of indolent vs aggressive epithelial cancers, including lung and breast cancer.

Details of experiments and description of their significance are set forth in the Examples.

*Sample Preparation: Protein and Nucleic Acid Extraction*

All of the gene, protein and mRNA sequences of the respective genes, proteins and mRNA used in the Examples are set forth herein.

Biological samples of the epithelial cancer in humans (such as prostate, breast and lung) can be conveniently collected by methods known in the art. Usually, the cancerous tissue can be harvested by trained medical staffs or physicians under sterile environment. Biopsies often are taken, for example, by endoscopic means. After harvested from patients, biological samples may be immediately frozen (under liquid nitrogen) or put into a storage, or transportation solution to preserve sample integrity. Such solutions are known in the art and commercially available, for example, UTM-RT transport medium (Copan Diagnostic, Inc, Corona, Calif.), Multitrans Culture Collection and Transport System (Starplex Scientific, Ontario, CN), ThinPrep® Paptest Preservcyt® Solution (Cytyc Corp., Boxborough, Mass.) and the like. Biological samples of cancerous cells can also come from urine of the subject, and the prognostic biomarker mRNA and protein can be found in blood, plasma and csf.

After collection, biological samples are prepared prior to detection of biomarkers. Sample preparation typically includes isolation of protein or nucleic acids (e.g., mRNA). These isolation procedures involve separation of cellular protein or nucleic acids from insoluble components (e.g., cytoskeleton) and cellular membranes. *In situ* immunostaining of prognostic biomarker proteins can also be done.

In one embodiment, the tissues in the biological samples are treated with a lysis buffer solution prior to isolation of protein or nucleic acids. A lysis buffer solution is designed to lyse tissues, cells, lipids and other biomolecules potentially present in the raw tissue samples. Generally, a lysis buffer of the present invention may contain one or more of the following ingredients: (i) chaotropic agents (e.g., urea, guanidine thiocyanate, or formamide); (ii) anionic detergents (e.g., SDS, N-lauryl sarcosine, sodium deoxycholate, olefine sulphates and sulphonates, alkyl isethionates, or sucrose esters); (iii) cationic detergents (e.g., cetyl trimethylammonium chloride); (iv) non-ionic detergents (e.g., Tween.RTM.-20, polyethylene glycol sorbitan monolaurate, nonidet P-40, Triton.RTM. X-100, NP-40, N-octyl-glucoside); (v) amphoteric detergents (e.g., CHAPS, 3-dodecyl-dimethylammonio-propane-1-sulfonate, lauryldimethylamine oxide); or (vi) alkali hydroxides (e.g., sodium hydroxide or potassium hydroxide). Suitable liquids that can solubilize the cellular components of biological samples are regarded as a lysis buffer for purposes of this application.

In another embodiment, a lysis buffer may contain additional substances to enhance the properties of the solvent in a lysis buffer (e.g., prevent degradation of protein or nucleic acid components within the raw biological samples). Such components may include proteinase inhibitors, RNase inhibitors, DNase inhibitors, and the like. Proteinase inhibitors include but not limited to inhibitors against serine proteinases, cysteine proteinases, aspartic proteinases, metallic proteinases, acidic proteinases, alkaline proteinases or neutral proteinases. RNase inhibitors include common commercially available inhibitors such as SUPERase.In.TM. (Ambion, Inc. Austin, Tex.), RNase Zap.RTM. (Ambion, Inc. Austin, Tex.), Qiagen RNase inhibitor (Valencia, Calif.), and the like.

*Quantification of proteins*

One of ordinary skill in the art will appreciate that proteins frequently exist in a biological sample in a plurality of different forms. These forms can result from either or both of pre- and post-translational modification. Pre-translational modified forms include allelic variants, splice variants and RNA editing forms. Post-translationally modified forms include forms resulting from proteolytic cleavage (e.g., cleavage of a signal sequence or fragments of a parent protein), glycosylation, phosphorylation, lipidation, oxidation, methylation, cysteinylation, sulphonation and acetylation. When detecting or measuring a prognostic protein biomarker of the invention in a sample, the ability to differentiate between different forms of a protein biomarker depends upon the nature of the difference and the method used to detect or measure. For example, an immunoassay using a monoclonal antibody will detect all forms of a protein containing the epitope and will not distinguish between them. However, a sandwich immunoassay that uses two antibodies directed against different epitopes on a protein will detect all forms of the protein that contain both epitopes and will not detect those forms that contain only one of the epitopes. The embodiments of the invention for determining protein levels include adaptations that permit detection of various forms of the protein.

The 3 prognostic protein (or mRNA) markers may be combined into one test for efficient processing of a multiple of samples. In addition, one skilled in the art would recognize the value of testing multiple samples (for example, at successive time points) from the same individual. Such testing of serial samples will allow the identification of changes in marker levels over time. Increases or decreases in marker levels, as well as the absence of change in marker levels, provide useful information as described herein to distinguish indolent from aggressive epithelial cancers as well as to determine the appropriateness of drug therapies, and identification of the patient's outcome, including risk of future events.

In diagnostic assays, the inability to distinguish different forms of a biomarker protein has little impact when the forms detected by the particular method used are equally good biomarkers as any other particular form. However, when a particular form (or a subset of particular forms) of a protein is a better biomarker than the collection of different forms detected together by a particular method, the power of the assay may suffer. In this case, it may be useful to employ an assay method that distinguishes between forms of a protein and that specifically detects and measures a desired form or forms of the protein. Distinguishing different forms of an analyte (e.g., a biomarker) or specifically detecting a particular form of an analyte is referred to as "resolving" the analyte.

Mass spectrometry is a particularly powerful methodology to resolve different forms of a protein because the different forms typically have different masses that can be resolved by mass spectrometry. Accordingly, if one form of a protein is a superior biomarker for a disease than another form of the biomarker, mass spectrometry may be able to specifically detect and measure the useful form where traditional immunoassay fails to distinguish the forms and fails to specifically detect to useful biomarker. A useful methodology combines mass spectrometry with immunoassay. Additionally, certain methods and devices, such as biosensors and optical immunoassays, may be employed to determine the presence or amount of analytes without the need for a labeled molecule. See, e.g., U.S. Pat. Nos. 5,631,171; and 5,955,377.

In embodiments where the three prognostic biomarker proteins are extracted from the biological samples for quantification, expression level can be determined using standard assays that are known in the art. These assays include, but not limited to Western blot analysis, ELISA, radioimmunoassay, competitive binding assays, immune-histochemistry assay and the like. A common assay for the prognostic protein biomarkers is an immunoassay, although other methods are well known to those skilled in the art. The presence or amount of a marker is generally determined using antibodies specific for each marker and detecting specific binding. Specific immunological binding of the antibody to the marker can be detected directly or indirectly. Direct labels include fluorescent or luminescent tags, metals, dyes, radionuclides, and the like, attached to the antibody. Indirect labels include various enzymes well known in the art, such as alkaline phosphatase, horseradish peroxidase and the like. In a preferred embodiment, expression level of the prognostic protein biomarkers may be detected by Western blot analysis.

For western blots, cellular proteins are extracted or isolated from the biological samples (e.g., cancerous tissues), and then separated using SDS-PAGE gel electrophoresis. The conditions for SDS-PAGE gel electrophoresis can be conveniently optimized by one skilled in the art. The three prognostic protein biomarkers in the gels can then be transferred onto a surface such as nitrocellulose paper, nylon membrane, PVDF membrane and the like. The conditions for protein transfer after SDS-PAGE gel electrophoresis may be optimized by one skilled in the art. Preferably, a PVDF membrane is used.

In some embodiments, biomarker proteins are detected using antibodies specific for each of the 3 biomarker proteins for example using immunohistochemical staining on a tissue microarray (TMA) comprised of primary prostate tumors. In the embodiments most of the tumors will have low (G 6 or less) or intermediate (G 7) Gleason scores (Fig. 4A, B; Table 1; Fig. 11). In some embodiments "first" antibodies that that specifically bind to each of the 3 prognostic protein biomarkers are used. Antibodies against the various protein biomarkers can be prepared using standard protocols or obtained from commercial sources. Techniques for preparing mouse monoclonal antibodies or goat or rabbit polyclonal antibodies (or fragments thereof) are well known in the art. See the Examples.

Direct detectable label or signal-generating systems are well known in the field of immunoassay. Labeling of a second antibody with a detectable label or a component of a signal-generating system may be carried out by techniques well known in the art. Examples of direct labels include radioactive labels, enzymes, fluorescent and chemiluminescent substances. Radioactive labels include .sup. 124I, .sup. 125I, .sup. 128I, .sup. 131I, and the like. A fluorescent label includes fluorescein, rhodamine, rhodamine derivatives, and the like. Chemiluminescent substances include ECL chemiluminescent.

ELISA

In another embodiment, detection and quantification of biomarker protein levels is determined by ELISA, typically wherein a first antibody is immobilized onto a solid surface, for example an inert support useful in immunological assays. Examples of inert support include sephadex beads, polyethylene plates, polypropylene plates, polystyrene plates, and the like. In one embodiment, the first antibody is immobilized by coating the antibody on a microtiter plate.

*Detection of mRNA Expression Level*

All mRNA was studied using published values for each respective dataset described herein, and as such was retrospective. The methods used for RNA isolation and running of the microarrays are described in those studies and are standard prortocols that are well known in the art. Details of the methods are described in:

1) Mouse: Ouyang et al.: Ouyang X, DeWeese TL, Nelson WG, Abate-Shen C. Loss-of-function of Nkx3.1 promotes increased oxidative damage in prostate carcinogenesis. Cancer Res 2005; 65: 6773-9.

2) Human a) Yu et al.: Yu YP, Landsittel D, Jing L, et al. Gene expression alterations in prostate cancer predicting tumor aggression and preceding development of malignancy. Journal of clinical oncology : official journal of the American Society of Clinical Oncology 2004; 22: 2790-9.

b) Taylor et al.: Taylor BS, Schultz N, Hieronymus H, et al. Integrative genomic profiling of human prostate cancer. Cancer cell 2010; 18: 11-22.

c) Sboner et al.: Sboner A, Demichelis F, Calza S, et al. Molecular sampling of prostate cancer: a dilemma for predicting disease progression. BMC medical genomics 2010; 3: 8.

The Examples have the materials and methods used to isolate mRNA, protein and to select subjects for the Ouyang, Yu, Taylor and Sboner data sets.

Methods for isolating nucleic acids including mRNA from a cell are well-known in the art. Detection and quantification of mRNA expression levels includes standard mRNA quantitation assays that are also well-known. These assays include but not limited to qRT-PCR (quantitative reverse transcription-polymerase chain reaction), Northern blot analysis, RNase protection assay, and the like. qRT-PCR is preferable to quantify mRNA levels from much smaller samples.

Real-time polymerase chain reaction, also called quantitative real time polymerase chain reaction (Q-PCR/qPCR/qRT-PCR), is used to amplify and simultaneously quantify a targeted DNA molecule. It enables both detection and quantification (as absolute number of copies or relative amount when normalized to DNA input or additional normalizing genes) of one or more specific sequences in a DNA sample. Currently at least four (4) different chemistries, TaqMan®. (Applied Biosystems, Foster City, Calif.), Molecular Beacons, Scorpions® and SYBR® Green (Molecular Probes), are available for real-time PCR.

All of these chemistries allow detection of PCR products via the generation of a fluorescent signal. TaqMan probes, Molecular Beacons and Scorpions depend on Forster Resonance Energy Transfer (FRET) to generate the fluorescence signal via the coupling of a fluorogenic dye molecule and a quencher moiety to the same or different oligonucleotide substrates. SYBR Green is a fluorogenic dye that exhibits little fluorescence when in solution, but emits a strong fluorescent signal upon binding to double-stranded DNA.

Two common methods for detection of products in real-time PCR are: (1) non-specific fluorescent dyes that intercalate with any double-stranded DNA, and (2) sequence-specific DNA probes consisting of oligonucleotides that are labeled with a fluorescent reporter which permits detection only after hybridization of the probe with its complementary DNA target.

Real-time PCR, when combined with reverse transcription, can be used to quantify messenger RNA (mRNA) in cells or tissues. An initial step in the reverse transcription PCR amplification is the synthesis of a DNA copy (i.e., cDNA) of the region to be amplified. Reverse transcription can be carried out as a separate step, or in a homogeneous reverse transcription-polymerase chain reaction (RT-PCR), a modification of the polymerase chain reaction for amplifying RNA. Reverse transcriptases suitable for synthesizing a cDNA from the RNA template are well known.

Following the cDNA synthesis, methods suitable for PCR amplification of ribonucleic acids are known in the art (See, Romero and Rotbart in Diagnostic Molecular Biology: Principles and Applications pp. 401-406). PCR reagents and protocols are also available from commercial vendors, such as Roche Molecular Systems. PCR can be performed using an automated process with a PCR machine.

Primer sets used in the present qRT-PCR reactions for various biomarkers may be prepared or obtained through commercial sources.

The primers used in the PCR amplification preferably contain at least 15 nucleotides to 50 nucleotides in length. More preferably, the primers may contain 20 nucleotides to 30 nucleotides in length. One skilled in the art recognizes the optimization of the temperatures of the reaction mixture, number of cycles and number of extensions in the reaction. The amplified product (i.e., amplicons) can be identified by gel electrophoresis. In real-time PCR assay, a fluorometer and a thermal cycler for the detection of fluorescence during the cycling process is used. A computer that communicates with the real-time machine collects fluorescence data. This data is displayed in a graphical format through software developed for real-time analysis.

In addition to the forward primer and reverse primer (obtained via commercial sources), a single-stranded hybridization probe is also used. The hybridization probe may be a short oligonucleotide, usually 20-35 by in length, and is labeled with a fluorescent reporting dye attached to its 5'-end as well as a quencher molecule attached to its 3'-end. When a first fluorescent moiety is excited with light of a suitable wavelength, the absorbed energy is transferred to a second fluorescent moiety (i.e., quencher molecule) according to the principles of FRET. Because the probe is only 20-35 by long, the reporter dye and quencher are in close proximity to each other and little fluorescence is detected. During the annealing step of the PCR reaction, the labeled hybridization probe binds to the target DNA (i.e., the amplification product). At the same time, Taq DNA polymerase extends from each primer. Because of its 5' to 3' exonuclease activity, the DNA polymerase cleaves the downstream hybridization probe during the subsequent elongation phase. As a result, the excited fluorescent moiety and the quencher moiety become spatially separated from one another. As a consequence, upon excitation of the first fluorescent moiety in the absence of the quencher, the fluorescence emission from the first fluorescent moiety can be detected. By way of example, a Rotor-Gene System is used and is suitable for performing the methods described herein. Further information on PCR amplification and detection using a Rotor-Gene can conveniently be found on Corbett's website.

In another embodiment, suitable hybridization probes such as intercalating dye (e.g., Sybr-Green I) or molecular beacon probes can be used. Intercalating dyes can bind to the minor grove of DNA and yield fluorescence upon binding to double-strand DNA. Molecular beacon probes are based on a hairpin structure design with a reporter fluorescent dye on one end and a quencher molecule on the other. The hairpin structure causes the molecular beacon probe to fold when not hybridized. This brings the reporter and quencher molecules in close proximity with no fluorescence emitted. When the molecular beacon probe hybridizes to the template DNA, the hairpin structure is broken and the reporter dye is no long quenched and the real-time instrument detects fluorescence.

The range of the primer concentration can optimally be determined. The optimization involves performing a dilution series of the primer with a fixed amount of DNA template. The primer concentration may be between about 50 nM to 300 nM. An optimal primer concentration for a given reaction with a DNA template should result in a low Ct-(threshold concentration) value with a high increase in fluorescence (5 to 50 times) while the reaction without DNA template should give a high Ct-value.

The probes and primers of the invention can be synthesized and labeled using well-known techniques. Oligonucleotides for use as probes and primers may be chemically synthesized according to the solid phase phosphoramidite triester method first described by Beaucage, S. L. and Caruthers, M. H., 1981, Tetrahedron Letts., 22 (20): 1859-1862 using an automated synthesizer, as described in Needham-VanDevanter, D. R., et al. 1984, Nucleic Acids Res., 12: 6159-6168. Purification of oligonucleotides can be performed, e.g., by either native acrylamide gel electrophoresis or by anion-exchange HPLC as described in Pearson, J. D. and Regnier, F. E., 1983, J. Chrom., 255: 137-149.

*Kits*

The present invention teaches a kit of manufacture, which may be used to perform detecting either the prognostic biomarker proteins (or fragments thereof) or the mRNA encoding them. In one embodiment, an article of manufacture (i.e., kit) according to the present invention includes a set of antibodies (i.e., a first antibody and a second antibody) specific for each of the 3 biomarker proteins. Antibodies against a house-keeper gene (e.g., GADPH) are provided as a control. In another embodiment, the present kit contains a set of primers (i.e., a forward primer and a reverse primer) (directed to a region of the gene specific to each of the 3 genes in the prognostic panel and optionally a hybridization probe (directed to the same genes, albeit a different region).

Kits taught herein may also include instructions, such as a package insert having instructions thereon, for using the reagents (e.g., antibodies or primers) to quantify the protein expression level of mRNA expression level of the epithelial cancer biomarkers in a biological sample. Such instructions may be for using the primer pairs and/or the hybridization probes to specifically detect mRNA of the prognostic genes. In an embodiment the kids may include oligonucleotides that specifically hybridize with each of the 3 prognostic mRNA biomarkers.

In another teaching, the kit further comprises reagents used in the preparation of the sample to be tested for protein (e.g. lysis buffer). In another embodiment, the kit comprises reagents used in the preparation of the sample to be tested for mRNA (e.g., guanidinium thiocyanate or phenol-chloroform extraction).

The analysis of a plurality of biomarkers may be carried out separately or simultaneously with one test sample. For separate or sequential assay of markers, suitable apparatuses include clinical laboratory analyzers such as the ELECSYS® (Roche), the AXSYM® (Abbott), the ACCESS® (Beckman), the ADVIA® CENTAUR® (Bayer) immunoassay systems, the NICHOLS ADVANTAGE®. (Nichols Institute) immunoassay system, etc. Preferred apparatuses or protein chips perform simultaneous assays of a plurality of markers on a single surface. Particularly useful physical formats comprise surfaces having a plurality of discrete, addressable locations for the detection of a plurality of different analytes. Such formats include protein microarrays, or "protein chips" (see, e.g., Ng and Ilag, J. Cell Mol. Med. 6: 329-340 (2002)) and certain capillary devices (see e.g., U.S. Pat. No. 6,019,944). In these embodiments each discrete surface location may comprise antibodies to immobilize one or more of the prognostic biomarker proteins in a sample for detection at each location.

Certain teachings are directed to microarrays or DNA chips and the like that can be used to quantify or detect the presence of the three prognostic biomarker proteins or mRNA isolated from a biological sample. An embodiment of a microarray for determining if an epithelial tumor is indolent or aggressive includes antibodies or fragments thereof that specifically bind to each of the prognostic biomarker proteins (or variants or fragments thereof) fixed on the array. Another microarray embodiment has at least one oligonucleotide probe that specifically hybridizes to each of the three prognostic biomarker mRNAs fixed on the array.

Surfaces may alternatively comprise one or more discrete particles (e.g., microparticles or nanoparticles) immobilized at discrete locations of a surface, where the microparticles comprise antibodies to immobilize one analyte (e.g., a marker) for detection. As noted, many protein biochips are described in the art. These further include, for example, protein biochips produced by Ciphergen Biosystems, Inc. (Fremont, Calif.), Packard BioScience Company (Meriden Conn.), Zyomyx (Hayward, Calif.), Phylos (Lexington, Mass.) and Biacore (Uppsala, Sweden). Examples of such protein bio chips are described in the following patents or published patent applications: U.S. Pat. No. 6,225,047; PCT International Publication No. WO 99/51773; U.S. Pat. No. 6,329,209, PCT International Publication No. WO 00/56934 and U.S. Pat. No. 5,242,828.

The antibodies and oligonucleotides can be immobilized onto a variety of solid supports, such as magnetic or chromatographic matrix particles, the surface of an assay place (such as microtiter wells), pieces of a solid substrate material or membrane (such as plastic, nylon, paper), and the like. An assay strip could be prepared by coating the antibody or a plurality of antibodies in an array on solid support. This strip could then be dipped into the test sample and then processed quickly through washes and detection steps to generate a measurable signal, such as a colored spot.

The invention has been described in the foregoing specification with reference to specific embodiments. It will however be evident that various modifications and changes may be made to the embodiments The specification and drawings are to be regarded in an illustrative rather than a restrictive sense. The invention is illustrated herein by the experiments described by the following examples, which should not be construed as limiting. Those skilled in the art will understand that this invention may be embodied in many different forms and should not be construed as limited to the embodiments set forth herein. Rather, these embodiments are provided so that this disclosure will fully convey the invention to those skilled in the art. Many modifications and other embodiments of the invention will come to mind in one skilled in the art to which this invention pertains having the benefit of the teachings presented in the foregoing description. Although specific terms are employed, they are used as in the art unless otherwise indicated.

**EXAMPLES**

**Example 1**

**Materials and Methods**

**Study Design:** The study design is shown in Fig. 1. The present study was designed to test the hypothesis that molecular processes of aging and senescence distinguish indolent versus aggressive prostate cancer (Fig. 1). This hypothesis was tested by first assembling a 377-gene signature of aging and cellular senescence, which was used to query human cancer profiles (Table 1), as well as using a mouse model of indolent prostate cancer using GSEA. This resulted in the identification of a 19-gene indolence signature, which was then used to perform decision-tree learning using an independent human cohort to identify a 3-gene prognostic panel that was validated at the mRNA and protein levels using independent cohorts, and then validated on biopsies from patients on active surveillance.

**Statistical methods:** K-means clustering was done using the "kmeans" function from the Statistical toolbox in MATLAB. For confusion matrices, accurate predictions were calculated for indolent or lethal clusters and combined to calculate an Odds Ratio. Kaplan-Meier analyses were conducted using the MATLAB script; *p*-values were computed using a log-rank test. The overall C-index (54), confidence intervals, and corresponding *p*-values were calculated using the survcomp package of R. The predicted probability of survival for computing C-index was obtained through the multivariate Cox proportional hazards models.

**Immunohistochemical analyses:** All studies involving human subjects were approved by the Institutional Review Board of Columbia University Medical Center. Tissue microarrays (TMAs) were comprised of primary prostate tumors obtained from the Herbert Irving Comprehensive Cancer Center Tissue Bank (Table 1). Biopsy samples were obtained from patients seen in the Department of Urology at Columbia University Medical Center from 1992 to 2012. Immunohistochemical analyses were performed using: anti-FGFR1 (Abcam, Cat# ab10646); anti-PMP22 (Sigma, Cat# #P0078); and anti-CDKNIA (BD Pharmingen, Cat#556431). The percentage of positive tumor cells (0% to 100%) and staining intensity (0-2) were assessed for each cores or biopsy, and composite scores were generated.

**Computational methods:** The 377-gene signature of aging and cellular senescence was assembled from the following sources: *(i)* Meta-profile analyses (22); *(ii)* Ingenuity pathway analysis [http://www.ingenuity.com/]; and *(iii)* manual curation (*50-52*). A complete description of the 377-gene set is provided in Table 1. GSEA was performed described (53). Integrative p-*values* were calculated using Fisher's combined probability test. The decision-tree learning algorithm was run by selecting the "classification" method from the "classregtree" function (MATLAB, Statistical toolbox).

**Curation of the aging and senescence signature:** The following resources were used to compile a 377-gene set associated with biological processes of aging and cellular senescence:

(i) Meta-profile analyses of 27 datasets from mouse, rat, and human samples (336 genes) (22);
(ii) Ingenuity pathway analysis for senescence related genes (44 genes) [http://www.ingenuity.com/]; and (iii) manual curation of senescence-related genes (3 genes) (*50-52*). A complete description of the 377-gene set is provided in Table 1.

**Datasets used:** Gene expression profile datasets used in this study are from: *(i)* Yu *et al*: primary human prostatectomy samples (n = aggressive tumors used in this study) with adjacent normal tissue (n = 58), on a Affymetrix U95a, U95b and U95c microarray platform (25); *(ii)* Taylor *et al*: primary human prostatectomy samples with adjacent normal tissue (n = 131 tumor; 95 Gleason 6 and 7(3 +4); n = 23 adjacent normal), on a Affymetrix human Exon 1.0 ST microarray platform (*14*); *(iii)* Sboner *et al* (also called the Swedish cohort): primary human prostate tissue from transurethral resection of the prostate (TURP) (n = 281; Training set used was 25 indolent and 29 lethal; test set used was 28 indolent and 8 lethal), on a 6K DASL microarray platform (*33*); *(iv)* Ouyang *et al*: prostate tissues from *Nkx3.1* homozygous null and wild-type mice (n = 9 total mice in each group), on a Affymetrix Mu74AV2 microarray platform (*31*); *(v)* TCGA breast cancer dataset: invasive breast carcinomas and normal breast tissue (n = 354), on an Agilent G4502A microarray (*27*); and (vi) Lung cancer dataset: lung tumors and normal lung tissue (n = 190), on an Affymetrix human U95A microarray platform (*26*). Available clinicopathological information for the specific patients/samples used in this study is provided in Table 1 and Table 2.

**Data normalization:** Normalized data was available for the Taylor *et al,* Sboner *et al,* breast cancer, and lung cancer datasets. For the Ouyang *et al* and Yu *et al* datasets, expression intensities were background-corrected, normalized, and summarized using the Gene Chip Robust Multiarray Algorithm (GC-RMA) (*55*) in the R/Bioconductor GCRMA package (*56*).

**Differential expression:** Differentially expressed genes were identified using Student's t-test by running "ttest2" command in MATLAB®. For comparing across platforms genes rather than probes were evaluated; if multiple probesets were present for a gene, the probe with the highest absolute differential expression between tumor and normal was selected. For cross-species comparison, mouse genes were first mapped to their human orthologs using the sequence-based method available from NCBI HomoloGene (http://www.ncbi.nlm.nih.gov/pubmed/21097890 and http://www.ncbi.nlm.nih.gov/books/NBK21083/#A866).

**Gene set enrichment analysis:** For Gene Set Enrichment Analysis (GSEA) (53, *57*) genes were ranked by computing their differential expression in the tumor versus normal samples using the Student's t-test method. Sample shuffling (human datasets) or gene shuffling (mouse dataset) with 1,000 shuffles allowed estimation of *p-values* with an accuracy of up to 1 X $10^{-3}$. A list of the leading and lagging edge genes is provided in Table 3.

**Integrative p-value analyses:** To compute the integrative *p-value* for the meta-analyses, first GSEA WAS performed on each of the datasets individually. Then the Fisher's combined probability test (also known as Fisher's method) was used to integrate *p-values.* Fisher's method is computed as follows:

$$X^2 = -2 \sum_{i=1}^{n} log_e(p_i)$$

where *n* is the number of *p-values* $p_i$ and $X^2$ is a variable that follows a chi-squared distribution with *2n* degrees of freedom under the hypothesis of no enrichment. Genes with integrated *p-values* below 0.05 are listed in Table 4. The criteria for inclusion of a given gene in the meta-analyses of the human cancers were as follows: *(i)* must be present in the lagging edge of prostate cancer dataset; *(ii)* must be present in the lagging edge of at least one of the other human datasets (*i.e.,* lung or breast); and *(iii)* must have an integrative *p-value* ≤0.05.

**The 19 Gene Indolence signature:** The 19-gene indolence signature was generated from the intersection of genes from the meta-analyses of human cancers (68 genes) and those in the leading edge from the GSEA of the indolent prostate cancer mouse model (73 genes). A description of the 19-gene indolence signature is provided in Table 5.

**Decision-tree learning model:** The decision-tree learning algorithm was run by selecting the "classification" method from the "classregtree" function (MATLAB, Statistical toolbox). The expression of each gene was discredited into 3 states (up, normal, and down) by comparing the expression in each sample to the average expression across all samples. Genes whose expression in a sample was $e_i \geq \mu + \sigma/2$ where $\mu$ is the average expression and $\sigma$ is the standard deviation were assigned an "up" value, while those whose expression was $e_i \leq \mu + \sigma/2$, were assigned a "down" value; the remaining samples were assigned a "normal" value. In the first step, individual genes were identified whose expression state was significantly predictive of the relative covariate (*i.e.*, indolence or lethality) ($p \leq 0.05$). The expression state of these genes was used to partition the patients. Then, 2-gene combinations were formed by combining each gene from the previous step (*e.g.*, A) with any additional gene (*e.g.*, B) from the remaining 18 genes in the signature (or the same gene with a different expression state). The 2-gene combinations that significantly improved predicted outcome classification over the corresponding single gene classifier were selected (*i.e.*, AB should predict outcome better than A alone; $p \leq 0.05$, to be selected). This process was repeated iteratively by adding more genes, one at the time, to the predictive combinations (*i.e.*, a new branch in the classification tree), up to a maximum of 4 genes. However, the tree pruning method revealed that more than 3 gene combination leads to over fitting suggesting that 3 genes is the optimal number with highest predictive value.

The combinations from the decision tree were verified using a 5-fold cross-validation procedure using the Sboner *et al* training set (Table 2). For the 5-fold cross-validation, 44 patient samples (*i.e.*, 4/5th of test set) were chosen at random for training and the remaining 11 samples (1/5th of the test set) were used to test the trained classifier performance. Gene combinations were ranked based on those with the minimum cross-validation error. A summary of the top combinations from the decision tree is provided in Table 6.

Computational methods are documented in a SWEAVE documents .

**Statistical methods for validation**

**K-means Clustering:** *K*-means clustering algorithm (58) was run using the "kmeans" function from the Statistical toolbox in MATLAB with n = 2 clusters and default values for the remaining parameters.

**Confusion matrices:** Accuracy of predictions were calculated by identifying patients within a test set from Sboner *et al,* which were correctly assigned to indolent (n = 26) or lethal (n = 9) clusters, as well as the number of incorrect predictions. These numbers were combined to calculate an Odds Ratio to assess the predictive accuracies.

**Kaplan-Meier:** Kaplan-Meier analyses for survival difference of patient clusters, partitioned using *K*-means clustering, were conducted using the MATLAB script; *p*-values were computed using a log-rank test.

**Prognostic models:** The overall C-index (*54*), confidence intervals, and corresponding *p*-values were calculated using the survcomp package of R. The predicted probability of survival for computing C-index was obtained through the multivariate Cox proportional hazards models. Statistical methods are documented in a SWEAVE documents.

### Example 2

**Methods for isolating protein and mRNA for the Ouyang, et al. dataset: Cancer Res 2005; 65: (15). August 1, 2005.**

To further minimize variability from individual specimens, prostate tissues from three independent animals were pooled to generate RNA for each array and a minimum of three arrays were probed for the wild-type and mutant mice (thus allowing comparison of a total of nine mice for each). RNA was extracted using Trizol (Invitrogen, Carlsbad, CA) and purified using an RNeasy kit (Qiagen, Chatsworth, CA). cDNA was labeled using a BioArray High-Yield RNA transcript labeling kit (Enzo Life Sciences, Farmingdale, NY) and hybridized to Affymetrix GeneChips (Mu74AV2). For statistical analyses, initial data acquisition and normalization was done using Affymetrix Microarray Suite 5.0 software followed by an ANOVA test. Validation of gene expression changes by quantitative reverse transcription- PCR was done using an Mx4000 Multiplex Quantitative PCR system (Stratagene, La Jolla, CA). Validation to tissue sections was done by in situ hybridization or immunohistochemistry as described, depending on the availability of antisera. For Western blot analyses, anterior prostate tissues were snap-frozen on liquid nitrogen and protein extracts were made by sonication in buffer containing 10 mmol/L Tris-HCl (pH 7.5), 0.15 mol/L NaCl, 1 mmol/L EDTA, 0.1% SDS, 1% deoxycholate (sodium salt), 1% Triton X-100, with freshly added protease inhibitor and phosphatase inhibitor cocktail (Sigma, St. Louis, MO). For in situ hybridization, sequence-verified expressed sequence tag clones were purchased from Invitrogen.

### Example 3

**Methods for isolating protein and mRNA for the Yu dataset:**

A comprehensive gene expression analysis was performed on 152 human prostate samples, including prostate cancer (PC), prostate tissues adjacent to (AT) cancer, and donor (OD) prostate tissue totally free of disease, using the Affymetrix (Santa Clara, CA) U95a, U95b, and U95c chip sets. A set of 671 genes were identified whose expression levels were significantly altered in PCs compared with normal tissues. Interestingly, the expression patterns of histological benign prostate tissues were significantly overlapped with those of PC, and were distinctly different than donor prostate tissue. Separately, a "70-gene" model was developed to predict the aggressiveness of the disease. Collectively, these data suggest that genetic alterations in a gland with PC are not limited to the malignant cells, and these patterns of alteration may predict the population both at risk for the disease and for disease progression.

**Sample Preparation:** Fresh prostate tissues, recovered immediately from the operating room after removal, were dissected and trimmed to obtain pure tumor (completely free of normal prostate acinar cells) or normal prostate (free of tumor cells) tissues. Microdissection was coupled with sandwich frozen and permanent section analyses to confirm the purity and homogeneity of the samples: gross and microscopic analyses were performed by board-certified genitourinary pathologists. For tumor tissues, only samples with less than 30% of stromal components were selected. For donor prostate tissues, obtained at the time of organ donation in brain-dead men, samples from peripheral zone of the prostate gland with at least 60% glandular components and free of any pathological alteration were selected For prostate tissues adjacent to cancer, samples free of cancer cells, high-grade prostatic neoplasia, or any obvious neoplastic alterations, containing at least 60% glandular cells, were selected. Whenever possible, all tissues were processed and frozen within 30 minutes after removal. These tissues were then homogenized. All patients with PCs have at least a 4-year follow-up, with regular evaluations for relapse or the presence of metastasis. Protocols for tissue banking, tissue anonymization, and tissue processing, were approved by the institutional review board.

**Affymetrix Chip Analysis cRNA preparation:** Total RNA was extracted and purified with Qiagen RNeasy kit (Qiagen, San Diego, CA). Five micrograms of total RNA were used in the first strand cDNA synthesis with T7-day(T)24 primer (GGCCAGTGAATTGTAATACGACTCACTATAGGGAGGCGG-(dT)24) by Superscript II (GIBCO-BRL, Rockville, MD). The second strand cDNA synthesis was carried out at 16°C by adding *Escherichia coli* DNA ligase, *E coli* DNA polymerase I, and RnaseH in the reaction. This was followed by the addition of T4 DNA polymerase to blunt the ends of newly synthesized cDNA. The cDNA was purified through phenol/chloroform and ethanol precipitation. The purified cDNA were then incubated at 37°C for 4 hours in an in vitro transcription reaction to produce cRNA labeled with biotin using MEGAscript system (Ambion Inc, Austin, TX). *Affymetrix chip hybridization.* Between 15 and 20 _g of cRNA were fragmented by incubating in a buffer containing 200 mmol/L Tris-acetate, pH8.1, 500 mmol/L KOAc, and 150 mmol/L MgOAc at 95°C for 35 minutes. The fragmented cRNA were then hybridized with a pre-equilibrated Affymetrix chip at 45°C for 14 to 16 hours. After the hybridization cocktails were removed, the chips were then washed in a fluidic station with low-stringency buffer (6_sodium chloride, sodium phosphate dibasic, and EDTA; 0.01% Tween 20; 0.005% antifoam) for 10 cycles (two mixes/cycle), and stringent buffer (100 mmol/L MES, 0.1MNaCl and 0.01% Tween 20) for four cycles (15 mixes/cycle), and stained with Strepto-avidin Phycoerythrin (SAPE; Molecular Probe, Eugene, OR). This was followed by incubation with biotinylated mouse antiavidin antibody, and restained with SAPE. The chips were scanned in aHPChipScanner (Affymetrix Inc) to detect hybridization signals. For quality assurance, all samples were run on Affymetrix test-3 chips to evaluate the integrity of RNA; samples with RNA 3_/5_ ratios less than 2.5 were accepted for further analysis.

**Data analysis:** Hybridization data were normalized to an average target intensity of 500 per chip, and were converted to Microsoft Excel spreadsheet text file (Redmond, WA). The primary comparison of OD to PC was conducted through the following steps: (1) Two sample $t$ tests of log-transformed gene expression values, (2) adjustment of $P$ values through the Benjamini and Hochberg procedure, (3) selection of genes that meet both the critical $P$ value and show at least a two-fold change in PC, (4) reduction of dimensionality through principal component analysis, (5) prediction of case status (ie, normal $v$ cancer tissue) through logistic regression, and (6) evaluation of the classification rate using 10- fold cross-validation. Regarding the second step, the Benjamini and Hochberg procedure calculates a conservative $P$ value to minimize the expected number of falsely significant results. For tests between PC and AT, the paired $t$ test (of log-transformed expressions) was utilized to account for the matching. A sufficient number of principle components (in the fourth step) were retained to quantify at least 90% of the variability in these genes. For the cross validation procedure (sixth step), a separate logistic model is fit for each of the ten subsets used for training, and then used to predict the outcome for the remaining subset of validation data. After this process is implemented for classifying donors versus PC, the resulting model parameters (using the entire data set) were saved and utilized to predict case status of adjacent to tumor normal tissue. The fitted logistic model (again using the entire data set) was also used to classify separate validation data sets collected from other institutions. These analyses were all conducted using S-PLUS statistical software (Insightful Corp, Seattle, WA).

## Example 4

**Methods for isolating protein and mRNA for the Sboner dataset,** *BMC* Medical Genomics **2010, 3:8:**

**Patient population:** This present study is nested in a cohort of men with localized prostate cancer diagnosed in the Orebro (1977 to 1994) and South East (1987 to 1999) Health Care Regions of Sweden. Eligible patients were identified through population- based prostate cancer quality databases maintained in these regions (described in Johansson et al., Aus et al., and Andren et al. and included men who were diagnosed with incidental prostate cancer through (TURP) or adenoma enucleation, i.e. stage T1a-b tumors. In accordance with standard treatment protocols at the time, patients with early stage/localized prostate cancer were followed expectantly ("watchful waiting"). No PSA screening programs were in place at the time. The study cohort was followed for cancer-specific and all cause mortality until March 1, 2006 through record linkages to the essentially complete Swedish Death Register, which provided date of death or migration. Information on causes of death was obtained through a complete review of medical records by a study end-point committee. Deaths were classified as cancer-specific when prostate cancer was the primary cause of death. Tumor tissue specimens were traced from 92% (1256/1367) of all potentially eligible cases. In order to provide complete and consistent information, available hematoxylin and eosin (H&E) slides from each case were reviewed to identify all tissue specimens with tumor tissue. Slides and corresponding paraffin-embedded formalin- fixed blocks were subsequently retrieved and rereviewed to confirm cancer status and to assess Gleason score and other notable histopathologic features. The reviewers were blinded with regard to disease outcome. Gleason score was evaluated according to Epstein et al. All patients gave informed consent for the study. Since our overarching aim was to identify signatures predicting a lethal or an indolent course of prostate cancer, efficiency was maximized by devising a study design that included men who either died from prostate cancer during follow up (lethal prostate cancer cases) or who survived at least 10 years after their diagnosis (men with indolent prostate cancer). Thus men with non-informative outcomes were excluded, namely those who died from other causes within ten years of their prostate cancer diagnosis or had been followed for less than 10 years with no disease progression (n = 595). All men with samples in which high-density tumor regions (defined as more than 90% tumor cells) could be identified were included (n = 381). Men who had received any type of androgen deprivation treatment during follow up (n = 79) were excluded from the indolent group, since some of these had potentially lethal disease that was deferred by therapy. Twenty-one men were further excluded due to poor sample quality. In total, 281 men (116 with indolent disease and 165 with lethal prostate cancer) were included in the analyses. The study design was approved by the Ethical Review Boards in Orebro and Linköping. The clinical and pathologic demographics of these of 281 men with prostate cancer are presented. In addition to the standard pathology evaluation each case was also characterized with respect to ERG gene rearrangement, since it appears that this event is an indicator of poor prognosis .

**Complementary DNA-mediated annealing, selection, ligation, and extension array design:** An array of 6100 genes (6K DASL) was designed for the discovery of molecular signatures relevant to prostate cancer by using four complementary DNA (cDNA)-mediated annealing, selection, ligation, and extension (DASL) assay panels (DAPs) See Gene Expression Omnibus (GEO: http://www.ncbi.nlm.nih.gov/geo/ with platform accession number: GPL5474. This data set is also available at GEO with accession number: GSE16560.

### Example 5

**Taylor dataset: Cancer Cell 18, 11-22, July 13, 2010 Cancer Cell 18, 11-22, July 13, 2010**

**Specimen collection and annotation:** A total of 218 tumor samples and 149 matched normal samples were obtained from patients treated by radical prostatectomy at Memorial Sloan-Kettering Cancer Center. All patients provided informed consent and samples were procured and the study was conducted under Memorial Sloan-Kettering Cancer Center Institutional Review Board approval. Clinical and pathologic data were entered and maintained in our prospective prostate cancer database. Following radical prostatectomy, patients were followed with history, physical exam, and serum PSA testing every 3 months for the first year, 6 months for the second year, and annually thereafter. For all analyses described here, biochemical recurrence (BCR) was defined as PSA R0.2 ng/ml on two occasions. At the time of data analysis, patient follow-up was completed through December 2008.

**Analyte extraction and microarray hybridization:** DNA and RNA were extracted from dissected tissue containing greater than 70% tumor cell content as well as from seven cell lines and seven xenografts (see Supplemental Information). Resulting DNA and RNA were hybridized to Agilent 244K array comparative genomic hybridization (aCGH) microarrays, Affymetrix Human Exon 1.0 ST arrays, and/or Agilent microRNA V2 arrays, respectively. The normalization and statistical analysis of both DNA copy-number and expression array data are available in the Supplemental Information.

**DNA sequencing:** In total, 251 million bases in coding exons and adjacent intronic sequences of 138 cancer-related genes in 91 samples were PCR-amplified and sequenced by Sanger capillary sequencing. Ninety-five sites of known mutation in 22 genes were also genotyped using the iPLEX Sequenom platform. The details of whole-genome amplification, sequencing, mutation detection pipelines, mutation validation, background mutation rate analysis, and Sequenom genotyping are described in the Supplemental Information.

**Outlier expression analysis:** Outlier profiles for all transcripts and outlier assignments in all tumors were determined from normalized expression data as previously described (Ghosh and Chinnaiyan, 2009). In brief, in this nonparametric approach an empirical distribution function generated from transcript expression in the 29 normal prostate tissues was used to transform expression in the tumor samples, from which outliers were determined with the criteria described in the Benjamini and Hochberg algorithm (Benjamini and Hochberg, 1995) at an error rate (a) = 0.01.

### Example 6 Validation of 3-gene prognostic panel by immunohistochemistry

**Immunohistochemical analyses:** All studies involving human subjects were approved by the Institutional Review Board of Columbia University Medical Center. Tissue microarrays (TMAs) were comprised of primary prostate tumors obtained from the Herbert Irving Comprehensive Cancer Center Tissue Bank from 121 radical prostatectomy specimens (including 44 that were Gleason 6 or Gleason 7 (3+4)) with 102 adjacent normal tissues as controls (Table 1). The TMA was constructed (Beecher Instruments, MD, USA) by punching triplicate cores of 1 mm for each sample.

Immunohistochemical analyses were performed using: anti-FGFR1 (Abcam, Cat# ab10646); anti-PMP22 (Sigma, Cat# #P0078); and anti-CDKNIA (BD Pharmingen, Cat#556431). The percentage of positive tumor cells (0% to 100%) and staining intensity (0-2) were assessed for each cores or biopsy, and composite scores were generated.

A cohort of retrospective biopsy samples were obtained from patients enrolled in a surveillance protocol in the Department of Urology at Columbia University Medical Center from 1992 to 2012. Patients included in the surveillance protocol presented with low risk prostate cancer with the following essential criteria: normal digital rectal exam (DRE), serum PSA <10 ng/ml, biopsy Gleason score ≤6 in no more than 2 cores, and cancer involving no more than 50% of any core on at least a 12-core biopsy. The current protocol to monitor these patients includes DRE and serum PSA testing every three months, and repeat biopsy every 12 or 18 months, or "for-cause biopsy" if any sign of progression (abnormal DRE, increasing PSA) becomes evident. Biopsy samples were immunostained and scored using the protocol outlined above.

Immunohistochemical analyses were performed using a rabbit polyclonal anti-FGFR1 antibody (Abcam, Cat# ab10646) at a concentration of 1 $\mu$g/ml; a rabbit polyclonal anti-PMP22 (Sigma, Cat# #P0078) at 1 $\mu$g/ml; and a mouse monoclonal CDKN1A (BD Pharmingen, Cat#556431) at 500 $\mu$g/ml. Controls for antibody specificity are shown in Fig. 11. Slides were deparaffinized in xylene, followed by antigen retrieval through boiling for 37 minutes at 100°C in Decloaking Solution (Citrate buffer, pH 6.0, Biocare Medical) in a pressure cooker. Following cooling, slides were incubated in 3% $H_2O_2$ and then blocked in 10% goat serum for rabbit primary antibodies or 10% horse serum for mouse primary antibodies. Following overnight incubation in primary antibody, slides were washed in PBS containing 0.05% Triton X-100 and then incubated for 1 hour at room temperature with biotinylated anti-rabbit or anti-mouse secondary antibody (Vector Laboratories.) The signal was amplified by Vectastain ABC system (Vector Laboratories, PK6200) and visualized with the NovaRed Substrate Kit (Vector Laboratories, SK4800). Slides were counterstained with Harris Modified Hematoxylin (1:4 diluted in H2O) (Fisher Scientific) and coverslipped with Clearmount (American Master*Tech Scientific). Negative and positive controls for each of the antibodies were used in parallel to assure antibody specificity (Fig. 11).

Stained slides were scanned using an Olympus BX61Whole Slide scanner. For CDKN1A nuclear expression was evaluated; for FGFR1 and PMP22 both nuclear and cytoplasmic/cell surface expression were analyzed. Scoring was performed without knowledge of the clinico-pathological variables. The percentage of positive tumor cells (from 0% to 100%), as well as staining intensity was assessed for each of the cores. For intensity, values were assigned on a three-point scale: 0 represents no staining, 1 represents a mild to moderate positivity and 2 represents an intense immunoreaction. Composite scores were generated by multiplying the percentage of positive cells and staining intensity; the mean score for each patient from the triplicate cores was used for *K*-means clustering to identify low-risk and high-risk groups based on the three proteins in classifier.

**Example 7**

**Methods for phenotypic analyses of *Nkx3.1* mutant mice:**

The *Nkx3.1* germline mutant mice have been described previously (28). Wild-type and null littermates were sacrificed for analyses at 4-month intervals from 3 to 24 months of age. For histological and immunohistochemical analyses, tissues were fixed in 10% formalin and analyses done as described previously (*59*). For SA-β-Gal analysis, freshly dissected (unfixed) prostatic tissues were cryopreserved in Optimal Cutting Temperature (OCT) compound and stained using the Chemicon SA-β-GAL kit (KAA002) following the manufacturer's instructions. For protein extraction, tissues were snap-frozen in liquid nitrogen, and processed for western blot analyses as described (*59*). Antibodies used in the mouse analyses were as follows: mouse monoclonal HP1γ (clone 2MOD-1G6) (EMD Millipore, Cat no. MAB3450); rabbit polyclonal Ab Ki67 (Novacastra/Leica, Cat no. NCL-Ki67p); rabbit polyclonal Ab GADD45alpha (Cell Signaling Technology, Cat no. 3518S); mouse monoclonal Ab PML clone 36.1-104 (Millipore, Cat no. 05-718), rabbit polyclonal Ab BECN1 (H-300) (Santa Cruz, Cat no. sc-11427) and rabbit monoclonal Ab B-Actin (13E5) (Cell Signaling Cat no 4970).
**Level of Evidence:** The current study falls into the Level of Evidence category D as it is a retrospective, observational study that involves multiple independent datasets. A REMARK

**Example 8 An "indolence gene signature" of aging and senescence distinguishes indolent versus aggressive prostate cancer**

A. Identification of a gene signature for prostate cancer that is associated with aging and senescence

A first step was the generation of a literature-, pathway-, and manually-curated 377 gene signature associated with aging and senescence (Fig. 1, Step 1; Table 1). This gene signature was primarily assembled from a meta-analyses of aging-related genes (22), and accordingly was enriched for biological pathways associated with various aging-associated diseases, while it had limited enrichment for pro-tumorigenic pathways such as those associated with cellular proliferation. Notably, the 377-gene signature had virtually no overlap with previously identified signatures associated with cellular proliferation (23, 24).
Gene set enrichment analyses (GSEA) was next done to evaluate whether this signature of aging and senescence was enriched in genes *down-regulated* in aggressive human prostate cancer and, conversely, *up-regulated* in indolent prostate cancer (Fig. 1, Step 2). These analyses were extended to infer that the intersection of the genes enriched among those *down-regulated* in aggressive human prostate cancer (*i.e.*, the lagging edge) and *up-regulated* in indolent prostate cancer (*i.e.*, the leading edge) would identify those most closely associated with indolence (*i.e.*, an "indolence signature", Fig. 1, Step 2). For these and subsequent analyses, published expression profiling datasets were used, either to discover or refine genes for classification purposes (training sets), or to validate their statistical power and performance (test/validation sets), but never for both purposes (Fig. 1, Table 1).
To evaluate the expression of the 377-gene signature of aging and senescence in aggressive prostate cancer, GSEA analyses using the Yu *et al* dataset was performed, which includes a subset of aggressive, locally invasive prostate tumors (n = 29) with adjacent normal prostate tissue (n = 58) as controls (25) (Table 1; Table 2A). Consistent with the hypothesis, the 377-gene signature was enriched among genes *down-regulated* in these aggressive prostate tumors compared with the normal controls (NES = -1.87; p < 0.001) (Fig. 2A; Table 3A). Interestingly, additional epithelial cancers, lung and breast (the references for the gene sets used for lung and breast are published datasets described in 26, 27) also showed significant enrichment of this indolence signature among genes *down-regulated* in aggressive tumors (NES = -1.90 and -1.52, respectively; p < 0.001 in both cases) (Fig. 5A; Table 3B,C). Meta-analysis of the down-regulated (*i.e.*, lagging-edge) genes from the prostate, lung, and breast tumors led to the refinement of the original 377 gene signature to a subset of 68 genes that were most significantly enriched in aggressive tumors (Table 4A). These findings support the hypothesis that genes associated with aging and senescence are enriched among down-regulated genes in aggressive prostate cancer, as well as other epithelial cancers.

B. Cross-species analysis identifies a 19-gene "indolence signature;" Nkx3.1 homozygous mutant mice are a relevant model of indolent prostate cancer.

Since the 377-gene set is enriched for genes *down-regulated* in aggressive prostate cancers (Fig. 2A), it was expected that the most informative genes in this signature should be *up-regulated* in indolent prostate tumors. However, independent human datasets containing purely indolent prostate tumors were not available to evaluate this hypothesis. Therefore, as a source of purely indolent prostate lesions, cross-species analyses was performed using a well-characterized mouse model of pre-invasive prostate cancer, which is based on germline loss-of-function of the *Nkx3.1* homeobox gene (28, 29). Notably, this cross-species approach, which uses enrichment analyses of relatively homogenous mouse model to "filter" the characteristically heterogeneous human prostate tumors, also enabled identification of the most conserved and relevant genes among the signature.

Human *NKX3.1* is localized to a chromosomal hotspot, 8p21, which is frequently lost in prostate intraepithelial neoplasia (PIN) and prostatic intraepithelial neoplasia (PIN). Down-regulation of Human *NKX3.1* expression is associated with cancer initiation, although it is not sufficient for overt carcinoma (*30*). Targeted inactivation of *Nkx3.1* in mice leads to PIN, which does not progress to adenocarcinoma even in aged mice (28, 29) (Fig. 6A-D). Further, this age-associated arrest in cancer progression in the *Nkx3.1* mutant mice is coincident with elevated cellular senescence and abrogation of cellular proliferation (Fig S2E-I). Since the *Nkx3.1* mutant mice develop pre-invasive prostate lesions with an aging-associated halt in tumor progression that is coincident with cellular senescence, it was hypothesized that they would provide a relevant model of indolent prostate cancer.

GSEA was performed using expression profiles from aged *Nkx3.1* homozygous mutant and control (age-matched) wild-type mouse prostates (n=9/group) (*31*). Whereas the 377-gene signature was enriched for genes *down-regulated* in the aggressive prostate tumors (*i.e.,* in the *lagging* edge) (see Fig. 2A), the indolent prostate lesions were enriched for the *up-regulated* genes (*i.e.,* in the *leading* edge) (NES = 1.81; p < 0.001) (Fig. 2B; Table 3D). Therefore it was hypothesized that the intersection of genes *down-regulated* in aggressive human tumors (*i.e.,* the 68 genes from the meta-analysis of human cancers) and those *up-regulated* in the indolent prostate lesions from the *Nkx3.1* mice (*i.e.,* the 73 genes from the leading edge) would identify the most consistently regulated genes for an effective indolence classifier (Fig. 2C). As predicted, these analyses identified 19 genes that are significantly *up-regulated* in indolent prostate cancer and *down-regulated* in aggressive tumors; herein the 19-gene "indolence signature" (Fig. 2C; Table 5). This intersection is highly statistically significant (p < 0.001, by Fisher Exact Test), suggesting that these genes are under coordinated regulation in the aggressive and indolent tumors, and are thus well-suited for classification of these states. Taken together, these findings show that genes associated with aging and senescence can be used to distinguish prostate cancers according to aggressive versus indolent behavior.

C. Gene signature of aging and senescence distinguishes disease outcome of low Gleason score prostate cancer

The Taylor *et al* dataset was used to independently validate these observations; it is one of the few publicly available human datasets with extensive clinical outcome data (14) (Table 1). Taylor *et al* contains a substantial number of prostatectomy samples (n = 131) with adjacent normal controls (n =23) from patients that encompass a wide range of Gleason scores and times to biochemical recurrence (*14*) (Table 1; Table 2B). This dataset includes a significant number (n = 13) of aggressive prostate tumors (*i.e.,* Gleason 8,9) with a short time to biochemical recurrence (< 22 months) (Table 1; Table 2B). GSEA analyses of these high Gleason grade tumors demonstrated their similar behavior to the aggressive tumors from Yu et al, since the 377-gene signature was significantly enriched for genes *down-regulated* in these aggressive prostate tumors (NES = -2.60 and p < 0.001), including most (18/19) of the 19-gene indolence signature (Fig. 2D; Table 5). Therefore, both the behavior and specific enrichment of the 377-gene signature was conserved in an independent dataset of aggressive human prostate cancer.

The Taylor et al. dataset also contains a substantial number of low Gleason score tumors (*i.e.,* Gleason 6; n = 41; and Gleason score 7(3 + 4); n = 54) with varying times of progression to biochemical recurrence (BCR) ranging from >100 months (*i.e.,* indolent) to <35 months (*i.e.,* aggressive) (Table 1; Table 2B). Experiments were conducted to recapitulate the differential enrichment of the 377-gene signature in the indolent versus aggressive tumors by limiting the sample to only to low Gleason score prostate tumors (Fig. 2E-F; Fig. 6B). These and most subsequent analyses focused primarily on Gleason score 6 tumors, but (for increased statistical power) the subset of Gleason score 7 tumors that were scored as 3+4 (refer to these combined Gleason 6 and Gleason 7(3+4) as "low Gleason score tumors") were also included. Interestingly, it was consistent in the molecular analyses that Gleason 7 tumors scored as 3+4 behaved more like the Gleason score 6 tumors, while those scored as 4+3 behaved more like the more advanced Gleason Score tumors, which is in agreement with a recent study by Balk and colleagues showing that Gleason 3 and 4 lesions have different molecular features and progressive potential (32).

First, GSEA was performed on the low Gleason Score prostate tumors to evaluate enrichment of the 377-gene signature of aging and senescence in the two extreme patient groups (*i.e.,* the most lethal versus the most indolent). In particular, the first group included patients with a short time to biochemical recurrence (*i.e.,* the aggressive group, Gleason score 6 and Gleason score 7(3 + 4) tumors having BCR < 35 months; n = 5) and the second included patients that did not recur within the considerable follow-up period of greater than 100 months (*i.e.,* the indolent group, Gleason score 6 and Gleason score 7(3 + 4) tumors BCR > 100 months; n = 5) (Fig. 2F; Table 2B). GSEA analyses demonstrated that the 377-gene signature was enriched in genes *up-regulated* in the indolent group (BCR > 100 months), with a positive NES score (NES=1.52 p value<0.001), whereas it was enriched in genes *down-regulated* in the aggressive group (BCR < 35 months), with a negative NES score (NES= -1.85, p value<0.001 Fig. 2F; Table 3E,F).

Enrichment of the 377-gene signature was further assessed in indolent versus aggressive low Gleason score tumors focusing only on the Gleason score 6 patients. In particular, the Gleason score 6 patients were partitioned into subgroups representing varying interval to biochemical recurrence: > 0 months (n = 41); >35 months (n=32), >50 months (n=20), >65 months (n=8), >80 months (n=5), > 100 months (n=3), and then GSEA was performed on each of these sub-groups. Strikingly, while all of the sub-groups displayed enrichment of the 377-gene signature, the direction of the enrichment was dependent on the interval to biochemical recurrence (Fig. 2E). In particular, Gleason grade 6 tumors with a longer interval to biochemical recurrence (> 65, > 80, and > 100 months) were enriched in the leading edge (and had a positive NES score), while those with a shorter interval to recurrence (> 0, > 35, > 50 months) were enriched in the lagging edge (and had a negative NES score) (Fig. 2E; Fig. 51B).

Taken together, these GSEA show that differential enrichment of a signature of aging and senescence can distinguish low Gleason score tumors that are destined to remain indolent from those destined to become aggressive. Furthermore, meta-analyses of the *leading* and *lagging* edge genes in these indolent versus aggressive sub-groups of Gleason 6 tumors included a majority of the 19-gene "indolence signature" among those that were significant (14/19 genes; Table 5). Taken together, these findings demonstrate that low Gleason score prostate tumors can be distinguished as indolent or aggressive based on enrichment for a gene signature of aging and senescence and constitute an independent validation of the indolence signature.

## D. A 3-gene prognostic biomarker panel low Gleason score prostate tumors

Notably, while the 19-gene indolence signature is differentially enriched in indolent versus aggressive subtypes, it was not sufficient to stratify patient patients using Kaplan Meier analyses (Fig. 9A). Thus, it was important to identify a minimal subset(s) of genes among those in the 19-gene indolence signature that most effectively predicts clinical outcome for low Gleason score prostate tumors. A decision-tree learning model was used to evaluate gene combinations among the 19-gene signature that best distinguish indolent versus lethal prostate tumors (Fig. 1, Step 3; Fig. 3A). The decision-tree model iteratively partitions patients according to the expression state of the gene with the highest predictive value, considering both synergistic and antagonistic affects between genes, and terminating once further partitioning has no additional statistical predictive value. Each leaf node in the resulting predictive tree corresponds to a set of patients with predicted prognostic outcome; each branch corresponds to the expression state of a predictive gene, and a walk from the root of the tree to a leaf node reveals the expression state of the gene panel used to predict outcome at the leaf node.

Decision-tree analyses was done using an independent dataset, namely the Swedish "watchful waiting" cohort of Sboner *et al.*, which includes expression profiles from transurethral resection of prostate (TURP) specimens from 281 patients with localized prostate cancer that were followed for up to 30 years (*33*). Notably, this dataset differs from the Taylor dataset in several important respects: (*i*) sample collection in Sboner predates the PSA screening era (tissues collected prior to 1996); (*ii*) expression profiles were obtained from TURP rather than prostatectomies; and (*iii*) the primary endpoint in the Sboner cohort is death due to prostate cancer rather than time to biochemical recurrence, as in the Taylor et al (Table 1). Considering these important distinctions between the Taylor and Sboner cohorts, biomarkers that show consistent stratification power in both were expected to be robust.

To focus on genes that most effectively inform outcome, analysis was limited to the extreme outcome of cases in the Sboner dataset. Specifically, two groups were identified: an "indolent group" with long-term survival following initial diagnosis ($t \geq 10$ years; n= 26), and a "lethal group" in which patients died early from prostate cancer ($t < 4$ years; n=29) (Table 1; Table 2). Thus, the decision tree was constructed using these extreme patients groups in the Sboner et al. training set.

Among thousands of possible trees evaluated in the decision tree model only fourteen 3-gene prognostic panel combinations had cross-validation power greater than 0.25 (Fig. 7A; Table 6). Trees with significant predictive power repeatedly included *CDNK1A*, *FGFR1*, *PMP22*, *Clusterin*, and *CLIC4* (Fig. 3B; Table 6A). The top-ranked combinations were tested for predictive accuracy using confusion matrices to "score" predicted versus actual indolent and lethal cases (Fig. 3B, Fig. 8). First, a test set was assembled from cases in Sboner et al that had not been used for decision tree learning (n = 28 indolent and 8 lethal; Table 1; Table 2). Then, each gene panel was used to classify patients based on survival. Interestingly, the best gene panel (odds ratio = 1.94) identified from confusion matrix analysis was also the top-ranked panel from the decision-tree model. This panel included *FGFR1, PMP22* and *CDKN1A* (Fig. 3B, Fig. 8) and was selected as our candidate biomarker panel to further evaluate for stratifying low Gleason score prostate tumors.

E. Validation of the 3-geneprognostic panel at the mRNA and protein levels

The prognostic accuracy of the 3-gene prognostic panel (*i.e.*, *FGFR1*, *PMP22* and *CDKN1A*) at the mRNA expression level (Fig. 1, Step 4) was first evaluated, using the low Gleason score (*i.e.,* Gleason score 6 and Gleason score 7(3+4)) tumors from Taylor et al. (n = 95; Table 1; Table 2). The ability of the 3-gene prognostic panel to segregate these low Gleason score tumors into low- and high-risk groups was evident in *k*-means clustering (Fig. 7B), an unsupervised clustering approach that relies only similarity of gene expression in different samples without using any clinical information about the patients. As is evident by Kaplan-Meier analysis, the 3-gene prognostic panel (*FGFR1*, *PMP22* and *CDKN1A)* robustly segregated the low Gleason score prostate tumors into high- and low-risk groups based on time to biochemical recurrence (n=95 cases; *p* = 0.005) (Fig. 3C).

Interestingly, in these and subsequent analyses, the 3-gene prognostic panel was were consistently more effective in stratification of low Gleason score tumors as compared with the entire patient population, including higher Gleason score tumors (n = 131; *p* = 0.047) (Fig. 9B). Furthermore, the 3-gene prognostic panel was significantly more effective in segregating patients than the 19-gene indolence signature (compare Fig. 3C with Fig. 9A, B), which further demonstrates the efficacy of the decision tree learning model for selecting the most clinically-relevant biomarkers among the 19-gene signature. Notably, only one of the other top six gene combinations from the decision tree model (*FGFR1*, *B2M* and *CDKN1A)* was significant (*p* = 0.02) in stratifying low Gleason score prostate tumors into high- and low-risk groups (Fig. 3B, Fig. 9C), and it is noteworthy that this combination shares two genes in common with the 3-gene prognostic panel. Finally, although certain of the individual genes (*FGFR1*, *PMP22* and *CDKN1A)* had prognostic power in some assays, only the 3-gene prognostic panel was consistently observed to have prognostic potential in all of the models and cohorts evaluated (see Fig. 10).

The prognostic value of the 3-gene prognostic panel was further evident using C-statistics in comparison with pathological Gleason score or the D'Amico classification nomogram, which takes into account Gleason score, Clinical T stage, and PSA levels (34) (Fig. 3D). In particular, the 3-gene prognostic panel performed better (C-index 0.86; CI 0.65-1.0; $p = 3.3 \times 10^{-4}$) than either Gleason score alone (C-index 0.82; CI 0.54-1.0; $p = 0.010$) or the D'Amico classification alone (C-index 0.72; CI 0.52-0.90; $p = 0.012$), while the 3-gene prognostic panel significantly improved prognostic capability when combined either with Gleason or D'Amico (C-index= 0.89; CI 0.74-1.0; $p=4.7 \times 10^{-8}$ and C-index= 0.83; CI 0.73-0.95; $p=1.8 \times 10^{-9}$, respectively) (Fig. 3D). Furthermore, multivariate Cox proportional hazard analysis showed that the 3-gene prognostic panel together with Gleason had statistically significant improved prognostic ability than using Gleason alone (*p*=0.04). For D'Amico classification, the improved prognostic ability was mostly due to additive effects of the 3-gene prognostic panel, which was significant (*p*=0.017). This improvement was diluted by the high degrees of freedom of the full interaction model between D'Amico covariates and the 3-gene prognostic panel prediction (*p* = 0.11) (Fig. 3E). Taken together, these findings demonstrate the independent prognostic value of the 3-gene prognostic panel at the mRNA level.

These findings were extended to evaluate whether the 3-gene prognostic panel was also prognostic at the protein level (Fig. 1, Step 4). Immunohistochemical staining was performed on a tissue microarray (TMA) comprised of primary prostate tumors that corresponded to a wide range of Gleason scores, although the focus was on the low Gleason score tumors (*i.e.,* the Gleason 6 and Gleason 7 (3+4)) (Fig. 4A, B; Table 1; Fig. 11). The predictive accuracy of the 3-gene prognostic panel was supported by unsupervised *k*-means clustering analyses, in which there was 2 to 4 fold higher staining intensity for tumors classified in the indolent versus the aggressive clusters (Fig. 7C). Moreover, Kaplan-Meier analyses revealed that the protein expression levels of FGFR1, PMP22 and CDKN1A effectively stratified the low Gleason score tumors into high- and low-risk groups (p= 0.015) (Fig. 4B).

C-statistic analyses of this cohort revealed that the 3-gene prognostic panel performed significantly better (C-index 0.95; CI 0.90-1.0; $p=2.0 \times 10^{-54}$) than Gleason score alone, which in this cohort displayed a relatively low C-index (C-index = 0.62; CI 0.34-.89; $p=0.198$), while the 3-gene prognostic panel significantly improved the prognostic accuracy of the Gleason score (C-index=0.82; CI 0.70-0.94; $p=1.0 \times 10^{-7}$) (Fig. 4C). Additionally, multivariate Cox proportional hazard analyses showed that the 3-gene prognostic panel together with Gleason had improved prognostic ability (*p* = 0.034) over using Gleason alone (Fig. 4C). Taken together, these findings demonstrate that the 3-gene prognostic panel (herein the "prognostic panel") can accurately stratify low Gleason score primary prostate tumors at both the mRNA and protein levels, and provides independent prognostic information that improves the predictions of widely-utilized clinical nomograms.

Specifically indolent prostate cancer expresses normal or elevated levels of the prognostic panel genes compared to normal prostate while aggressive prostate tumors express significantly lower levels (about 2-fold or less).

F. Prognostic capability of the 3-gene prognostic panel on biopsy samples from surveillance patients

Analyses of protein expression of the 3-gene prognostic panel was done to determine if it could be effectively incorporated into clinical diagnosis of patients with low Gleason score prostate cancer (Fig. 1, Step 5). Toward this end, a retrospective analyses was performed of biopsy specimens from patients who had been monitored by surveillance in the Department of Urology at Columbia University Medical Center from 1992 to 2012 (35). In particular, a cohort of patients was assembled that had presented with clinically-low risk prostate cancer as defined by: normal digital rectal exam (DRE), serum PSA <10 ng/ml, biopsy Gleason score ≤ 6 in no more than 2 cores, and cancer involving no more than 50% of any core on at least a 12-core biopsy (35). The protocol to monitor these patients included DRE and serum PSA testing every three months, and repeat biopsy every 12 months for the first three years and every 18 months for the next three years, or a "for-cause" biopsy if there was any sign of progression (*i.e.*, abnormal DRE, increasing PSA). As long as all parameters and biopsy findings remained stable, patients were advised to remain on the surveillance protocol (and are referred to here as "non-failed"). Patients were considered "failure" for surveillance if they showed increasing cancer grade or volume on biopsy. Notably, all patients included in the "failed" group herein had "failed" based on these defined clinical parameters and not, for example, those who opted to undergo treatment for other reasons such as anxiety about having an untreated cancer, etc.

From a consecutive series of 213 patients that strictly adhered to the above criteria, all patients were identified that "failed" surveillance for which the *initial* biopsy tissue was available (n = 14) (Table 1). For comparison, an equivalent group of patients was analyzed that did not fail surveillance for at least ten years for which *initial* biopsy tissue was available (n = 29) (Table 1). Note that in both cases the *initial* biopsies used to enroll the patients to surveillance monitoring were evaluated.

Immunohistochemical analyses of these "failed" and "non-failed" groups of biopsy samples showed a striking correlation between the expression of FGFR1, PMP22 and CDKN1A and outcome (Fig. 4D, E; Fig. 11). In particular, all of the biopsies from the Gleason 6 patients that did not fail surveillance had robust and fairly uniform levels of expression of FGFR1, PMP22 and CDKN1A (average composite staining score of 4.11 ± 1.0). In striking contrast, the biopsies from the Gleason 6 patients that had failed active surveillance had reduced staining overall, as well as much more variable levels of FGFR1, PMP22 and CDKN1A (average composite staining score of 1.71 ± 1.2). Notably, the difference in the protein expression levels of the 3-gene prognostic panel (FGFR1, PMP22 and CDKN1A) in these Gleason 6 biopsy samples from patients that had "failed" or had "not-failed" surveillance was highly significant (t test *p* value = $1.5 \times 10^{-5}$), showing that expression levels of this 3-gene prognostic panel can be used as a prognostic indicator for these low Gleason score prostate tumors.

In certain embodiments, detection of FGFR1, PMP22 and CDKN1A on biopsy samples is used, in conjunction with other clinical parameters, to identify the subset of patients with low Gleason score prostate tumors that are likely to progress to aggressive disease and to monitor indolent tumors on active surveillance protocols.

CDKN1A (p21) is a cell-cycle regulatory gene whose expression is closely linked to senescence, whose down-regulation has been associated with promoting cancer progression in general, including prostate cancer (37, 38). The findings showing that CDKN1A (p21) expression is associated with indolence are consistent with previous studies. In contrast, the findings showing that expression of FGFR1 is associated with indolence was unexpected. FGFR1 is the major receptor for FGF growth factor signaling in the prostate and known to play a critical role in prostate development as well as prostate tumorigenesis (39, 40). Based on previous analyses of its functional role in cancer, including a recent study that evaluated the functional consequences of FGFR1 in a mutant mouse model of lethal prostate cancer (41), it might have been predicted that elevated expression of FGFR1 should be associated with cancer progression, rather than indolence. However, the complexity of FGFR1 status in prostate cancer is highlighted by the fact that while a subset of aggressive, castration-resistant prostate tumors have been shown to display amplification of the gene locus including FGFR1 (42), in the Taylor dataset, the specific genomic region that includes FGFR1 is frequently deleted, which is correlated with down-regulation of FGFR1 gene expression (14).

**References**

1. A. Jemal, R. Siegel, J. Xu, E. Ward, Cancer statistics, 2010, *CA: a cancer journal for clinicians* **60**, 277 (2010).

2. M. R. Cooperberg, J. M. Broering, P. W. Kantoff, P. R. Carroll, Contemporary trends in low risk prostate cancer: risk assessment and treatment, The Journal of urology 178, S14 (2007).

3. H. G. Welch, P. C. Albertsen, Prostate cancer diagnosis and treatment after the introduction of prostate-specific antigen screening: 1986-2005, Journal of the National Cancer Institute 101, 1325 (2009).

4. J. R. Prensner, M. A. Rubin, J. T. Wei, A. M. Chinnaiyan, Beyond PSA: the next generation of prostate cancer biomarkers, Science translational medicine 4, 127rv3 (2012).

5. D. F. Gleason, Histologic grading of prostate cancer: a perspective, Human pathology 23, 273 (1992).

6. T. J. Wilt, R. MacDonald, I. Rutks, T. A. Shamliyan, B. C. Taylor, R. L. Kane, Systematic review: comparative effectiveness and harms of treatments for clinically localized prostate cancer, Annals of internal medicine 148, 435 (2008).

7. T. J. Daskivich, K. Chamie, L. Kwan, J. Labo, R. Palvolgyi, A. Dash, S. Greenfield, M. S. Litwin, Overtreatment of men with low-risk prostate cancer and significant comorbidity, Cancer 117, 2058 (2011).

8. H. G. Welch, W. C. Black, Overdiagnosis in cancer, Journal of the National Cancer Institute 102, 605 (2010).

9. B. B. Cantrell, D. P. DeKlerk, J. C. Eggleston, J. K. Boitnott, P. C. Walsh, Pathological factors that influence prognosis in stage A prostatic cancer: the influence of extent versus grade, The Journal of urology 125, 516 (1981).

10. M. R. Cooperberg, P. R. Carroll, L. Klotz, Active surveillance for prostate cancer: progress and promise, Journal of clinical oncology : official journal of the American Society of Clinical Oncology 29, 3669 (2011).

11. J. H. Hayes, D. A. Ollendorf, S. D. Pearson, M. J. Barry, P. W. Kantoff, S. T. Stewart, V. Bhatnagar, C. J. Sweeney, J. E. Stahl, P. M. McMahon, Active surveillance compared with initial treatment for men with low-risk prostate cancer: a decision analysis, JAMA : the journal of the American Medical Association 304, 2373 (2010).

12. J. J. Tosoian, B. J. Trock, P. Landis, Z. Feng, J. I. Epstein, A. W. Partin, P. C. Walsh, H. B. Carter, Active surveillance program for prostate cancer: an update of the Johns Hopkins experience, Journal of clinical oncology : official journal of the American Society of Clinical Oncology 29, 2185 (2011).

13. M. M. Shen, C. Abate-Shen, Molecular genetics of prostate cancer: new prospects for old challenges, Genes Dev 24, 1967 (2010).

14. B. S. Taylor, N. Schultz, H. Hieronymus, A. Gopalan, Y. Xiao, B. S. Carver, V. K. Arora, P. Kaushik, E. Cerami, B. Reva, Y. Antipin, N. Mitsiades, T. Landers, I. Dolgalev, J. E. Major, M. Wilson, N. D. Socci, A. E. Lash, A. Heguy, J. A. Eastham, H. I. Scher, V. E. Reuter, P. T. Scardino, C. Sander, C. L. Sawyers, W. L. Gerald, Integrative genomic profiling of human prostate cancer, Cancer cell 18, 11 (2010).

15. M. Narita, S. W. Lowe, Senescence comes of age, Nature medicine 11, 920 (2005).

16. J. Campisi, Senescent cells, tumor suppression, and organismal aging: good citizens, bad neighbors, Cell 120, 513 (2005).

17. J. Campisi, Aging, tumor suppression and cancer: high wire-act!, Mechanisms of ageing and development 126, 51 (2005).

18. M. Collado, M. A. Blasco, M. Serrano, Cellular senescence in cancer and aging, Cell 130, 223 (2007).

19. J. Choi, I. Shendrik, M. Peacocke, D. Peehl, R. Buttyan, E. F. Ikeguchi, A. E. Katz, M. C. Benson, Expression of senescence-associated beta-galactosidase in enlarged prostates from men with benign prostatic hyperplasia, Urology 56, 160 (2000).

20. P. Castro, D. Giri, D. Lamb, M. Ittmann, Cellular senescence in the pathogenesis of benign prostatic hyperplasia, The Prostate 55, 30 (2003).

21. Z. Chen, L. C. Trotman, D. Shaffer, H. K. Lin, Z. A. Dotan, M. Niki, J. A. Koutcher, H. I. Scher, T. Ludwig, W. Gerald, C. Cordon-Cardo, P. P. Pandolfi, Crucial role of p53-dependent cellular senescence in suppression of Pten-deficient tumorigenesis, Nature 436, 725 (2005).

22. J. P. de Magalhaes, J. Curado, G. M. Church, Meta-analysis of age-related gene expression profiles identifies common signatures of aging, Bioinformatics 25, 875 (2009).

23. P. Wirapati, C. Sotiriou, S. Kunkel, P. Farmer, S. Pradervand, B. Haibe-Kains, C. Desmedt, M. Ignatiadis, T. Sengstag, F. Schutz, D. R. Goldstein, M. Piccart, M. Delorenzi, Meta-analysis of gene expression profiles in breast cancer: toward a unified understanding of breast cancer subtyping and prognosis signatures, Breast cancer research : BCR 10, R65 (2008).

24. J. Cuzick, G. P. Swanson, G. Fisher, A. R. Brothman, D. M. Berney, J. E. Reid, D. Mesher, V. O. Speights, E. Stankiewicz, C. S. Foster, H. Moller, P. Scardino, J. D. Warren, J. Park, A. Younus, D. D. Flake, 2nd, S. Wagner, A. Gutin, J. S. Lanchbury, S. Stone, Prognostic value of an RNA expression signature derived from cell cycle proliferation genes in patients with prostate cancer: a retrospective study, The lancet oncology 12, 245 (2011).

25. Y. P. Yu, D. Landsittel, L. Jing, J. Nelson, B. Ren, L. Liu, C. McDonald, R. Thomas, R. Dhir, S. Finkelstein, G. Michalopoulos, M. Becich, J. H. Luo, Gene expression alterations in prostate cancer predicting tumor aggression and preceding development of malignancy, Journal of clinical oncology : official journal of the American Society of Clinical Oncology 22, 2790 (2004).

26. A. Bhattacharjee, W. G. Richards, J. Staunton, C. Li, S. Monti, P. Vasa, C. Ladd, J. Beheshti, R. Bueno, M. Gillette, M. Loda, G. Weber, E. J. Mark, E. S. Lander, W. Wong, B. E. Johnson, T. R. Golub, D. J. Sugarbaker, M. Meyerson, Classification of human lung carcinomas by mRNA expression profiling reveals distinct adenocarcinoma subclasses, Proceedings of the National Academy of Sciences of the United States of America 98, 13790 (2001).

27. Comprehensive molecular portraits of human breast tumours, Nature 490, 61 (2012).

28. R. Bhatia-Gaur, A. A. Donjacour, P. J. Sciavolino, M. Kim, N. Desai, P. Young, C. R. Norton, T. Gridley, R. D. Cardiff, G. R. Cunha, C. Abate-Shen, M. M. Shen, Roles for Nkx3.1 in prostate development and cancer, Genes Dev 13, 966 (1999).

29. M. J. Kim, R. Bhatia-Gaur, W. A. Banach-Petrosky, N. Desai, Y. Wang, S. W. Hayward, G. R. Cunha, R. D. Cardiff, M. M. Shen, C. Abate-Shen, Nkx3.1 mutant mice recapitulate early stages of prostate carcinogenesis, Cancer Res 62, 2999 (2002).

30. C. Abate-Shen, M. M. Shen, E. Gelmann, Integrating differentiation and cancer: the Nkx3.1 homeobox gene in prostate organogenesis and carcinogenesis, Differentiation 76, 717 (2008).

31. X. Ouyang, T. L. DeWeese, W. G. Nelson, C. Abate-Shen, Loss-of-function of Nkx3.1 promotes increased oxidative damage in prostate carcinogenesis, Cancer Res 65, 6773 (2005).

32. A. G. Sowalsky, H. Ye, G. J. Bubley, S. P. Balk, Clonal progression of prostate cancers from Gleason grade 3 to grade 4, Cancer Res 73, 1050 (2013).

33. A. Sboner, F. Demichelis, S. Calza, Y. Pawitan, S. R. Setlur, Y. Hoshida, S. Perner, H. O. Adami, K. Fall, L. A. Mucci, P. W. Kantoff, M. Stampfer, S. O. Andersson, E. Varenhorst, J. E. Johansson, M. B. Gerstein, T. R. Golub, M. A. Rubin, O. Andren, Molecular sampling of prostate cancer: a dilemma for predicting disease progression, BMC medical genomics 3, 8 (2010).

34. A. V. D'Amico, R. Whittington, S. B. Malkowicz, D. Schultz, K. Blank, G. A. Broderick, J. E. Tomaszewski, A. A. Renshaw, I. Kaplan, C. J. Beard, A. Wein, Biochemical outcome after radical prostatectomy, external beam radiation therapy, or interstitial radiation therapy for clinically localized prostate cancer, JAMA : the journal of the American Medical Association 280, 969 (1998).

35. P. Motamedinia, J. L. Richard, J. M. McKiernan, G. J. Decastro, M. C. Benson, Role of immediate confirmatory prostate biopsy to ensure accurate eligibility for active surveillance, Urology 80, 1070 (2012).

36. J. Campisi, Cancer and ageing: rival demons?, Nature reviews. Cancer 3, 339 (2003).

37. S. Roy, R. P. Singh, C. Agarwal, S. Siriwardana, R. Sclafani, R. Agarwal, Downregulation of both p21/Cip1 and p27/Kip1 produces a more aggressive prostate cancer phenotype, Cell Cycle 7, 1828 (2008).

38. T. Abbas, A. Dutta, p21 in cancer: intricate networks and multiple activities, Nature reviews. Cancer 9, 400 (2009).

39. V. D. Acevedo, M. Ittmann, D. M. Spencer, Paths of FGFR-driven tumorigenesis, Cell Cycle 8, 580 (2009).

40. N. Turner, R. Grose, Fibroblast growth factor signalling: from development to cancer, Nature reviews. Cancer 10, 116 (2010).

41. F. Yang, Y. Zhang, S. J. Ressler, M. M. Ittmann, G. E. Ayala, T. D. Dang, F. Wang, D. R. Rowley, FGFR1 is Essential for Prostate Cancer Progression and Metastasis, Cancer Res, (2013).

42. J. Edwards, N. S. Krishna, C. J. Witton, J. M. Bartlett, Gene amplifications associated with the development of hormone-resistant prostate cancer, Clinical cancer research : an official journal of the American Association for Cancer Research 9, 5271 (2003).

43. G. Meyer Zu Horste, K. A. Nave, Animal models of inherited neuropathies, Current opinion in neurology 19, 464 (2006).

44. K. Adlkofer, R. Martini, A. Aguzzi, J. Zielasek, K. V. Toyka, U. Suter, Hypermyelination and demyelinating peripheral neuropathy in Pmp22-deficient mice, Nature genetics 11, 274 (1995).

45. U. Suter, G. J. Snipes, Peripheral myelin protein 22: facts and hypotheses, Journal of neuroscience research 40, 145 (1995).

46. Z. Ding, C. J. Wu, G. C. Chu, Y. Xiao, D. Ho, J. Zhang, S. R. Perry, E. S. Labrot, X. Wu, R. Lis, Y. Hoshida, D. Hiller, B. Hu, S. Jiang, H. Zheng, A. H. Stegh, K. L. Scott, S. Signoretti, N. Bardeesy, Y. A. Wang, D. E. Hill, T. R. Golub, M. J. Stampfer, W. H. Wong, M. Loda, L. Mucci, L. Chin, R. A. DePinho, SMAD4-dependent barrier constrains prostate cancer growth and metastatic progression, Nature 470, 269 (2011).

47. E. K. Markert, H. Mizuno, A. Vazquez, A. J. Levine, Molecular classification of prostate cancer using curated expression signatures, Proceedings of the National Academy of Sciences of the United States of America 108, 21276 (2011).

48. S. A. Tomlins, S. M. Aubin, J. Siddiqui, R. J. Lonigro, L. Sefton-Miller, S. Miick, S. Williamsen, P. Hodge, J. Meinke, A. Blase, Y. Penabella, J. R. Day, R. Varambally, B. Han, D. Wood, L. Wang, M. G. Sanda, M. A. Rubin, D. R. Rhodes, B. Hollenbeck, K. Sakamoto, J. L. Silberstein, Y. Fradet, J. B. Amberson, S. Meyers, N. Palanisamy, H. Rittenhouse, J. T. Wei, J. Groskopf, A. M. Chinnaiyan, Urine TMPRSS2:ERG fusion transcript stratifies prostate cancer risk in men with elevated serum PSA, Science translational medicine 3, 94ra72 (2011).

49. D. Olmos, D. Brewer, J. Clark, D. C. Danila, C. Parker, G. Attard, M. Fleisher, A. H. Reid, E. Castro, S. K. Sandhu, L. Barwell, N. B. Oommen, S. Carreira, C. G. Drake, R. Jones, C. S. Cooper, H. I. Scher, J. S. de Bono, Prognostic value of blood mRNA expression signatures in castration-resistant prostate cancer: a prospective, two-stage study, The lancet oncology 13, 1114 (2012).

50. M. Braig, S. Lee, C. Loddenkemper, C. Rudolph, A. H. Peters, B. Schlegelberger, H. Stein, B. Dorken, T. Jenuwein, C. A. Schmitt, Oncogene-induced senescence as an initial barrier in lymphoma development, Nature 436, 660 (2005).

51. M. Collado, J. Gil, A. Efeyan, C. Guerra, A. J. Schuhmacher, M. Barradas, A. Benguria, A. Zaballos, J. M. Flores, M. Barbacid, D. Beach, M. Serrano, Tumour biology: senescence in premalignant tumours, Nature 436, 642 (2005).

52. M. Malumbres, I. Perez De Castro, M. I. Hernandez, M. Jimenez, T. Corral, A. Pellicer, Cellular response to oncogenic ras involves induction of the Cdk4 and Cdk6 inhibitor p15(INK4b), Mol Cell Biol 20, 2915 (2000).

53. A. Subramanian, P. Tamayo, V. K. Mootha, S. Mukherjee, B. L. Ebert, M. A. Gillette, A. Paulovich, S. L. Pomeroy, T. R. Golub, E. S. Lander, J. P. Mesirov, Gene set enrichment analysis: a knowledge-based approach for interpreting genome-wide expression profiles, Proceedings of the National Academy of Sciences of the United States of America 102, 15545 (2005).

54. M. J. Pencina, R. B. D'Agostino, Overall C as a measure of discrimination in survival analysis: model specific population value and confidence interval estimation, Statistics in medicine 23, 2109 (2004).

55. R. A. Irizarry, B. Hobbs, F. Collin, Y. D. Beazer-Barclay, K. J. Antonellis, U. Scherf, T. P. Speed, Exploration, normalization, and summaries of high density oligonucleotide array probe level data, Biostatistics 4, 249 (2003).

56. Z. Wu, R. Irizarry, R. Gentleman, F. M. Murillo, F. Spencer, A Model Based Background Adjustment for Oligonucleotide Expression Arrays, Johns Hopkins University, Dept. of Biostatistics Working Papers, (2004).

57. V. K. Mootha, C. M. Lindgren, K. F. Eriksson, A. Subramanian, S. Sihag, J. Lehar, P. Puigserver, E. Carlsson, M. Ridderstrale, E. Laurila, N. Houstis, M. J. Daly, N. Patterson, J. P. Mesirov, T. R. Golub, P. Tamayo, B. Spiegelman, E. S. Lander, J. N. Hirschhorn, D. Altshuler, L. C. Groop, PGC-1alpha-responsive genes involved in oxidative phosphorylation are coordinately downregulated in human diabetes, Nature genetics 34, 267 (2003).

58. G. A. F. Seber, Multivariate Observations. (John Wiley & Sons, Inc., Hoboken, NJ, 1984).

59. C. W. Kinkade, M. Castillo-Martin, A. Puzio-Kuter, J. Yan, T. H. Foster, H. Gao, Y. Sun, X. Ouyang, W. L. Gerald, C. Cordon-Cardo, C. Abate-Shen, Targeting AKT/mTOR and ERK MAPK signaling inhibits hormone-refractory prostate cancer in a preclinical mouse model, The Journal of clinical investigation 118, 3051 (2008).

| Table 1: Description of the 377 genes in the aging and senescence signature | | | | | | |
|---|---|---|---|---|---|---|
| Entrez ID | Gene Symbol | HyperLink | #NAME? | Meta-analysis (aging-related) de Magalhaes et al, 2009 | Ingenuity pathway analysis (senescence related) http://www.ingenuity.com/ | Manual curation (senescence related) Malumbres et al, 2000; Collado et al, 2005; Braig et al, 2005; Collado et al, 2005 |
| 55902 | ACSS2 | ACSS2 | acyl-CoA s | ✓ | - | - |
| 4185 | ADAM11 | ADAM11 | ADAM met | ✓ | - | - |
| 81794 | ADAMTS1( | ADAMTS1 | ADAM met | ✓ | - | - |
| 108 | ADCY2 | ADCY2 | adenylate ( | ✓ | - | - |
| 128 | ADH5 | ADH5 | alcohol del | ✓ | - | - |
| 79602 | ADIPOR2 | ADIPOR2 | adiponectir | ✓ | - | - |
| 121536 | AEBP2 | AEBP2 | AE binding | ✓ | - | - |
| 4299 | AFF1 | AFF1 | AF4/FMR2 | ✓ | - | - |
| 79026 | AHNAK | AHNAK | AHNAK nu | ✓ | - | - |
| 84883 | AIFM2 | AIFM2 | apoptosis-i | ✓ | - | - |
| 11214 | AKAP13 | AKAP13 | A kinase (F | ✓ | - | - |
| 126133 | ALDH16A1 | ALDH16A1 | aldehyde d | ✓ | - | - |
| 51421 | AMOTL2 | AMOTL2 | angiomotin | ✓ | - | - |
| 93550 | ANUBL1 | ANUBL1 | AN1, ubiqu | ✓ | - | - |
| 306 | ANXA3 | ANXA3 | annexin A3 | ✓ | - | - |
| 307 | ANXA4 | ANXA4 | annexin A4 | ✓ | - | - |
| 308 | ANXA5 | ANXA5 | annexin A5 | ✓ | - | - |
| 347 | APOD | APOD | apolipoprot | ✓ | - | - |
| 351 | APP | APP | amyloid be | - | ✓ | - |
| 382 | ARF6 | ARF6 | ADP-ribosy | ✓ | - | - |
| 115761 | ARL11 | ARL11 | ADP-ribosy | ✓ | - | - |
| 81873 | ARPC5L | ARPC5L | actin relate | ✓ | - | - |
| 443 | ASPA | ASPA | aspartoacy | ✓ | - | - |
| 445 | ASS1 | ASS1 | argininosuc | ✓ | - | - |
| 466 | ATF1 | ATF1 | activating t | ✓ | - | - |
| 472 | ATM | ATM | ataxia telar | - | ✓ | - |
| 482 | ATP1B2 | ATP1B2 | ATPase, N | ✓ | - | - |
| 498 | ATP5A1 | ATP5A1 | ATP syntha | ✓ | - | - |
| 509 | ATP5C1 | ATP5C1 | ATP syntha | ✓ | - | - |

| | | | | | | |
|---|---|---|---|---|---|---|
| 515 | ATP5F1 | ATP5F1 | ATP syntha | ✓ | - | - |
| 516 | ATP5G1 | ATP5G1 | ATP syntha | ✓ | - | - |
| 518 | ATP5G3 | ATP5G3 | ATP syntha | ✓ | - | - |
| 522 | ATP5J | ATP5J | ATP syntha | ✓ | - | - |
| 545 | ATR | ATR | ataxia telar | - | ✓ | - |
| 8313 | AXIN2 | AXIN2 | axin 2 | ✓ | - | - |
| 567 | B2M | B2M | beta-2-micr | ✓ | - | - |
| 23786 | BCL2L13 | BCL2L13 | BCL2-like 1 | ✓ | - | - |
| 633 | BGN | BGN | biglycan | ✓ | - | - |
| 641 | BLM | BLM | Bloom synd | - | ✓ | - |
| 648 | BMI1 | BMI1 | BMI1 polyc | - | ✓ | - |
| 653 | BMP5 | BMP5 | bone morpl | ✓ | - | - |
| 672 | BRCA1 | BRCA1 | breast canc | - | ✓ | - |
| 55108 | BSDC1 | BSDC1 | BSD doma | ✓ | - | - |
| 9184 | BUB3 | BUB3 | BUB3 budd | - | ✓ | - |
| 79864 | C11orf63 | C11orf63 | chromoson | ✓ | - | - |
| 55196 | C12orf35 | C12orf35 | chromoson | ✓ | - | - |
| 79622 | C16orf33 | C16orf33 | chromoson | ✓ | - | - |
| 712 | C1QA | C1QA | complemer | ✓ | - | - |
| 713 | C1QB | C1QB | complemer | ✓ | - | - |
| 714 | C1QC | C1QC | complemer | ✓ | - | - |
| 715 | C1R | C1R | complemer | ✓ | - | - |
| 716 | C1S | C1S | complemer | ✓ | - | - |
| 116151 | C20orf108 | C20orf108 | chromoson | ✓ | - | - |
| 8209 | C21orf33 | C21orf33 | chromoson | ✓ | - | - |
| 718 | C3 | C3 | complemer | ✓ | - | - |
| 720 | C4A | C4A | Compleme | ✓ | - | - |
| 85438 | C4orf35 | C4orf35 | chromoson | ✓ | - | - |
| 9315 | C5orf13 | C5orf13 | chromoson | ✓ | - | - |
| 221545 | C6orf136 | C6orf136 | chromoson | ✓ | - | - |
| 79017 | C7orf24 | C7orf24 | gamma-glu | ✓ | - | - |
| 84302 | C9orf125 | C9orf125 | chromoson | ✓ | - | - |
| 79095 | C9orf16 | C9orf16 | chromoson | ✓ | - | - |
| 762 | CA4 | CA4 | carbonic ar | ✓ | - | - |
| 23705 | CADM1 | CADM1 | cell adhesi | ✓ | - | - |
| 793 | CALB1 | CALB1 | calbindin 1 | ✓ | - | - |
| 794 | CALB2 | CALB2 | calbindin 2 | ✓ | - | - |
| 847 | CAT | CAT | catalase | - | ✓ | - |
| 1235 | CCR6 | CCR6 | chemokine | - | - | ✓ |
| 963 | CD53 | CD53 | CD53 mole | ✓ | - | - |

| 967 | CD63 | CD63 | CD63 mole | ✓ | - | - |
|---|---|---|---|---|---|---|
| 968 | CD68 | CD68 | CD68 mole | ✓ | - | - |
| 972 | CD74 | CD74 | CD74 mole | ✓ | - | - |
| 3732 | CD82 | CD82 | CD82 mole | ✓ | - | - |
| 928 | CD9 | CD9 | CD9 molec | ✓ | - | - |
| 990 | CDC6 | CDC6 | cell division | ✓ | - | - |
| 999 | CDH1 | CDH1 | cadherin 1, | ✓ | - | - |
| 1026 | CDKN1A | CDKN1A | cyclin-depe | ✓ | ✓ | - |
| 1029 | CDKN2A | CDKN2A | cyclin-depe | - | ✓ | - |
| 1030 | CDKN2B | CDKN2B | cyclin-depe | - | - | ✓ |
| 1051 | CEBPB | CEBPB | CCAAT/en | - | ✓ | - |
| 3075 | CFH | CFH | complemer | ✓ | - | - |
| 11200 | CHEK2 | CHEK2 | CHK2 chec | - | ✓ | - |
| 1134 | CHRNA1 | CHRNA1 | cholinergic | ✓ | - | - |
| 10462 | CLEC10A | CLEC10A | C-type lect | ✓ | - | - |
| 6320 | CLEC11A | CLEC11A | C-type lect | ✓ | - | - |
| 25932 | CLIC4 | CLIC4 | chloride int | ✓ | - | - |
| 1191 | CLU | CLU | clusterin | ✓ | - | - |
| 1306 | COL15A1 | COL15A1 | collagen, ty | ✓ | - | - |
| 80781 | COL18A1 | COL18A1 | collagen, ty | ✓ | - | - |
| 1277 | COL1A1 | COL1A1 | collagen, ty | ✓ | - | - |
| 1281 | COL3A1 | COL3A1 | collagen, ty | ✓ | - | - |
| 1287 | COL4A5 | COL4A5 | collagen, ty | ✓ | - | - |
| 1289 | COL5A1 | COL5A1 | collagen, ty | ✓ | - | - |
| 1290 | COL5A2 | COL5A2 | collagen, ty | ✓ | - | - |
| 1312 | COMT | COMT | catechol-O | ✓ | - | - |
| 51004 | COQ6 | COQ6 | coenzyme | ✓ | - | - |
| 1351 | COX8A | COX8A | cytochrome | ✓ | - | - |
| 1356 | CP | CP | ceruloplasr | ✓ | - | - |
| 1393 | CRHBP | CRHBP | corticotropi | ✓ | - | - |
| 1410 | CRYAB | CRYAB | crystallin, a | ✓ | - | - |
| 1453 | CSNK1D | CSNK1D | casein kina | ✓ | - | - |
| 1465 | CSRP1 | CSRP1 | cysteine ar | ✓ | - | - |
| 1466 | CSRP2 | CSRP2 | cysteine ar | ✓ | - | - |
| 1509 | CTSD | CTSD | cathepsin [ | ✓ | - | - |
| 1512 | CTSH | CTSH | cathepsin H | ✓ | - | - |
| 1520 | CTSS | CTSS | cathepsin S | ✓ | - | - |
| 1522 | CTSZ | CTSZ | cathepsin Z | ✓ | - | - |
| 6376 | CX3CL1 | CX3CL1 | chemokine | ✓ | - | - |
| 58191 | CXCL16 | CXCL16 | chemokine | ✓ | - | - |

| 1620 | DBC1 | DBC1 | deleted in k | ✓ | - | - |
|---|---|---|---|---|---|---|
| 28960 | DCPS | DCPS | decapping | ✓ | - | - |
| 11258 | DCTN3 | DCTN3 | dynactin 3 | ✓ | - | - |
| 54541 | DDIT4 | DDIT4 | DNA-dama | ✓ | - | - |
| 7913 | DEK | DEK | DEK oncod | ✓ | - | - |
| 79139 | DERL1 | DERL1 | Der1-like d | ✓ | - | - |
| 56616 | DIABLO | DIABLO | diablo hom | ✓ | - | - |
| 3300 | DNAJB2 | DNAJB2 | DnaJ (Hsp | ✓ | - | - |
| 29103 | DNAJC15 | DNAJC15 | DnaJ (Hsp | ✓ | - | - |
| 113878 | DTX2 | DTX2 | Deltex hom | ✓ | - | - |
| 151636 | DTX3L | DTX3L | deltex 3-lik | ✓ | - | - |
| 1778 | DYNC1H1 | DYNC1H1 | Dynein, cyt | ✓ | - | - |
| 1869 | E2F1 | E2F1 | E2F transc | - | ✓ | - |
| 1889 | ECE1 | ECE1 | endothelin | ✓ | - | - |
| 2202 | EFEMP1 | EFEMP1 | EGF-conta | ✓ | - | - |
| 1958 | EGR1 | EGR1 | early growt | - | ✓ | - |
| 30845 | EHD3 | EHD3 | EH-domain | ✓ | - | - |
| 2006 | ELN | ELN | elastin | ✓ | - | - |
| 2033 | EP300 | EP300 | E1A bindin | - | ✓ | - |
| 80314 | EPC1 | EPC1 | enhancer o | ✓ | - | - |
| 2160 | F11 | F11 | coagulatior | ✓ | - | - |
| 2170 | FABP3 | FABP3 | fatty acid b | ✓ | - | - |
| 11170 | FAM107A | FAM107A | family with | ✓ | - | - |
| 54463 | FAM134B | FAM134B | family with | ✓ | - | - |
| 404636 | FAM45A | FAM45A | Family with | ✓ | - | - |
| 137392 | FAM92A1 | FAM92A1 | family with | ✓ | - | - |
| 25940 | FAM98A | FAM98A | family with | ✓ | - | - |
| 2203 | FBP1 | FBP1 | fructose-1, | ✓ | - | - |
| 2212 | FCGR2A | FCGR2A | Fc fragmen | ✓ | - | - |
| 2213 | FCGR2B | FCGR2B | Fc fragmen | ✓ | - | - |
| 2214 | FCGR3A | FCGR3A | Fc fragmen | ✓ | - | - |
| 83706 | FERMT3 | FERMT3 | fermitin fan | ✓ | - | - |
| 2260 | FGFR1 | FGFR1 | fibroblast g | - | ✓ | - |
| 2271 | FH | FH | fumarate h | ✓ | - | - |
| 54621 | FLJ20674 | FLJ20674 | hypothetica | ✓ | - | - |
| 64926 | FLJ21438 | FLJ21438 | hypothetica | ✓ | - | - |
| 728772 | FLJ77644 | FLJ77644 | hypothetica | ✓ | - | - |
| 2321 | FLT1 | FLT1 | fms-related | - | ✓ | - |
| 2335 | FN1 | FN1 | fibronectin | ✓ | - | - |
| 64838 | FNDC4 | FNDC4 | fibronectin | ✓ | - | - |

32

| | | | | | | |
|---|---|---|---|---|---|---|
| 442425 | FOXB2 | FOXB2 | Forkhead b | ✓ | - | - |
| 2305 | FOXM1 | FOXM1 | forkhead bc | - | ✓ | - |
| 5348 | FXYD1 | FXYD1 | FXYD dom | ✓ | - | - |
| 486 | FXYD2 | FXYD2 | FXYD dom | ✓ | - | - |
| 2571 | GAD1 | GAD1 | glutamate c | ✓ | - | - |
| 2628 | GATM | GATM | glycine ami | ✓ | - | - |
| 57704 | GBA2 | GBA2 | glucosidase | ✓ | - | - |
| 2634 | GBP2 | GBP2 | guanylate b | ✓ | - | - |
| 2670 | GFAP | GFAP | glial fibrillar | ✓ | - | - |
| 2675 | GFRA2 | GFRA2 | GDNF fami | ✓ | - | - |
| 27069 | GHITM | GHITM | growth horr | ✓ | - | - |
| 2696 | GIPR | GIPR | gastric inhi | ✓ | - | - |
| 51228 | GLTP | GLTP | glycolipid tr | ✓ | - | - |
| 2799 | GNS | GNS | glucosamin | ✓ | - | - |
| 2805 | GOT1 | GOT1 | glutamic-ox | ✓ | - | - |
| 2806 | GOT2 | GOT2 | glutamic-ox | ✓ | - | - |
| 10457 | GPNMB | GPNMB | glycoprotei | ✓ | - | - |
| 7107 | GPR137B | GPR137B | G protein-c | ✓ | - | - |
| 9737 | GPRASP1 | GPRASP1 | G protein-c | ✓ | - | - |
| 2878 | GPX3 | GPX3 | glutathione | ✓ | - | - |
| 2896 | GRN | GRN | granulin | ✓ | - | - |
| 2938 | GSTA1 | GSTA1 | glutathione | ✓ | - | - |
| 3020 | H3F3A | H3F3A | H3 histone | ✓ | - | ✓ |
| 10456 | HAX1 | HAX1 | HCLS1 ass | ✓ | - | - |
| 3039 | HBA1 | HBA1 | Hemoglobi | ✓ | - | ✓ |
| 3043 | HBB | HBB | Hemoglobi | ✓ | - | - |
| 10870 | HCST | HCST | hematopoie | ✓ | - | - |
| 3066 | HDAC2 | HDAC2 | histone dea | - | ✓ | - |
| 84064 | HDHD2 | HDHD2 | haloacid de | ✓ | - | - |
| 3070 | HELLS | HELLS | helicase, ly | - | ✓ | - |
| 3006 | HIST1H1C | HIST1H1C | histone clus | ✓ | - | - |
| 3109 | HLA-DMB | HLA-DMB | major histo | ✓ | - | - |
| 3117 | HLA-DQA1 | HLA-DQA1 | major histo | ✓ | - | - |
| 3122 | HLA-DRA | HLA-DRA | major histo | ✓ | - | - |
| 3134 | HLA-F | HLA-F | major histo | ✓ | - | - |
| 3135 | HLA-G | HLA-G | major histo | ✓ | - | - |
| 3148 | HMGB2 | HMGB2 | high-mobili | ✓ | - | - |
| 54511 | HMGCLL1 | HMGCLL1 | 3-hydroxyn | ✓ | - | - |
| 3157 | HMGCS1 | HMGCS1 | 3-hydroxy-3 | ✓ | - | - |
| 3172 | HNF4A | HNF4A | hepatocyte | ✓ | - | - |

33

| 9987 | HNRPDL | HNRPDL | heterogene | ✓ | - | - |
|---|---|---|---|---|---|---|
| 3208 | HPCA | HPCA | hippocalcin | ✓ | - | - |
| 3265 | HRAS | HRAS | v-Ha-ras H | - | ✓ | - |
| 259217 | HSPA12A | HSPA12A | heat shock | ✓ | - | - |
| 3303 | HSPA1A | HSPA1A | Heat shock | ✓ | ✓ | - |
| 3315 | HSPB1 | HSPB1 | heat shock | ✓ | - | - |
| 3336 | HSPE1 | HSPE1 | heat shock | ✓ | - | - |
| 3459 | IFNGR1 | IFNGR1 | interferon g | ✓ | - | - |
| 3479 | IGF1 | IGF1 | insulin-like | ✓ | - | - |
| 3512 | IGJ | IGJ | immunoglo | ✓ | - | - |
| 90865 | IL33 | IL33 | interleukin | ✓ | - | - |
| 3624 | INHBA | INHBA | inhibin, bet | ✓ | - | - |
| 8826 | IQGAP1 | IQGAP1 | IQ motif co | ✓ | - | - |
| 79191 | IRX3 | IRX3 | iroquois ho | ✓ | - | - |
| 3689 | ITGB2 | ITGB2 | integrin, be | ✓ | - | - |
| 3696 | ITGB8 | ITGB8 | integrin, be | ✓ | - | - |
| 9452 | ITM2A | ITM2A | integral me | ✓ | - | - |
| 152789 | JAKMIP1 | JAKMIP1 | janus kinas | ✓ | - | - |
| 3727 | JUND | JUND | jun D proto | - | ✓ | - |
| 9813 | KIAA0494 | KIAA0494 | KIAA0494 | ✓ | - | - |
| 57650 | KIAA1524 | KIAA1524 | KIAA1524 | ✓ | - | - |
| 9314 | KLF4 | KLF4 | Kruppel-lik | ✓ | - | - |
| 8844 | KSR1 | KSR1 | kinase sup | ✓ | ✓ | - |
| 3916 | LAMP1 | LAMP1 | lysosomal- | ✓ | - | - |
| 7805 | LAPTM5 | LAPTM5 | lysosomal | ✓ | - | - |
| 84247 | LDOC1L | LDOC1L | leucine zip | ✓ | - | - |
| 3958 | LGALS3 | LGALS3 | lectin, gala | ✓ | - | - |
| 22998 | LIMCH1 | LIMCH1 | LIM and ca | ✓ | - | - |
| 9516 | LITAF | LITAF | lipopolysac | ✓ | - | - |
| 284194 | LOC28419 | LOC28419 | Lectin, gala | ✓ | - | - |
| 4057 | LTF | LTF | lactotransf | ✓ | - | - |
| 4069 | LYZ | LYZ | lysozyme ( | ✓ | - | - |
| 256691 | MAMDC2 | MAMDC2 | MAM doma | ✓ | - | - |
| 5604 | MAP2K1 | MAP2K1 | mitogen-ac | ✓ | - | - |
| 23118 | MAP3K7IP | MAP3K7IP | mitogen-ac | ✓ | - | - |
| 1432 | MAPK14 | MAPK14 | mitogen-ac | - | ✓ | - |
| 64844 | 7-Mar | MARCH7 | Membrane | ✓ | - | - |
| 4170 | MCL1 | MCL1 | myeloid cel | ✓ | - | - |
| 4190 | MDH1 | MDH1 | malate deh | ✓ | - | - |
| 4204 | MECP2 | MECP2 | methyl CpG | ✓ | - | - |

| 4257 | MGST1 | MGST1 | microsoma | ✓ | ~ | - |
|---|---|---|---|---|---|---|
| 4282 | MIF | MIF | Macrophag | ✓ | ~ | - |
| 219972 | MPEG1 | MPEG1 | macrophag | ✓ | ~ | - |
| 64981 | MRPL34 | MRPL34 | mitochondr | ✓ | ~ | - |
| 64979 | MRPL36 | MRPL36 | mitochondr | ✓ | ~ | - |
| 28973 | MRPS18B | MRPS18B | mitochondr | ✓ | ~ | - |
| 4478 | MSN | MSN | moesin | ✓ | ~ | - |
| 4493 | MT1E | MT1E | metallothio | ✓ | ~ | - |
| 4494 | MT1F | MT1F | metallothio | ✓ | ~ | - |
| 4507 | MTAP | MTAP | methylthioa | ✓ | ~ | - |
| 23788 | MTCH2 | MTCH2 | mitochondr | ✓ | ~ | - |
| 9961 | MVP | MVP | major vault | ✓ | ~ | - |
| 4609 | MYC | MYC | v-myc mye | - | ✓ | - |
| 55930 | MYO5C | MYO5C | myosin VC | ✓ | - | - |
| 10135 | NAMPT | NAMPT | nicotinamid | ✓ | ✓ | - |
| 4677 | NARS | NARS | asparaginy | ✓ | - | - |
| 10397 | NDRG1 | NDRG1 | N-myc dow | ✓ | - | - |
| 57447 | NDRG2 | NDRG2 | NDRG fam | ✓ | - | - |
| 54539 | NDUFB11 | NDUFB11 | NADH deh | ✓ | - | - |
| 4711 | NDUFB5 | NDUFB5 | NADH deh | ✓ | - | - |
| 4712 | NDUFB6 | NDUFB6 | NADH deh | ✓ | - | - |
| 4714 | NDUFB8 | NDUFB8 | NADH deh | ✓ | - | - |
| 4717 | NDUFC1 | NDUFC1 | NADH deh | ✓ | - | - |
| 4722 | NDUFS3 | NDUFS3 | NADH deh | ✓ | - | - |
| 4729 | NDUFV2 | NDUFV2 | NADH deh | ✓ | - | - |
| 4738 | NEDD8 | NEDD8 | neural prec | ✓ | - | - |
| 140609 | NEK7 | NEK7 | NIMA (nev | ✓ | - | - |
| 4780 | NFE2L2 | NFE2L2 | nuclear fac | ✓ | - | - |
| 4864 | NPC1 | NPC1 | Niemann-P | ✓ | - | - |
| 10577 | NPC2 | NPC2 | Niemann-P | ✓ | - | - |
| 79023 | NUP37 | NUP37 | nucleoporir | ✓ | - | - |
| 10215 | OLIG2 | OLIG2 | oligodendro | ✓ | - | - |
| 64805 | P2RY12 | P2RY12 | purinergic r | ✓ | - | - |
| 23022 | PALLD | PALLD | Palladin, cy | ✓ | - | - |
| 24145 | PANX1 | PANX1 | pannexin 1 | ✓ | - | - |
| 10914 | PAPOLA | PAPOLA | poly(A) pol | ✓ | - | - |
| 5046 | PCSK6 | PCSK6 | proprotein c | ✓ | - | - |
| 5138 | PDE2A | PDE2A | phosphodie | ✓ | - | - |
| 5154 | PDGFA | PDGFA | platelet-der | - | - | - |
| 8800 | PEX11A | PEX11A | Peroxisoma | ✓ | - | - |

| 5213 | PFKM | PFKM | phosphofru | ✓ | - | - |
|---|---|---|---|---|---|---|
| 5305 | PIP4K2A | PIP4K2A | phosphatid | ✓ | - | - |
| 8502 | PKP4 | PKP4 | plakophilin | ✓ | - | - |
| 5331 | PLCB3 | PLCB3 | phospholip | ✓ | - | - |
| 5341 | PLEK | PLEK | pleckstrin | ✓ | - | - |
| 5371 | PML | PML | promyelocy | - | ✓ | - |
| 5376 | PMP22 | PMP22 | peripheral | ✓ | - | - |
| 5406 | PNLIP | PNLIP | pancreatic | ✓ | - | - |
| 9588 | PRDX6 | PRDX6 | peroxiredo | ✓ | - | - |
| 9588 | PRDX6 | PRDX6 | peroxiredo | ✓ | - | - |
| 5696 | PSMB8 | PSMB8 | proteasom | ✓ | - | - |
| 5717 | PSMD11 | PSMD11 | proteasom | ✓ | - | - |
| 5717 | PSMD11 | PSMD11 | proteasom | ✓ | - | - |
| 5723 | PSPH | PSPH | phosphose | ✓ | - | - |
| 5728 | PTEN | PTEN | phosphatas | - | ✓ | - |
| 10728 | PTGES3 | PTGES3 | prostagland | ✓ | - | - |
| 2185 | PTK2B | PTK2B | PTK2B pro | ✓ | - | - |
| 51495 | PTPLAD1 | PTPLAD1 | protein tyro | ✓ | - | - |
| 5800 | PTPRO | PTPRO | protein tyro | ✓ | - | - |
| 29942 | PURG | PURG | purine-rich | ✓ | - | - |
| 54517 | PUS7 | PUS7 | pseudourid | ✓ | - | - |
| 25945 | PVRL3 | PVRL3 | poliovirus r | ✓ | - | - |
| 5828 | PXMP3 | PXMP3 | Peroxisoma | ✓ | - | - |
| 10966 | RAB40B | RAB40B | RAB40B, n | ✓ | - | - |
| 8480 | RAE1 | RAE1 | RAE1 RNA | - | ✓ | - |
| 22821 | RASA3 | RASA3 | RAS p21 p | ✓ | - | - |
| 5925 | RB1 | RB1 | retinoblasto | - | ✓ | - |
| 473 | RERE | RERE | arginine-glu | ✓ | - | - |
| 162494 | RHBDL3 | RHBDL3 | rhomboid, | ✓ | - | - |
| 9912 | RICH2 | RICH2 | Rho-type G | ✓ | - | - |
| 8780 | RIOK3 | RIOK3 | RIO kinase | ✓ | - | - |
| 8635 | RNASET2 | RNASET2 | ribonucleas | ✓ | - | - |
| 55298 | RNF121 | RNF121 | ring finger | ✓ | - | - |
| 57674 | RNF213 | RNF213 | ring finger | ✓ | - | - |
| 6096 | RORB | RORB | RAR-relate | ✓ | - | - |
| 6122 | RPL3 | RPL3 | ribosomal | ✓ | - | - |
| 6241 | RRM2 | RRM2 | ribonucleot | ✓ | - | - |
| 6281 | S100A10 | S100A10 | S100 calciu | ✓ | - | - |
| 6275 | S100A4 | S100A4 | S100 calciu | ✓ | - | - |
| 6277 | S100A6 | S100A6 | S100 calciu | ✓ | - | - |

| | | | | | | |
|---|---|---|---|:---:|:---:|:---:|
| 29901 | SAC3D1 | SAC3D1 | SAC3 dom | ✓ | - | - |
| 6385 | SDC4 | SDC4 | syndecan 4 | ✓ | - | - |
| 6390 | SDHB | SDHB | succinate d | ✓ | - | - |
| 6392 | SDHD | SDHD | succinate d | ✓ | - | - |
| 9554 | SEC22B | SEC22B | SEC22 ves | ✓ | - | - |
| 5267 | SERPINA4 | SERPINA4 | serpin pept | ✓ | - | - |
| 5269 | SERPINB6 | SERPINB6 | serpin pept | ✓ | - | - |
| 710 | SERPING1 | SERPING1 | serpin pept | ✓ | - | - |
| 11129 | SFRS16 | SFRS16 | splicing fac | ✓ | - | - |
| 6430 | SFRS5 | SFRS5 | splicing fac | ✓ | - | - |
| 6446 | SGK1 | SGK1 | serum/gluc | ✓ | - | - |
| 8879 | SGPL1 | SGPL1 | sphingosine | ✓ | - | - |
| 10603 | SH2B2 | SH2B2 | SH2B adap | ✓ | - | - |
| 51100 | SH3GLB1 | SH3GLB1 | SH3-doma | ✓ | - | - |
| 23411 | SIRT1 | SIRT1 | sirtuin (sile | - | ✓ | - |
| 8935 | SKAP2 | SKAP2 | src kinase | ✓ | - | - |
| 6558 | SLC12A2 | SLC12A2 | solute carri | ✓ | - | - |
| 292 | SLC25A5 | SLC25A5 | solute carri | ✓ | - | - |
| 6550 | SLC9A3 | SLC9A3 | solute carri | ✓ | - | - |
| 4092 | SMAD7 | SMAD7 | SMAD fam | ✓ | ✓ | - |
| 23137 | SMC5 | SMC5 | structural n | ✓ | - | - |
| 8723 | SNX4 | SNX4 | sorting nex | ✓ | - | - |
| 9021 | SOCS3 | SOCS3 | suppressor | ✓ | - | - |
| 9655 | SOCS5 | SOCS5 | suppressor | ✓ | - | - |
| 6647 | SOD1 | SOD1 | superoxide | - | ✓ | - |
| 6272 | SORT1 | SORT1 | sortilin 1 | ✓ | - | - |
| 6664 | SOX11 | SOX11 | SRY (sex d | ✓ | - | - |
| 10417 | SPON2 | SPON2 | spondin 2, | ✓ | - | - |
| 6696 | SPP1 | SPP1 | Secreted p | ✓ | - | - |
| 6794 | STK11 | STK11 | serine/thre | - | ✓ | - |
| 6814 | STXBP3 | STXBP3 | syntaxin bir | ✓ | - | - |
| 6839 | SUV39H1 | SUV39H1 | suppressor | - | - | ✓ |
| 6902 | TBCA | TBCA | tubulin fold | ✓ | - | - |
| 6929 | TCF3 | TCF3 | transcriptio | - | ✓ | - |
| 7015 | TERT | TERT | telomerase | - | ✓ | - |
| 7018 | TF | TF | transferrin | ✓ | - | - |
| 7037 | TFRC | TFRC | transferrin | ✓ | - | - |
| 7040 | TGFB1 | TGFB1 | transformin | - | ✓ | - |
| 64114 | TMBIM1 | TMBIM1 | transmemb | ✓ | - | - |
| 10972 | TMED10 | TMED10 | transmemb | ✓ | - | - |

| 55365 | TMEM176A | TMEM176A | transmemb | ✓ | - | - |
|---|---|---|---|---|---|---|
| 28959 | TMEM176B | TMEM176B | transmemb | ✓ | - | - |
| 7157 | TP53 | TP53 | tumor prote | - | ✓ | |
| 8626 | TP63 | TP63 | tumor prote | - | ✓ | - |
| 57761 | TRIB3 | TRIB3 | tribbles hor | ✓ | - | - |
| 57570 | TRMT5 | TRMT5 | TRM5 tRNA | ✓ | - | - |
| 85480 | TSLP | TSLP | thymic stro | ✓ | - | - |
| 10078 | TSSC4 | TSSC4 | tumor supp | ✓ | - | - |
| 203068 | TUBB | TUBB | tubulin, bet | ✓ | - | - |
| 7295 | TXN | TXN | thioredoxin | ✓ | - | - |
| 10628 | TXNIP | TXNIP | thioredoxin | ✓ | - | - |
| 7305 | TYROBP | TYROBP | TYRO prote | ✓ | - | - |
| 7307 | U2AF1 | U2AF1 | U2 small nu | ✓ | - | - |
| 6675 | UAP1 | UAP1 | UDP-N-act | ✓ | - | - |
| 29796 | UCRC | UCRC | ubiquinol-c | ✓ | - | - |
| 7353 | UFD1L | UFD1L | ubiquitin fu | ✓ | - | - |
| 7386 | UQCRFS1 | UQCRFS1 | ubiquinol-c | ✓ | ✓ | - |
| 27089 | UQCRQ | UQCRQ | ubiquinol-c | ✓ | - | - |
| 7390 | UROS | UROS | uroporphyr | ✓ | - | - |
| 57602 | USP36 | USP36 | ubiquitin sp | ✓ | - | - |
| 10493 | VAT1 | VAT1 | vesicle ami | ✓ | - | - |
| 7422 | VEGFA | VEGFA | vascular en | - | ✓ | - |
| 7436 | VLDLR | VLDLR | very low de | ✓ | - | - |
| 7450 | VWF | VWF | von Willebr | ✓ | - | - |
| 7486 | WRN | WRN | Werner syn | - | ✓ | - |
| 7639 | ZNF85 | ZNF85 | zinc finger | ✓ | - | - |
| 223082 | ZNRF2 | ZNRF2 | zinc and rir | ✓ | - | - |
| 2 | A2M | A2M | alpha-2-ma | ✓ | - | - |

**Table 2B: Description of human prostate cancer patients selected from**

| Patient ID | Age at diagnosis | BCR-Free Time | BCR-Event | Pathological Gleason Score | ClinT_Stage | PreDx BxPSA | PreTx PSA | BxGGS | Analysis Used in |
|---|---|---|---|---|---|---|---|---|---|
| PCA0008 | 64.23682 | 149.194 | No | 6 | T2C | 10.4 | 10.4 | 3+4 | GSEA using 377 aging and senescence signature (Fig 2F); Validation of 3-gene combination Fig. 3 and Supplementary Fig 5, 6A, 8 |
| PCA0012 | 66.17527 | 128.43 | No | 6 | T2C | 3.3 | 3.26 | 6 | |
| PCA0005 | 64.69953 | 126.097 | No | 3+4 | T1C | 8.7 | 5.9 | 3+3 | |
| PCA0027 | 50.75262 | 116.832 | No | 6 | T1C | 6.69 | 6.69 | 3+2 | |
| PCA0030 | 60.28329 | 115.091 | No | 3+4 | T2A | 8.55 | 12.8 | 6 | |
| PCA0017 | 55.91632 | 104.38 | No | 3+4 | T2A | 9.32 | 7.65 | 3+4 | |
| PCA0037 | 42.79078 | 104.052 | No | 3+4 | T2A | 5 | 5 | 6 | GS6 used in GSEA using 377 aging and senescence signature (Fig 2E and Supplementary 1B ); Validation of 3-gene combination Fig. 3 and Supplementary Fig 5, 6A, 8 |
| PCA0052 | 56.67198 | 102.54 | No | 3+4 | T1C | 12 | 12 | 6 | |
| PCA0007 | 56.76507 | 98.5976 | No | 3+4 | T1C | 14 | 3.8 | 3+4 | |
| PCA0003 | 67.93575 | 93.1437 | No | 3+4 | T2B | 4.6 | 11.3 | 3+4 | |
| PCA0040 | 46.83741 | 89.4968 | No | 3+4 | T2A | 4.6 | 4.6 | 6 | |
| PCA0074 | 58.69256 | 84.3057 | No | 3+4 | T1C | 4.5 | 5.11 | 3+4 | |
| PCA0050 | 59.68642 | 83.8457 | No | 3+4 | T2B | 6.7 | 6.7 | 3+4 | |
| PCA0057 | 67.46482 | 82.8601 | No | 3+4 | T1C | 5 | 5.43 | 6 | |
| PCA0011 | 52.1161 | 82.1701 | No | 6 | T1C | 2 | 6.7 | 6 | |
| PCA0026 | 57.11279 | 78.1618 | No | 3+4 | T1C | 8.6 | 9.49 | 6 | |
| PCA0035 | 62.75014 | 77.8004 | No | 6 | T2B | 13.1 | 13.1 | 6 | |
| PCA0065 | 70.18904 | 77.3733 | No | 3+4 | T1C | 4 | 5.04 | | 6 |
| PCA0066 | 51.61507 | 77.1105 | No | 3+4 | T2B | 11.5 | 13.6 | | 3+4 |
| PCA0014 | 60.349 | 76.4534 | No | 3+4 | T1C | 4.2 | 2.91 | | 6 |
| PCA0089 | 57.30992 | 70.1781 | No | 6 | T1C | 7 | 7 | | 6 |
| PCA0033 | 61.38119 | 69.0939 | No | 6 | T2B | 9.6 | 14.47 | | 6 |

| Table 2B: Description of human prostate cancer patients selected from | | | | | | | | | |
|---|---|---|---|---|---|---|---|---|---|
| Patient ID | Age at diagnosis | BCR-Free Time | BCR-Event | Pathological Gleason Score | ClinT_Stage | PreDx BxPSA | PreTx PSA | BxGGS | Analysis Used in |
| PCA0094 | 44.43353 | 66.1041 | No | 3+4 | T1C | 8.6 | 8.6 | | 6 |
| PCA0120 | 53.46589 | 62.6543 | No | 6 | T1C | 3.8 | 4.07 | | 6 |
| PCA0101 | 59.51667 | 62.3586 | No | 6 | T1C | 5 | 5 | | 6 |
| PCA0077 | 48.31588 | 61.7015 | No | 6 | T2B | 2.9 | 2.77 | | 6 |
| PCA0021 | 57.99439 | 61.5044 | No | 3+4 | T2C | 9.24 | 9.64 | | 4+3 |
| PCA0125 | 54.35297 | 61.373 | No | 3+4 | T2C | 5 | 4.11 | | 3+4 |
| PCA0086 | 37.2958 | 60.8473 | No | 3+4 | T1C | 3.4 | 6.63 | | 4+3 |
| PCA0113 | 56.91018 | 60.453 | No | 3+4 | T1C | 4.2 | 4.2 | | 3+4 |
| PCA0123 | 67.83718 | 60.0588 | No | 6 | T1C | 6.6 | 6.6 | | 6 |
| PCA0149 | 52.51857 | 59.1717 | No | 3+4 | T2C | 16 | 20.4 | | 3+4 |
| PCA0108 | 49.49592 | 59.1388 | No | 6 | T3A | 4.9 | 4.9 | | 6 |
| PCA0082 | 60.67754 | 58.9746 | No | 6 | T1C | 4.11 | 4.13 | | 6 |
| PCA0110 | 58.85136 | 58.9089 | No | 6 | T1C | 5.7 | 5.7 | | 6 |
| PCA0020 | 59.88629 | 56.9376 | No | 3+4 | T2B | 3.8 | 3.8 | | 3+4 |
| PCA0129 | 62.39421 | 56.8719 | No | 3+4 | T2A | 11.7 | 11.7 | | 3+4 |
| PCA0087 | 50.99356 | 56.839 | No | 3+4 | T2C | 4.9 | 4.9 | | 6 |
| PCA0107 | 46.49244 | 56.149 | No | 6 | T2B | 1.8 | 1.8 | | 6 |
| PCA0124 | 50.50348 | 55.1963 | No | 3+4 | T2A | 8.93 | 10.8 | | 6 |
| PCA0164 | 58.15046 | 54.5392 | No | 6 | T1C | 7.67 | 7.67 | | 6 |
| PCA0122 | 51.28378 | 52.4693 | No | 6 | T1C | 4.2 | 4.2 | | 6 |
| PCA0126 | 61.61391 | 51.8451 | No | 3+4 | T1C | 4.39 | 3.33 | | 6 |
| PCA0135 | 56.85268 | 51.6479 | No | 3+4 | T1C | 6.1 | 6.1 | | 6 |

| Table 2B: Description of human prostate cancer patients selected from | | | | | | | | | |
|---|---|---|---|---|---|---|---|---|---|
| Patient ID | Age at diagnosis | BCR-Free Time | BCR-Event | Pathological Gleason Score | ClinT_Stage | PreDx BxPSA | PreTx PSA | BxGGS | Analysis Used in |
| PCA0095 | 56.22023 | 51.5822 | No | 6 | T1C | 4.5 | 4.5 | | 6 |
| PCA0146 | 48.38981 | 50.8923 | No | 6 | T1C | 3.1 | 3.1 | | 6 |
| PCA0111 | 60.49411 | 49.8409 | No | 3+4 | T1C | 9.2 | 6.69 | | 3+4 |
| PCA0075 | 54.45428 | 49.3481 | No | 3+4 | T2A | 4.8 | 4.62 | | 6 |
| PCA0168 | 56.75412 | 49.0195 | No | 3+4 | T2C | 3.94 | 4.27 | | 6 |
| PCA0132 | 55.92453 | 48.5596 | No | 6 | T2A | 4.4 | 5.29 | | 6 |
| PCA0090 | 58.9773 | 48.4281 | No | 3+4 | T1C | 7.4 | 7.4 | | 6 |
| PCA0145 | 56.28594 | 48.4281 | No | 3+4 | T1C | 6.6 | 6.6 | | 3+4 |
| PCA0151 | 58.36949 | 47.3439 | No | 6 | T1C | 4.5 | 4.5 | | 6 |
| PCA0093 | 46.1283 | 46.424 | No | 3+4 | T1C | 6.74 | 4.97 | | 6 |
| PCA0147 | 52.47751 | 45.734 | No | 6 | T1C | 5.4 | 5.4 | | 6 |
| PCA0163 | 67.70028 | 45.3726 | No | 3+4 | T1C | 2.65 | 2.65 | | 3+4 |
| PCA0118 | 51.40151 | 43.8285 | No | 3+4 | T1C | 4 | 5.08 | | 6 |
| PCA0104 | 69.89335 | 43.4671 | No | 3+4 | T2A | 2.1 | 1.6 | | 6 |
| PCA0062 | 52.46382 | 42.9414 | No | 6 | T1C | 1.09 | 1.15 | | 6 |
| PCA0169 | 52.84713 | 42.9414 | No | 6 | T1C | 6.7 | 6.7 | | 6 |
| PCA0141 | 60.57351 | 41.7586 | No | 3+4 | T1C | 6.55 | 5.44 | | 6 |
| PCA0157 | 47.38499 | 39.853 | No | 6 | T1C | 3.5 | 3.5 | | 6 |
| PCA0084 | 70.80781 | 39.6559 | No | 6 | T1C | 3.1 | 3.69 | | 6 |
| PCA0100 | 60.30519 | 38.2103 | No | 3+4 | T2A | 6.95 | 6.95 | | 4+3 |
| PCA0178 | 61.82747 | 37.6846 | No | 6 | T1C | 4.6 | 4.65 | | 6 |
| PCA0144 | 56.15178 | 37.586 | No | 6 | T1C | 5.6 | 5.6 | | 3+4 |

EP 2 885 429 B1

| Table 2B: Description of human prostate cancer patients selected from | | | | | | | | | |
|---|---|---|---|---|---|---|---|---|---|
| Patient ID | Age at diagnosis | BCR-Free Time | BCR-Event | Pathological Gleason Score | ClinT_ Stage | PreDx BxPSA | PreTx PSA | BxGGS | Analysis Used in |
| PCA0175 | 50.99903 | 36.009 | No | 6 | T1C | 5.6 | 5.6 | | 3+4 |
| PCA0010 | 49.86554 | 35.0562 | No | 6 | T1C | 5.2 | 5.2 | | 6 |
| PCA0173 | 66.15884 | 32.6906 | No | 6 | T1C | 5.24 | 5.24 | | 3+4 |
| PCA0158 | 56.90197 | 31.6064 | No | 6 | T1C | 4.8 | 3.2 | | 6 |
| PCA0165 | 64.81453 | 30.5222 | No | 6 | T1C | 6.34 | 6.34 | | 6 |
| PCA0058 | 67.72492 | 30.1937 | No | 6 | T1C | 7.2 | 7.66 | | 6 |
| PCA0133 | 58.35854 | 28.0581 | No | 3+4 | T1C | 6.98 | 6.98 | | 6 |
| PCA0167 | 55.26196 | 26.8425 | No | 3+4 | T2B | 2.5 | 2.98 | | 6 |
| PCA0029 | 53.81361 | 26.6782 | No | 3+4 | T2B | 6.9 | 6.82 | | 6 |
| PCA0115 | 60.25864 | 26.2182 | No | 3+4 | T2A | 5.97 | 5.97 | | 6 |
| PCA0056 | 83 | 25 | No | 6 | T1C | NA | NA | | 6 |
| PCA0064 | 45.09063 | 24.2798 | No | 6 | T2B | 3.3 | 5.52 | 6 | |
| PCA0015 | 58.71446 | 22.7027 | No | 3+4 | T2C | 2.9 | 2.9 | 6 | |
| PCA0109 | 58.91159 | 13.8648 | No | 6 | T1C | 4.8 | 5.38 | 6 | |
| PCA0160 | 62.42433 | 12.9777 | No | 6 | T1C | 4 | 4 | 6 | |
| PCA0162 | 55.87525 | 11.8278 | No | 3+4 | T1C | 6.2 | 7.11 | 4+3 | |
| PCA0156 | 51.43436 | 10.8093 | No | 3+4 | T1C | 13.3 | 13.3 | 3+4 | |
| PCA0013 | 54.20513 | 10.3822 | No | 3+4 | T1C | 9.4 | 9.35 | 3+4 | |
| PCA0171 | 60.8692 | 8.83797 | No | 6 | T2A | 8.2 | 8.2 | 6 | |
| PCA0097 | 62.5448 | 1.87273 | No | 6 | T1C | 5.3 | 5.3 | 6 | |
| PCA0034 | 57 | 92.9794 | Yes | 6 | T1C | 5.4 | 5.4 | 6 | |
| PCA0025 | 61.16489 | 68.0425 | Yes | 3+4 | T2B | 3.7 | 3.7 | 4+3 | |

| Table 2B: Description of human prostate cancer patients selected from | | | | | | | | | |
|---|---|---|---|---|---|---|---|---|---|
| Patient ID | Age at diagnosis | BCR-Free Time | BCR-Event | Pathological Gleason Score | ClinT_ Stage | PreDx BxPSA | PreTx PSA | BxGGS | Analysis Used in |
| **PCA0009** | 56.5789 | 64.757 | Yes | 3+4 | T2A | 14.6 | 12.9 | 6 | |
| **PCA0022** | 57.67132 | 39.9516 | Yes | 6 | T1C | 5.8 | 5.8 | 3+3 | |
| **PCA0103** | 59.4318 | 28.6495 | Yes | 3+4 | T1C | 4.5 | 4.5 | 3+4 | **GSEA using 377 aging and senescence signature (Fig 2F); Validation of 3-gene combination Fig. 3 and Supplementary Fig 5, 6A, 8** |
| **PCA0161** | 64.0041 | 19.023 | Yes | 3+4 | T1C | 6.9 | 6.9 | 6 | |
| **PCA0117** | 58.36675 | 18.8259 | Yes | 3+4 | T1C | 18.58 | 22.36 | 8 | |
| **PCA0081** | 49.98875 | 9.85647 | Yes | 3+4 | T2B | 5.8 | 5.8 | 3+4 | |
| **PCA0024** | 56.58163 | 3.94259 | Yes | 3+4 | T1C | 14.9 | 18.41 | 3+4 | |
| PCA0130 | 64.52705 | 27.861 | Yes | 4+4 | T1C | 3.6 | 2.21 | 4+4 | **GSEA using 377 aging and senescence signature (Fig 2D) and Supplementary Fig 5** |
| PCA0028 | 61.47154 | 27.5981 | Yes | 5+3 | T1C | 3.82 | 3.82 | 3+3 | |
| PCA0092 | 67.45113 | 16.8217 | Yes | 4+4 | T3A | 5.8 | 5 | 4+3 | |
| PCA0112 | 58.77196 | 13.2077 | Yes | 4+4 | T3A | 25 | 33.71 | 4+4 | |
| PCA0054 | 54.97722 | 2.10271 | Yes | 3+5 | T1C | 31 | 39.9 | 4+3 | |
| PCA0159 | 45.97771 | 1.41276 | Yes | 4+4 | T2A | 4 | 5.36 | 4+3 | |
| PCA0096 | 71.20206 | 42.3828 | Yes | 4+5 | T2A | 5.8 | 8.32 | 3+4 | |
| PCA0172 | 51.99564 | 30.5551 | Yes | 4+5 | T1C | 17.2 | 22.82 | 4+4 | |
| PCA0181 | 69.00626 | 30.0294 | Yes | 4+5 | T1C | 27 | 27 | 4+5 | |
| PCA0032 | 56.36808 | 3.71261 | Yes | 4+5 | T2A | 9.6 | 16.71 | 3+3 | |
| PCA0179 | 64.89119 | 2.92409 | Yes | 4+5 | T1C | 40.24 | 46.36 | 4+5 | |
| PCA0176 | 53.54803 | 2.56268 | Yes | 4+5 | T1C | 8.1 | 8.66 | 3+3 | |
| PCA0180 | 67.17461 | 1.37991 | Yes | 4+5 | T1C | 8.03 | 13.34 | 4+3 | |

| 3A: Lagging edge genes from GSEA | | |
|---|---|---|
| *Entrez* ID | Gene Symbol | Hyperlink |
| 23118 | MAP3K7IP2 | MAP3K7IP2 |
| 121536 | AEBP2 | AEBP2 |
| 9314 | KLF4 | KLF4 |
| 80314 | EPC1 | EPC1 |
| 10628 | TXNIP | TXNIP |
| 1432 | MAPK14 | MAPK14 |
| 5828 | PXMP3 | PXMP3 |
| 3075 | CFH | CFH |
| 152789 | JAKMIP1 | JAKMIP1 |
| 6122 | RPL3 | RPL3 |
| 79023 | NUP37 | NUP37 |
| 29103 | DNAJC15 | DNAJC15 |
| 3134 | HLA-F | HLA-F |
| 8635 | RNASET2 | RNASET2 |
| 6430 | SFRS5 | SFRS5 |
| 4711 | NDUFB5 | NDUFB5 |
| 1356 | CP | CP |
| 509 | ATP5C1 | ATP5C1 |
| 5138 | PDE2A | PDE2A |
| 1312 | COMT | COMT |
| 847 | CAT | CAT |
| 443 | ASPA | ASPA |
| 1281 | COL3A1 | COL3A1 |
| 9452 | ITM2A | ITM2A |
| 10914 | PAPOLA | PAPOLA |
| 3135 | HLA-G | HLA-G |
| 57602 | USP36 | USP36 |
| 23786 | BCL2L13 | BCL2L13 |
| 4738 | NEDD8 | NEDD8 |
| 3459 | IFNGR1 | IFNGR1 |
| 29796 | UCRC | UCRC |
| 3122 | HLA-DRA | HLA-DRA |
| 4092 | SMAD7 | SMAD7 |
| 10135 | NAMPT | NAMPT |
| 28959 | TMEM176B | TMEM176B |
| 653 | BMP5 | BMP5 |
| 5717 | PSMD11 | PSMD11 |

(continued)

| 3A: Lagging edge genes from GSEA | | |
|---|---|---|
| *Entrez* ID | Gene Symbol | Hyperlink |
| 1026 | CDKN1A | CDKN1A |
| 2202 | EFEMP1 | EFEMPL1 |
| 4057 | LTF | LTF |
| 7386 | UQCRFS1 | UQCRFS1 |
| 3043 | HBB | HBB |
| 64114 | TMBIM1 | TMBIM1 |
| 4677 | NARS | NARS |
| 1512 | CTSH | CTSH |
| 3916 | LAMP1 | LAMP1 |
| 351 | APP | APP |
| 10493 | VAT1 | VAT1 |
| 30845 | EHD3 | EHD3 |
| 11258 | DCTN3 | DCTN3 |
| 10972 | TMED10 | TMED10 |
| 2634 | GBP2 | GBP2 |
| 1466 | CSRP2 | CSRP2 |
| 2628 | GATM | GATM |
| 79602 | ADIPOR2 | ADIPOR2 |
| 23411 | SIRT1 | SIRT1 |
| 3696 | ITGB8 | ITGB8 |
| 84883 | AIFM2 | AIFM2 |
| 25940 | FAM98A | FAM98A |
| 2878 | GPX3 | GPX3 |
| 1051 | CEBPB | CEBPB |
| 51421 | AMOTL2 | AMOTL2 |
| 5213 | PFKM | PFKM |
| 10728 | PTGES3 | PTGES3 |
| 79026 | AHNAK | AHNAK |
| 9516 | LITAF | LITAF |
| 6392 | SDHD | SDHD |
| 64981 | MRPL34 | MRPL34 |
| 7913 | DEK | DEK |
| 522 | ATP5J | ATP5J |
| 9315 | C5orf13 | C5orf13 |
| 4714 | NDUFB8 | NDUFB8 |
| 140609 | NEK7 | NEK7 |
| 567 | B2M | B2M |

(continued)

| 3A: Lagging edge genes from GSEA | | |
|---|---|---|
| *Entrez* ID | Gene Symbol | Hyperlink |
| 648 | BMI1 | BMI1 |
| 9813 | KIAA0494 | KIAA0494 |
| 1306 | COL15A1 | COL15A1 |
| 967 | CD63 | CD63 |
| 9987 | HNRPDL | HNRPDL |
| 2799 | GNS | GNS |
| 4494 | MT1F | MT1F |
| 6275 | S100A4 | S100A4 |
| 4493 | MT1E | MT1E |
| 4204 | MECP2 | MECP2 |
| 8626 | TP63 | TP63 |
| 2260 | FGFR1 | FGFR1 |
| 715 | C1R | C1R |
| 8313 | AXIN2 | AXIN2 |
| 84302 | C9orf125 | C9orf125 |
| 85480 | TSLP | TSLP |
| 3315 | HSPB1 | HSPB1 |
| 9021 | SOCS3 | SOCS3 |
| 22998 | LIMCH1 | LIMCH1 |
| 137392 | FAM92A1 | FAM92A1 |
| 1287 | COL4A5 | COL4A5 |
| 84247 | LDOC1L | LDOC1L |
| 4780 | NFE2L2 | NFE2L2 |
| 6376 | CX3CL1 | CX3CL1 |
| 90865 | IL33 | IL33 |
| 5728 | PTEN | PTEN |
| 3479 | IGF1 | IGF1 |
| 6272 | SORT1 | SORT1 |
| 307 | ANXA4 | ANXA4 |
| 8826 | IQGAP1 | IQGAP1 |
| 3958 | LGALS3 | LGALS3 |
| 5376 | PMP22 | PMP22 |
| 716 | C1S | C1S |
| 4478 | MSN | MSN |
| 710 | SERPING1 | SERPING1 |
| 9737 | GPRASP1 | GPRASP1 |
| 51100 | SH3GLB1 | SH3GLB1 |

(continued)

| 3A: Lagging edge genes from GSEA | | |
|---|---|---|
| *Entrez* ID | Gene Symbol | Hyperlink |
| 2335 | FN1 | FN1 |
| 498 | ATP5A1 | ATP5A1 |
| 1410 | CRYAB | CRYAB |
| 11170 | FAM107A | FAM107A |
| 5348 | FXYD1 | FXYD1 |
| 23022 | PALLD | PALLD |
| 25932 | CLIC4 | CLIC4 |
| 1191 | CLU | CLU |
| 1465 | CSRP1 | CSRP1 |
| 128 | ADH5 | ADH5 |

| 4C: Meta Analysis using Fisher combined me | | | |
|---|---|---|---|
| *Entrez* ID | Gene Symbol | HyperLink | Gene Description |
| 1287 | COL4A5 | COL4A5 | collagen, type IV, alpha 5 |
| 57447 | NDRG2 | NDRG2 | NDRG family member 2 |
| 3315 | HSPB1 | HSPB1 | heat shock 27kDa protein 1 |
| 9737 | GPRASP1 | GPRASP1 | G protein-coupled receptor associated sorting protein 1 |
| 8626 | TP63 | TP63 | tumor protein p63 |
| 6277 | S100A6 | S100A6 | S100 calcium binding protein A6 |
| 54541 | DDIT4 | DDIT4 | DNA-damage-inducible transcript 4 |
| 2628 | GATM | GATM | glycine amidinotransferase (L-arginine:glycine amidinotransferase) |
| 2170 | FABP3 | FABP3 | fatty acid binding protein 3, muscle and heart (mammary-derived growth inhibitor) |
| 445 | ASS1 | ASS1 | argininosuccinate synthetase 1 |
| 1191 | CLU | CLU | clusterin |
| 6385 | SDC4 | SDC4 | syndecan 4 |
| 108 | ADCY2 | ADCY2 | adenylate cyclase 2 (brain) |
| 4204 | MECP2 | MECP2 | methyl CpG binding protein 2 (Rett syndrome) |
| 6675 | UAP1 | UAP1 | UDP-N-acteylglucosamine pyrophosphorylase 1 |
| 5213 | PFKM | PFKM | phosphofructokinase, muscle |
| 10493 | VAT1 | VAT1 | vesicle amine transport protein 1 homolog (T. californica) |
| 8844 | KSR1 | KSR1 | kinase suppressor of ras 1 |
| 4609 | MYC | MYC | v-myc myelocytomatosis viral oncogene homolog (avian) |
| 152789 | JAKMIP1 | JAKMIP1 | janus kinase and microtubule interacting protein 1 |
| 1410 | CRYAB | CRYAB | crystallin, alpha B |
| 23705 | CADM1 | CADM1 | cell adhesion molecule 1 |

(continued)

| 4C: Meta Analysis using Fisher combined me | | | |
|---|---|---|---|
| *Entrez* ID | Gene Symbol | HyperLink | Gene Description |
| 6096 | RORB | RORB | RAR-related orphan receptor B |
| 10577 | NPC2 | NPC2 | Niemann-Pick disease, type C2 |
| 137392 | FAM92A1 | FAM92A1 | family with sequence similarity 92, member A1 |
| 219972 | MPEG1 | MPEG1 | macrophage expressed gene 1 |
| 1026 | CDKN1A | CDKN1A | cyclin-dependent kinase inhibitor 1A (p21, Cip1) |
| 2938 | GSTA1 | GSTA1 | glutathione S-transferase A1 |
| 1465 | CSRP1 | CSRP1 | cysteine and glycine-rich protein 1 |
| 4170 | MCL1 | MCL1 | myeloid cell leukemia sequence 1 (BCL2-related) |
| 518 | ATP5G3 | ATP5G3 | ATP synthase, H+ transporting, mitochondrial F0 complex, subunit C3 (subunit 9) |
| 10417 | SPON2 | SPON2 | spondin 2, extracellular matrix protein |
| 5341 | PLEK | PLEK | pleckstrin |
| 967 | CD63 | CD63 | CD63 molecule |
| 1512 | CTSH | CTSH | cathepsin H |
| 1277 | COL1A1 | COL1A1 | collagen, type I, alpha 1 |
| 3109 | HLA-DMB | HLA-DMB | major histocompatibility complex, class II, DM beta |
| 3696 | ITGB8 | ITGB8 | integrin, beta 8 |
| 7157 | TP53 | TP53 | tumor protein p53 |
| 51228 | GLTP | GLTP | glycolipid transfer protein |
| 3732 | CD82 | CD82 | CD82 molecule |
| 2202 | EFEMP1 | EFEMP1 | EGF-containing fibulin-like extracellular matrix protein 1 |
| 5376 | PMP22 | PMP22 | peripheral myelin protein 22 |
| 5046 | PCSK6 | PCSK6 | proprotein convertase subtilisin/kexin type 6 |
| 22998 | LIMCH1 | LIMCH1 | LIM and calponin homology domains 1 |
| 4714 | NDUFB8 | NDUFB8 | NADH dehydrogenase (ubiquinone) 1 beta subcomplex, 8, 19kDa |
| 4478 | MSN | MSN | moesin |
| 7805 | LAPTM5 | LAPTM5 | lysosomal multispanning membrane protein 5 |
| 84302 | C9orf125 | C9orf125 | chromosome 9 open reading frame 125 |
| 2260 | FGFR1 | FGFR1 | fibroblast growth factor receptor 1 |
| 51004 | COQ6 | COQ6 | coenzyme Q6 homolog, monooxygenase (S. cerevisiae) |
| 6376 | CX3CL1 | CX3CL1 | chemokine (C-X3-C motif) ligand 1 |
| 25945 | PVRL3 | PVRL3 | poliovirus receptor-related 3 |
| 1281 | COL3A1 | COL3A1 | collagen, type III, alpha 1 |
| 1958 | EGR1 | EGR1 | early growth response 1 |
| 10135 | NAMPT | NAMPT | nicotinamide phosphoribosyltransferase |
| 963 | CD53 | CD53 | CD53 molecule |
| 9021 | SOCS3 | SOCS3 | suppressor of cytokine signaling 3 |

| 4C: Meta Analysis using Fisher combined me | | | |
|---|---|---|---|
| **Entrez ID** | **Gene Symbol** | **HyperLink** | **Gene Description** |
| 3958 | LGALS3 | LGALS3 | lectin, galactoside-binding, soluble, 3 |
| 9314 | KLF4 | KLF4 | Kruppel-like factor 4 (gut) |
| 2335 | FN1 | FN1 | fibronectin 1 |
| 713 | C1QB | C1QB | complement component 1, q subcomponent, B chain |
| 23022 | PALLD | PALLD | Palladin, cytoskeletal associated protein |
| 6392 | SDHD | SDHD | succinate dehydrogenase complex, subunit D, integral membrane protein |
| 728772 | FLJ77644 | FLJ77644 | hypothetical protein FLJ77644 |
| 4864 | NPC1 | NPC1 | Niemann-Pick disease, type C1 |
| 256691 | MAMDC2 | MAMDC2 | MAM domain containing 2 |
| 968 | CD68 | CD68 | CD68 molecule |
| 79026 | AHNAK | AHNAK | AHNAK nucleoprotein |
| 3039 | HBA1 | HBA1 | Hemoglobin, alpha 1 |
| 1778 | DYNC1 H1 | DYNC1H1 | Dynein, cytoplasmic 1, heavy chain 1 |
| 57704 | GBA2 | GBA2 | glucosidase, beta (bile acid) 2 |
| 9961 | MVP | MVP | major vault protein |
| 10966 | RAB40B | RAB40B | RAB40B, member RAS oncogene family |
| 9315 | C5orf13 | C5orf13 | chromosome 5 open reading frame 13 |
| 57650 | KIAA1524 | KIAA1524 | KIAA1524 |
| 85480 | TSLP | TSLP | thymic stromal lymphopoietin |
| 4738 | NEDD8 | NEDD8 | neural precursor cell expressed, developmentally down-regulated 8 |
| 9516 | LITAF | LITAF | lipopolysaccharide-induced TNF factor |
| 4507 | MTAP | MTAP | methylthioadenosine phosphorylase |
| 80314 | EPC1 | EPC1 | enhancer of polycomb homolog 1 (Drosophila) |
| 3689 | ITGB2 | ITGB2 | integrin, beta 2 (complement component 3 receptor 3 and 4 subunit) |
| 3043 | HBB | HBB | Hemoglobin, beta |
| 3075 | CFH | CFH | complement factor H |
| 347 | APOD | APOD | apolipoprotein D |
| 4722 | NDUFS3 | NDUFS3 | NADH dehydrogenase (ubiquinone) Fe-S protein 3, 30kDa (NADH-coenzyme Q reductase) |
| 8480 | RAE1 | RAE1 | RAE1 RNA export 1 homolog (S. pombe) |
| 3122 | HLA-DRA | HLA-DRA | major histocompatibility complex, class II, DR alpha |
| 1889 | ECE1 | ECE1 | endothelin converting enzyme 1 |
| 259217 | HSPA12A | HSPA12A | heat shock 70kDa protein 12A |
| 11214 | AKAP13 | AKAP13 | A kinase (PRKA) anchor protein 13 |
| 11258 | DCTN3 | DCTN3 | dynactin 3 (p22) |
| 5331 | PLCB3 | PLCB3 | phospholipase C, beta 3 (phosphatidylinositol-specific) |

(continued)

| 4C: Meta Analysis using Fisher combined me | | | |
|---|---|---|---|
| **Entrez ID** | **Gene Symbol** | **HyperLink** | **Gene Description** |
| 51495 | PTPLAD1 | PTPLAD1 | protein tyrosine phosphatase-like A domain containing 1 |
| 1453 | CSNK1D | CSNK1D | casein kinase 1, delta |
| 8313 | AXIN2 | AXIN2 | axin 2 |
| 55902 | ACSS2 | ACSS2 | acyl-CoA synthetase short-chain family member 2 |
| 382 | ARF6 | ARF6 | ADP-ribosylation factor 6 |
| 10628 | TXNIP | TXNIP | thioredoxin interacting protein |
| 3300 | DNAJB2 | DNAJB2 | DnaJ (Hsp40) homolog, subfamily B, member 2 |
| 5305 | PIP4K2A | PIP4K2A | phosphatidylinositol-5-phosphate 4-kinase, type II, alpha |
| 5138 | PDE2A | PDE2A | phosphodiesterase 2A, cGMP-stimulated |
| 24145 | PANX1 | PANX1 | pannexin 1 |
| 90865 | IL33 | IL33 | interleukin 33 |
| 4092 | SMAD7 | SMAD7 | SMAD family member 7 |
| 121536 | AEBP2 | AEBP2 | AE binding protein 2 |
| 2212 | FCGR2A | FCGR2A | Fc fragment of IgG, low affinity IIa, receptor (CD32) |
| 6272 | SORT1 | SORT1 | sortilin 1 |
| 443 | ASPA | ASPA | aspartoacylase (Canavan disease) |
| 64114 | TMBIM1 | TMBIM1 | transmembrane BAX inhibitor motif containing 1 |
| 28973 | MRPS18B | MRPS18B | mitochondrial ribosomal protein S18B |
| 10397 | NDRG1 | NDRG1 | N-myc downstream regulated gene 1 |
| 8826 | IQGAP1 | IQGAP1 | IQ motif containing GTPase activating protein 1 |
| 55298 | RNF121 | RNF121 | ring finger protein 121 |
| 1351 | COX8A | COX8A | cytochrome c oxidase subunit 8A (ubiquitous) |
| 6558 | SLC12A2 | SLC12A2 | solute carrier family 12 (sodium/potassium/chloride transporters), member 2 |
| 27069 | GHITM | GHITM | growth hormone inducible transmembrane protein |
| 7018 | TF | TF | transferrin |
| 5348 | FXYD1 | FXYD1 | FXYD domain containing ion transport regulator 1 |
| 9655 | SOCS5 | SOCS5 | suppressor of cytokine signaling 5 |
| 4257 | MGST1 | MGST1 | microsomal glutathione S-transferase 1 |
| 2634 | GBP2 | GBP2 | guanylate binding protein 2, interferon-inducible |
| 404636 | FAM45A | FAM45A | Family with sequence similarity 45, member A |
| 25932 | CLIC4 | CLIC4 | chloride intracellular channel 4 |
| 10457 | GPNMB | GPNMB | glycoprotein (transmembrane) nmb |
| 57602 | USP36 | USP36 | ubiquitin specific peptidase 36 |
| 7107 | GPR137B | GPR137B | G protein-coupled receptor 137B |
| 3916 | LAMP1 | LAMP1 | lysosomal-associated membrane protein 1 |
| 7037 | TFRC | TFRC | transferrin receptor (p90, CD71) |

(continued)

| 4C: Meta Analysis using Fisher combined me | | | |
|---|---|---|---|
| *Entrez* ID | Gene Symbol | HyperLink | Gene Description |
| 7436 | VLDLR | VLDLR | very low density lipoprotein receptor |
| 7040 | TGFB1 | TGFB1 | transforming growth factor, beta 1 |
| 2805 | GOT1 | GOT1 | glutamic-oxaloacetic transaminase 1, soluble (aspartate aminotransferase 1) |
| 2203 | FBP1 | FBP1 | fructose-1,6-bisphosphatase 1 |
| 3727 | JUND | JUND | jun D proto-oncogene |
| 83706 | FERMT3 | FERMT3 | fermitin family homolog 3 (Drosophila) |
| 2896 | GRN | GRN | granulin |
| 4717 | NDUFC1 | NDUFC1 | NADH dehydrogenase (ubiquinone) 1, subcomplex unknown, 1, 6kDa |
| 3135 | HLA-G | HLA-G | major histocompatibility complex, class I, G |
| 972 | CD74 | CD74 | CD74 molecule, major histocompatibility complex, class II invariant chain |
| 3157 | HMGCS1 | HMGCS1 | 3-hydroxy-3-methylglutaryl-Coenzyme A synthase 1 (soluble) |
| 3512 | IGJ | IGJ | immunoglobulin J polypeptide, linker protein for immunoglobulin alpha and mu polypeptides |
| 1312 | COMT | COMT | catechol-O-methyltransferase |
| 29103 | DNAJC15 | DNAJC15 | DnaJ (Hsp40) homolog, subfamily C, member 15 |

| Method for all Gleason score 6 patients |
|---|
| *P-value* |
| 0 |
| 0 |
| 0 |
| 0 |
| 7.77E-16 |
| 1.64E-14 |
| 1.88E-14 |
| 2.30E-14 |
| 2.82E-14 |
| 5.47E-14 |
| 5.50E-14 |
| 1.58E-13 |
| 3.93E-13 |
| 5.24E-13 |
| 1.27E-12 |
| 3.15E-12 |

(continued)

| Method for all Gleason score 6 patients |
| --- |
| *P-value* |
| 9.07E-12 |
| 2.31E-11 |
| 3.10E-11 |
| 1.03E-10 |
| 3.13E-10 |
| 3.27E-10 |
| 9.95E-10 |
| 1.04E-09 |
| 1.52E-09 |
| 2.88E-09 |
| 9.00E-09 |
| 3.83E-08 |
| 5.04E-08 |
| 5.98E-08 |
| 7.21 E-08 |
| 9.56E-08 |
| 1.15E-07 |
| 1.22E-07 |
| 1.26E-07 |
| 1.91E-07 |
| 4.16E-07 |
| 5.46E-07 |
| 7.46E-07 |
| 7.97E-07 |
| 1.13E-06 |
| 1.25E-06 |
| 1.28E-06 |
| 2.26E-06 |
| 3.37E-06 |
| 3.45E-06 |
| 3.70E-06 |
| 3.95E-06 |
| 5.74E-06 |
| 6.30E-06 |
| 7.81 E-06 |
| 8.32E-06 |
| 9.23E-06 |

(continued)

| Method for all Gleason score 6 patients |
| --- |
| *P-value* |
| 1.20E-05 |
| 1.35E-05 |
| 1.54E-05 |
| 1.76E-05 |
| 2.13E-05 |
| 2.13E-05 |
| 2.18E-05 |
| 2.37E-05 |
| 4.55E-05 |
| 7.42E-05 |
| 7.56E-05 |
| 7.81 E-05 |
| 9.70E-05 |
| 1.23E-04 |
| 1.57E-04 |
| 1.79E-04 |
| 1.82E-04 |
| 1.84E-04 |
| 2.75E-04 |
| 3.40E-04 |
| 4.15E-04 |
| 4.33E-04 |
| 4.50E-04 |
| 4.77E-04 |
| 5.04E-04 |
| 5.06E-04 |
| 5.12E-04 |
| 6.28E-04 |
| 6.71 E-04 |
| 7.31 E-04 |
| 7.36E-04 |
| 1.03E-03 |
| 1.05E-03 |
| 1.27E-03 |
| 1.29E-03 |
| 1.30E-03 |
| 1.38E-03 |

(continued)

| Method for all Gleason score 6 patients |
|:---:|
| *P-value* |
| 1.47E-03 |
| 1.48E-03 |
| 1.79E-03 |
| 1.88E-03 |
| 1.96E-03 |
| 3.02E-03 |
| 3.08E-03 |
| 3.14E-03 |
| 3.43E-03 |
| 3.48E-03 |
| 3.55E-03 |
| 3.87E-03 |
| 3.96E-03 |
| 4.23E-03 |
| 4.23E-03 |
| 4.59E-03 |
| 5.25E-03 |
| 6.20E-03 |
| 6.69E-03 |
| 6.73E-03 |
| 6.93E-03 |
| 8.49E-03 |
| 9.45E-03 |
| 9.77E-03 |
| 1.11 E-02 |
| 1.26E-02 |
| 1.35E-02 |
| 1.65E-02 |
| 1.65E-02 |
| 1.68E-02 |
| 1.80E-02 |
| 1.87E-02 |
| 1.96E-02 |
| 2.03E-02 |
| 2.11 E-02 |
| 2.14E-02 |
| 2.28E-02 |

(continued)

| Method for all Gleason score 6 patients |
| --- |
| *P-value* |
| 2.32E-02 |
| 2.77E-02 |
| 2.85E-02 |
| 2.99E-02 |
| 3.10E-02 |
| 3.22E-02 |
| 3.23E-02 |
| 3.25E-02 |
| 3.39E-02 |
| 4.05E-02 |
| 4.09E-02 |
| 4.11 E-02 |
| 4.24E-02 |
| 4.70E-02 |
| 4.78E-02 |
| 4.87E-02 |

## 5: Differential expression and integrative *p-values* of 19 gene indolence signature

| Entrez ID | Gene Symbol | Hyper link | Gene Description | Human aggressive prostate cancer (Yu et al, 2004) | Human lung cancer (Bhattercharje e et al, 2001 ) | Human breast carcer (TCGA 2011) |
|---|---|---|---|---|---|---|
| | | | | T-Score | T-Score | T-Score |
| 567 | B2M | B2M | beta-2-microglobulin | -3.325415 | -5.460412 | 2.92021 |
| 847 | CAT | CAT | catalase | -1.978303 | -8.206645 | -12.782956 |
| 1026 | CDKN1A | CDKN1A | cyclin-dependent kinase inhibitor 1A (p21, Cip1) | -2.432792 | -5.54278 | 1.921197 |
| 3075 | CFH | CFH | complement factor H | -1.757822 | -4.748899 | -8.212641 |
| 25932 | CLIC4 | CLIC4 | chloride intracellular channel 4 | -5.851153 | -5.30767 | -5.662074 |
| 1191 | CLU | CLU | clusterin | -6.094146 | -3.484007 | -5.390307 |
| 1512 | CTSH | CTSH | cathepsin H | -2.569518 | -4.408833 | 0.680695 |

EP 2 885 429 B1

| Meta-analysis between human prostate cancer (Yu et al, 2004); lung cancer (Bhattercharjee et al, 2001) & breas cancer (TCGA 2011) | Mouse indolent prostate lesions (Ouyang et al, 2005) | Human prostate cancer Gleason Score 8, 9 and with BCR <22 months (Taylor et al 2010) | Human prostate cancer Gleason Score 6 and 3+4 patients with varying BCR (Taylor et al 2010) | Intergrative analysis of all Gleason Score 6 patients with varying BCR (Taylor et al 2010) |
|---|---|---|---|---|
| Fisher combined method *P-value* | T-Score | T-Score | Fisher combined method *P-value* | Fisher combined method *P-value* |
| 5.8556E-06 | 0.6709767 | 0.01167 | 0.74689 | 0.64813 |
| <1E-16 | 1.0665958 | 0.00067213 | 0.4784 | 0.22064 |
| 4.0983E-06 | 0.69371456 | 0.87023 | 0.000505 | 9.0017E-09 |
| <1E-16 | 1.2873303 | 0.0041078 | 0.17777 | 0.00073642 |
| 3.04619E-11 | 0.53181756 | 0.00079583 | 0.35546 | 0.020272 |
| 4.11702E-07 | 3.2068775 | 1.0085E-07 | 5.42E-04 | 5.4956E-14 |
| 0.0167646 | 1.1162424 | 0.0050619 | 0.29919 | 1.2579E-07 |

| 6376 | CX3CL1 | CX3CL1 | chemokine (C-X3-C motif) ligand 1 | -4.143521 | -6.809325 | -9.390615 |
|---|---|---|---|---|---|---|
| 2260 | FGFR1 | FGFR1 | fibroblast growth factor receptor 1 | -3.825348 | -3.64716 | -6.300478 |
| 2878 | GPX3 | GPX3 | glutathione peroxidase 3 (plasma) | -2.980245 | -9.935951 | -11.027885 |
| 3479 | IGF1 | IGF1 | insulin-like growth factor 1 (somatomedin C) | -4.40515 | -3.266738 | -9.436766 |
| 9452 | ITM2A | ITM2A | integral membrane protein 2A | -2.00719 | -12.259881 | -11.776247 |
| 3958 | LGALS3 | LGALS3 | lectin, galactoside-binding, soluble, 3 | -4.527011 | -2.377751 | -5.7778 |
| 4204 | MECP2 | MECP2 | methyl CpG binding protein 2 (Rett syndrome) | -3.806367 | -4.259788 | -5.348661 |
| 4478 | MSN | MSN | moesin | -4.603885 | -4.528371 | 3.538711 |
| 4780 | NFE2L2 | NFE2L2 | nuclear factor (erythroid-derived 2)-like 2 | -4.135936 | -3.096358 | -8.153571 |

EP 2 885 429 B1

| | | | | |
|---|---|---|---|---|
| <1E-16 | 1.7263488 | 0.000090064 | 0.018933 | 8.3217E-06 |
| 1.6653E-09 | 0.5589273 | 0.000045696 | 0.005684 | 6.3042E-06 |
| <1E-16 | 0.9465966 | 0.00026909 | 0.13693 | 0.73623 |
| <1E-16 | 1.5195578 | 0.2222 | 0.56307 | 0.47131 |
| <1E-16 | 2.7188826 | 0.11519 | 0.20422 | 0.96051 |
| 5.78844E-07 | 1.0102977 | 1.1614E-08 | 0.008531 | 0.00002131 |
| 1.0561E-09 | 0.8091755 | 0.00032587 | 2.58E-03 | 5.2447E-13 |
| 0.002108 | 0.83625895 | 0.000064305 | 2.15E-02 | 3.6991E-06 |
| 5.9952E-15 | 0.78006816 | 0.00040586 | 0.57369 | 0.2679 |

| 5376 | PMP22 | PMP22 | peripheral myelin protein 22 | -4.535381 | -8.41597 | -4.958029 |
|---|---|---|---|---|---|---|
| 710 | SERPING1 | SERPING1 | serpin peptidase inhibitor, clade G (C1 inhibitor), member 1 | -4.662694 | -6.192108 | -4.24183 |
| 10628 | TXNIP | TXNIP | thioredoxin interacting protein | -1.692751 | -4.683998 | -9.926255 |

| | | | | |
|---|---|---|---|---|
| 1.2797E-06 | 2.09E-02 | 0.00043947 | 0.71103945 | <1E-16 |
| 0.61665 | 0.3352 | 0.05689 | 0.75945884 | <1E-16 |
| 0.0034315 | 0.82459 | 0.0031478 | 1.2740818 | <1E-16 |

| 6A: Top 3-gene combinations from the decision-tree learning model with less than 25% cross validation error | | | | |
|---|---|---|---|---|
| Gene 1 | Gene 2 | Gene 3 | Cross validation error | Resubstitution error |
| CDKN1A | FGFR1 | PMP22 | 0.218182 | 0.218182 |
| B2M | CDKN1A | FGFR1 | 0.218182 | 0.218182 |

(continued)

| 6A: Top 3-gene combinations from the decision-tree learning model with less than 25% cross validation error | | | | |
|---|---|---|---|---|
| Gene 1 | Gene 2 | Gene 3 | Cross validation error | Resubstitution error |
| CTSH | FGFR1 | PMP22 | 0.2 | 0.218182 |
| FGFR1 | PMP22 | SERPING1 | 0.2 | 0.181818 |
| CLU | CTSH | PMP22 | 0.218182 | 0.2 |
| CTSH | GPX3 | PMP22 | 0.218182 | 0.2 |
| CLIC4 | LGALS3 | SERPING1 | 0.236364 | 0.181818 |
| CLIC4 | CLU | LGALS3 | 0.254545 | 0.218182 |
| CLIC4 | CTSH | LGALS3 | 0.254545 | 0.218182 |
| CLU | IGF1 | PMP22 | 0.254545 | 0.218182 |
| FGFR1 | LGALS3 | NFE2L2 | 0.254545 | 0.218182 |
| FGFR1 | NFE2L2 | PMP22 | 0.254545 | 0.218182 |
| CX3CL1 | FGFR1 | NFE2L2 | 0.254545 | 0.2 |
| FGFR1 | LGALS3 | MSN | 0.254545 | 0.181818 |

**Claims**

1. A method comprising

(a) determining a level of expression of a mRNA or protein encoded by each of three prognostic genes selected from the group consisting of *FGFR1, PMP22,* and *CDKN1A* in a test sample of prostate cancer obtained from a subject having prostate cancer, as compared to the level of expression in a control sample of benign noncancerous prostate tissue obtained from the subject or from a normal subject, and
(b) if the level of expression of the mRNA or protein or both is the same or higher than the corresponding level in the control sample, then determining that the prostate cancer is indolent, and

if there is about a two-fold or greater decrease in the level of expression of the mRNA or protein compared to the control sample, then determining that the prostate cancer is at high risk of progressing to an aggressive form.

2. A method comprising

(a) determining a level of expression of a mRNA or protein or both encoded by each of three prognostic genes selected from the group consisting of FGFR1, PMP22, and CDKN1A in a first and a second samples obtained from the subject having prostate cancer at the respective first and second time points,
(b) comparing the expression levels of the mRNA or protein at the first time point to the expression levels at the second time point, and
(c) determining that the prostate cancer is not progressing to an aggressive form if the level of expression of the prognostic mRNA or the protein or both at the second time point is the same or greater than at the first time point, and
(d) determining that the prostate cancer is at a high risk of progressing toward an aggressive form if there is about a two-fold or greater decrease in the level of expression of the prognostic mRNA or a protein at the second time point compared to the levels at the first time point.

3. The method of claims 1 or 2, wherein the prostate cancer has a Gleason score of 7 or less.

4. The method of claim 1 or claim 2, wherein the mRNA in the nucleic acid sample is amplified.

5. The method of claim 1 or claim 2, wherein determining expression level of a prognostic protein comprises immunohistochemistry using one or more antibodies or fragments thereof that specifically binds to the proteins or Western Blot.

6. The method of claim 1 or claim 2, wherein determining the level of *mRNA* expression is performed by qRT-PCR.

7. The method of claim 1 or claim 2, wherein the biological sample is blood, plasma, urine or cerebrospinal fluid.

8. Use of a diagnostic kit in a method according to any preceding claim, the kit comprising oligonucleotides that specifically hybridize to each of the respective mRNAs or one or more agents that specifically bind to each of the respective proteins, or both, the kit optionally comprising a forward primer and a reverse primer specific for each mRNA encoded by each of the prognostic genes for use in a qRT-PCR assay to specifically quantify the expression level of each mRNA.

9. Use of the diagnostic kit in a method according to claim 8, wherein the agents comprise one or more antibodies or antibody fragments that specifically bind to each of the respective proteins.

10. Use of the kit in a method according to claim 8, further comprising a forward primer and a reverse primer specific for each mRNA encoded by each of the prognostic genes for using a qRT-PCR assay to specifically quantify the expression level of each mRNA.

11. Use of the kit in a method according to claim 8, further comprising a reagent for isolating mRNA.

12. Use of a microarray in a method according to any of claims 1 - 7, the microarray comprising a plurality of oligonucleotides, such as cDNAs, that specifically hybridize to an mRNA encoded by each of three prognostic genes selected from the group consisting of FGFR1, PMP22, and CDKN1A, which cDNAs or oligonucleotides are fixed on the microarray, optionally wherein the oligonucleotides are labeled to facilitate detection of hybridization to the mRNAs, such as wherein the oligonucleotides may be radio-labeled, or biotin-labeled, and/or wherein the antibody or antibody fragment may be radio-labeled, chromophore-labeled, fluorophore-labeled, or enzyme-labeled.

13. Use of a microarray in a method according to any of claims 1 - 7, the microarray comprising a plurality of antibodies or antibody fragments that specifically bind to a prognostic protein or variant or fragment thereof encoded by each of three prognostic genes selected from the group consisting of *FGFR1*, *PMP22*, and *CDKN1A*, which antibodies or antibody fragments are fixed on the microarray, optionally wherein the antibodies or antibody fragments may be labeled to facilitate detection of hybridization to the mRNAs, such as wherein the antibodies or antibody fragments may be radio-labeled, biotin-labeled, chromophore-labeled, fluorophore-labeled, or enzyme-labeled.

14. Use of an immunoassay kit in a method according to any of claims 1 - 7, wherein the immunoassay kit comprises a plurality of antibodies or antibody fragments that specifically bind to prognostic proteins encoded by each of three prognostic genes selected from the group consisting of *FGFR1*, *PMP22*, and *CDKN1A.*

**Patentansprüche**

1. Verfahren, umfassend

(a) Feststellen eines Expressionsniveaus einer mRNA oder eines Proteins, codiert von jedem von drei prognostischen Genen, ausgewählt aus der Gruppe, bestehend aus *FGFR1, PMP22* und *CDKN1A,* in einer Testprobe von Prostatakrebs, erhalten von einem Individuum mit Prostatakrebs, gegenüber dem Expressionsniveau in einer Kontrollprobe von gutartigem nichtkanzerösem Prostatagewebe, erhalten von dem Individuum oder von einem normalen Individuum, und
(b) falls das Expressionsniveau der mRNA oder des Proteins oder beider das gleiche wie oder höher als das entsprechende Niveau in der Kontrollprobe ist, dann Feststellen, dass der Prostatakrebs indolent ist, und

falls ungefähr ein zweifacher oder größerer Rückgang des Expressionsniveaus der mRNA oder des Proteins gegenüber der Kontrollprobe vorliegt, dann Feststellen, dass der Prostatakrebs ein hohes Risiko birgt, zu einer aggressiven Form fortzuschreiten.

2. Verfahren, umfassend

(a) Feststellen eines Expressionsniveaus einer mRNA oder eines Proteins oder beider, codiert von jedem von drei prognostischen Genen, ausgewählt aus der Gruppe, bestehend aus *FGFR1, PMP22* und *CDKN1A,* in einer ersten und einer zweiten Probe, erhalten vom Individuum mit Prostatakrebs zu dem jeweiligen ersten und zweiten Zeitpunkt,

(b) Vergleichen der Expressionsniveaus der mRNA oder des Proteins zum ersten Zeitpunkt mit den Expressionsniveaus zum zweiten Zeitpunkt, und

(c) Feststellen, dass der Prostatakrebs nicht zu einer aggressiven Form fortschreitet, falls das Expressionsniveau der prognostischen mRNA oder des Proteins oder beider zum zweiten Zeitpunkt das gleiche wie oder größer als zum ersten Zeitpunkt ist, und

(d) Feststellen, dass der Prostatakrebs ein hohes Risiko birgt, zu einer aggressiven Form fortzuschreiten, falls ein ungefähr zweifacher oder größerer Rückgang des Expressionsniveaus der prognostischen mRNA oder eines Proteins zum zweiten Zeitpunkt gegenüber den Niveaus zum ersten Zeitpunkt vorliegt.

3. Verfahren nach Anspruch 1 oder Anspruch 2, wobei der Prostatakrebs einen Gleason-Score von 7 oder weniger aufweist.

4. Verfahren nach Anspruch 1 oder Anspruch 2, wobei die mRNA in der Nukleinsäureprobe amplifiziert wird.

5. Verfahren nach Anspruch 1 oder Anspruch 2, wobei das Feststellen des Expressionsniveaus eines prognostischen Proteins Immunhistochemie unter Verwendung eines oder mehrerer Antikörper oder Fragmente davon, die spezifisch an die Proteine binden, oder Western Blot umfasst.

6. Verfahren nach Anspruch 1 oder Anspruch 2, wobei das Feststellen des *m*RNA-Expressionsniveaus mittels qRT-PCR durchgeführt wird.

7. Verfahren nach Anspruch 1 oder Anspruch 2, wobei die biologische Probe Blut, Plasma, Urin oder Gehirn-Rückenmark-Flüssigkeit ist.

8. Verwendung eines diagnostischen Kits in einem Verfahren nach einem der vorstehenden Ansprüche, das Kit umfassend Oligonukleotide, die spezifisch an jede der jeweiligen mRNAs hybridisieren, oder ein oder mehrere Mittel, die spezifisch an jedes der jeweiligen Proteine binden, oder beides, das Kit gegebenenfalls umfassend einen Forward-Primer und einen Reverse-Primer, die für jede mRNA, codiert von jedem der prognostischen Gene, spezifisch sind, zwecks Verwendung in einem qRT-PCR-Assay, um das Expressionsniveau jeder *m*RNA spezifisch zu quantifizieren.

9. Verwendung des diagnostischen Kits in einem Verfahren nach Anspruch 8, wobei die Mittel einen oder mehrere Antikörper oder Antikörperfragmente umfassen, die spezifisch an jedes der jeweiligen Proteine binden.

10. Verwendung des Kits in einem Verfahren nach Anspruch 8, weiterhin umfassend einen Forward-Primer und einen Reverse-Primer, die für jede mRNA, codiert von jedem der prognostischen Gene, spezifisch sind, zwecks Verwendung eines qRT-PCR-Assays, um das Expressionsniveau jeder mRNA spezifisch zu quantifizieren.

11. Verwendung des Kits in einem Verfahren nach Anspruch 8, weiterhin umfassend ein Reagens zum Isolieren von mRNA.

12. Verwendung eines Mikroarrays in einem Verfahren nach einem der Ansprüche 1-7, wobei der Mikroarray mehrere Oligonukleotide, wie z. B. cDNAs, umfasst, die spezifisch an eine mRNA hybridisieren, codiert von jedem von drei prognostischen Genen, ausgewählt aus der Gruppe, bestehend aus *FGFR1, PMP22* und *CDKN1A,* welche cDNAs oder Oligonukleotide auf dem Mikroarray fixiert sind, wobei die Oligonukleotide gegebenenfalls markiert sind, um einen Nachweis der Hybridisierung an die mRNAs zu erleichtern, beispielsweise, wobei die Oligonukleotide radioaktiv markiert oder biotinmarkiert sein können, und/oder wobei der Antikörper oder das Antikörperfragment radioaktiv markiert, chromophormarkiert, fluorophormarkiert oder enzymmarkiert sein kann.

13. Verwendung eines Mikroarrays in einem Verfahren nach einem der Ansprüche 1-7, wobei der Mikroarray mehrere Antikörper oder Antikörperfragmente umfasst, die spezifisch an ein prognostisches Protein oder eine Variante oder ein Fragment davon binden, codiert von jedem von drei prognostischen Genen, ausgewählt aus der Gruppe, bestehend aus *FGFR1, PMP22* und *CDKN1A,* welche Antikörper oder Antikörperfragmente auf dem Mikroarray fixiert sind, wobei die Antikörper oder Antikörperfragmente gegebenenfalls markiert sein können, um einen Nachweis der Hybridisierung an die mRNAs zu erleichtern, beispielsweise, wobei die Antikörper oder Antikörperfragmente radioaktiv markiert, biotinmarkiert, chromophormarkiert, fluorophormarkiert oder enzymmarkiert sein können.

14. Verwendung eines Immunassay-Kits in einem Verfahren nach einem der Ansprüche 1-7, wobei das Immunassay-Kit mehrere Antikörper oder Antikörperfragmente umfasst, die spezifisch an prognostische Proteine binden, codiert von jedem von drei prognostischen Genen, ausgewählt aus der Gruppe, bestehend aus *FGFR1, PMP22* und *CDKN1A*.

**Revendications**

1. Méthode comprenant:

   (a) la détermination d'un niveau d'expression d'un ARNm ou d'une protéine codés par chacun de trois gènes pronostiques choisis dans le groupe constitué de *FGFR1, PMP22* et *CDKN1A* dans un échantillon test de cancer de la prostate obtenu à partir d'un sujet ayant un cancer de la prostate, lorsqu'il est comparé au niveau d'expression dans un échantillon témoin de tissu de prostate non cancéreux bénin obtenu à partir du sujet ou à partir d'un sujet normal, et
   (b) si le niveau d'expression de l'ARNm ou de la protéine ou des deux est supérieur ou égal au niveau correspondant dans l'échantillon témoin, alors la détermination que le cancer de la prostate est indolent, et

   s'il y a une diminution d'environ deux fois ou plus dans le niveau d'expression de l'ARNm ou de la protéine comparé à l'échantillon témoin, alors la détermination que le cancer de la prostate est à haut risque de progresser vers une forme agressive.

2. Méthode comprenant:

   (a) la détermination d'un niveau d'expression d'un ARNm ou d'une protéine ou des deux codés par chacun de trois gènes pronostiques choisis dans le groupe constitué de FGFR1, PMP22 et CDKN1A dans un premier et un deuxième échantillons obtenus à partir du sujet ayant un cancer de la prostate aux premier et deuxième points temporels respectifs,
   (b) la comparaison des niveaux d'expression de l'ARNm ou de la protéine au premier point temporel avec les niveaux d'expression au deuxième point temporel, et
   (c) la détermination que le cancer de la prostate ne progresse pas vers une forme agressive si le niveau d'expression de l'ARNm ou de la protéine pronostique ou des deux au deuxième point temporel est supérieur ou égal à celui au premier point temporel, et
   (d) la détermination que le cancer de la prostate est à haut risque de progresser vers une forme agressive s'il y a une diminution environ deux fois ou plus dans le niveau d'expression de l'ARNm ou de la protéine pronostique au deuxième point temporel comparé aux niveaux au premier point temporel.

3. Méthode selon les revendications 1 ou 2, dans laquelle le cancer de la prostate a un score Gleason de 7 ou moins.

4. Méthode selon la revendication 1 ou la revendication 2, dans laquelle l'ARNm dans l'échantillon d'acide nucléique est amplifié.

5. Méthode selon la revendication 1 ou la revendication 2, dans laquelle la détermination du niveau d'expression d'une protéine pronostique comprend une immunocytochimie utilisant un ou plusieurs anticorps ou fragments de ceux-ci qui se lient spécifiquement aux protéines ou un transfert de type Western.

6. Méthode selon la revendication 1 ou la revendication 2, dans laquelle la détermination du niveau d'expression d'ARNm est effectuée par qRT-PCR.

7. Méthode selon la revendication 1 ou la revendication 2, dans laquelle l'échantillon biologique est un sang, un plasma, une urine ou un fluide cérébrospinal.

8. Utilisation d'un kit diagnostique dans une méthode selon l'une quelconque des revendications précédentes, le kit comprenant des oligonucléotides qui s'hybrident spécifiquement à chacun des ARNms respectifs ou un ou plusieurs agents qui se lient spécifiquement à chacune des protéines respectives, ou les deux, le kit comprenant optionnellement une amorce sens et une amorce anti-sens spécifiques à chaque ARNm codé par chacun des gènes pronostiques pour une utilisation dans un essai qRT-PCR pour quantifier spécifiquement le niveau d'expression de chaque ARNm.

9. Utilisation du kit diagnostique dans une méthode selon la revendication 8, dans laquelle les agents comprennent un ou plusieurs anticorps ou fragments d'anticorps qui se lient spécifiquement à chacune des protéines respectives.

10. Utilisation du kit dans une méthode selon la revendication 8, comprenant en outre une amorce sens et une amorce anti-sens spécifiques à chaque ARNm codé par chacun des gènes pronostiques pour une utilisation d'un essai qRT-PCR pour quantifier spécifiquement le niveau d'expression de chaque ARNm.

11. Utilisation du kit dans une méthode selon la revendication 8, comprenant en outre un réactif pour isoler un ARNm.

12. Utilisation d'un micro-réseau dans une méthode selon l'une quelconque des revendications 1-7, le micro-réseau comprenant une pluralité d'oligonucléotides, tels que des ADNcs, qui s'hybrident spécifiquement à un ARNm codé par chacun des trois gènes pronostiques choisis dans le groupe constitué de FGFR1, PMP22 et CDKN1A, lesquels ADNcs ou oligonucléotides sont fixés sur le micro-réseau, optionnellement dans laquelle les oligonucléotides sont marqués pour faciliter la détection de l'hybridation aux ARNms, comme dans laquelle les oligonucléotides peuvent être marqués par marqueur radioactif ou marqués par biotine et/ou dans laquelle l'anticorps ou le fragment d'anticorps peut être marqué par marqueur radioactif, marqué par chromophore, marqué par fluorophore ou marqué par enzyme.

13. Utilisation d'un micro-réseau dans une méthode selon l'une quelconque des revendications 1-7, le micro-réseau comprenant une pluralité d'anticorps ou de fragments d'anticorps qui se lient spécifiquement à une protéine pronostique ou un variant ou un fragment de celle-ci codée par chacun des trois gènes pronostiques choisis dans le groupe constitué de *FGFR1, PMP22* et *CDKN1A,* lesquels anticorps ou fragments d'anticorps sont fixés sur le micro-réseau, optionnellement dans laquelle les anticorps ou les fragments d'anticorps peuvent être marqués pour faciliter la détection de l'hybridation aux ARNms, comme dans laquelle les anticorps ou les fragments d'anticorps peuvent être marqués par marqueur radioactif, marqués par biotine, marqués par chromophore, marqués par fluorophore ou marqués par enzyme.

14. Utilisation d'un kit d'immuno-essai dans une méthode selon l'une quelconque des revendications 1-7, dans laquelle le kit d'immuno-essai comprend une pluralité d'anticorps ou de fragments d'anticorps qui se lient spécifiquement aux protéines pronostiques codées par chacun des trois gènes pronostiques choisis dans le groupe constitué de *FGFR1, PMP22* et *CDKN1A.*

| STUDY DESIGN | | |
|---|---|---|
| **METHOD/MODEL** | **DATASET(S)/COHORT(S)** | **RESULT** |
| **STEP 1: MOLECULAR SIGNATURE OF AGING AND SENESCENCE** | | |
| CURATION OF AGING AND SENESCENCE SIGNATURE | META-ANALYSES OF AGING GENES<br>INGENUITY-BASED ANALYSIS OF SENESCENCE<br>MANUAL CURATION | 377-GENE SIGNATURE |
| **STEP 2: IDENTIFICATION OF INDOLENCE SIGNATURE** | | |
| GSEA ANALYSES ON AGGRESSIVE HUMAN PROSTATE TUMORS (LAGGING EDGE) | YU DATASET (TRAINING SET) | |
| META-ANALYSES WITH OTHER AGGRESSIVE HUMAN TUMORS (LAGGING EDGE) | BREAST AND LUNG DATASETS (TRAINING SET) | INTERSECTION OF LEADING AND LAGGING EDGE = 19 GENE INDOLENCE SIGNATURE |
| CROSS-SPECIES ANALYSES W/ MOUSE INDOLENT LESIONS (LEADING EDGE) | OUYANG DATASET (TRAINING SET) | |
| VALIDATION | TAYLOR DATASET (TEST SET)<br>GLEASON GRADE 8,9 (AGGRESSIVE)<br>GLEASON GRADE 6 AND 7(3+4) TUMORS (LOW GLEASON SCORE) | |
| **STEP 3: IDENTIFICATION OF PROGNOSTIC BIOMARKERS** | | |
| DECISION TREE LEARNING ALGORITHM W/ 19 - GENE INDOLENCE SIGNATURE | SBONER - SWEDISH COHORT (TRAINING SET) | 3-GENE PROGNOSTIC PANEL |
| **STEP 4: VALIDATION OF 3-GENE PANEL ON LOW GLEASON SCORE PRIMARY TUMORS** | | |
| CONFUSION MATRIX<br>KAPLAN-MEIER<br>C - STATICAL ANALYSES<br>COX PROPORTIONAL HAZARD MODEL | SBONER - SWEDISH COHORT (TEST SET)<br>RNA - TAYLOR DATASET-GLEASON GRADE 6 AND 7(3+4) (TEST SET)<br>PROTEIN-HICCC TISSUE MICROARRAY | VALIDATION OF 3-GENE PANEL |
| **STEP 5: VALIDATION OF 3-GENE PANEL ON BIOPSY SAMPLES** | | |
| IMMUNOSTAINING | RETROSPECTIVE SURVEILLENCE COHORT -GLEASON GRADE 6 | DIAGNOSTIC POTENTIAL OF 3-GENE PANEL |

FIG. 1

FIG. 2A

FIG. 2B

FIG. 2C

FIG. 2D

FIG. 2E

LOW GLEASON SCORE PROSTATE CANCER (GLEASON 6 AND 7(3+4))

FIG. 2F

FIG. 3A

**PREDICTIVE ACCURACY / KAPLAN MEIER**

| RANKED 3-GENE COMBINATIONS | (ODDS RATIO) SBONER ET AL. | (GLEASON 6 &3 +4) TAYLOR ET AL. |
|---|---|---|
| FGFR1/PMP22/CDKN1A | 1.94 | p=0.005 |
| CTSH/FGFR1/PMP22 | 1.25 | p=0.99 |
| FGFR1/PMP22/SERPING1 | 1.30 | p=0.42 |
| FGFR1/CDKN1A/B2M | 1.50 | p=0.02 |
| CLU/CTSH/PMP22 | 1.33 | p=0.81 |
| CTSH/GPX3/PMP22 | 1.25 | p=0.90 |

FIG. 3B

FIG. 3C

**C-STATISTICS MODEL- TAYLOR DATASET**

| | C INDEX | CONFIDENCE INTERVAL | p VALUE |
|---|---|---|---|
| D'AMICO CLASSIFICATION | 0.72 | 0.52-0.90 | 0.012 |
| GLEASON | 0.82 | 0.54-1.00 | 0.010 |
| FGFR1/PMP22/CDKN1A | 0.86 | 0.65-1.00 | $3.3 \times 10\text{-}4$ |
| D'AMICO + FGFR1/PMP22/CDKN1A | 0.83 | 0.73-0.95 | $1.8 \times 10\text{-}9$ |
| GLEASON + FGFR1/PMP22/CDKN1A | 0.89 | 0.74-1.00 | $4.7 \times 10\text{-}8$ |

FIG. 3D

**COX PROPORTIONAL HAZARD MODEL-TAYLOR DATASET**

| | LOG (LIKELIHOOD) | Chi-Sq | DF | p VALUE |
|---|---|---|---|---|
| GLEASON | -34.6 | | | |
| GLEASON + FGFR1/PMP22/CDKN1A | -31.5 | 6.33 | 2 | 0.042 |
| D'AMICO | -32.81 | | | |
| D'AMICO + FGFR1/PMP22/CDKN1A (+) | -29.97 | 5.67 | 1 | 0.017 |
| D'AMICO + FGFR1/PMP22/CDKN1A (*) | -29.02 | 7.57 | 4 | 0.110 |
| NOTE: (+), ADDITIVE MODEL; (*), INTERACTION MODEL | | | | |

FIG. 3E

FIG. 4A

FIG. 4B

### C-STATISTICS MODEL - HICCC TMA

|  | C INDEX | CONFIDENCE INTERVAL | p VALUE |
|---|---|---|---|
| GLEASON | 0.62 | 0.34 - 0.89 | 0.198 |
| FGFR1/PMP22/CDKN1A | 0.95 | 0.90 - 1.00 | 2 x 10-54 |
| GLEASON+ FGFR1/PMP22/CDKN1A | 0.82 | 0.70 - 0.94 | 1 x 10-7 |

### COX PROPORTIONAL HAZARD MODEL - HICCC TMA

|  | LOG (LIKELIHOOD) | Chi-Sq | DF | p VALUE |
|---|---|---|---|---|
| GLEASON | -40.9 |  |  |  |
| GLEASON+ FGFR1/PMP22/CDKN1A | -37.5 | 6.74 | 2 | 0.034 |

FIG. 4C

FIG. 4D

### BIOPSY SAMPLES FROM GLEASON 6 PATIENTS ON SURVEILLANCE

|  | COMPOSITE SCORE [SD] | [CI] |
|---|---|---|
| NON-FAILURES | 4.11 [1.0] | [3.59-4.63] |
| FAILURES | 1.71 [1.2] | [1.03-2.39] |
|  | P=1.54 x 10-5 |  |

FIG. 4E

FIG. 5A

FIG. 5B

FIG. 5B
CONTINUED

Nkx3.1^{+/+}
15 mths

Nkx3.1^{-/-}
4 mths    15 mths    23 mths

H&E

FIG. 6A    FIG. 6B    FIG. 6C    FIG. 6D

SAβ-gal

FIG. 6E    FIG. 6F    FIG. 6G    FIG. 6H

FIG. 6I

FIG. 6J

FIG. 7A

FIG. 7B

FIG. 7C

FGFR1/PMP22/CDKN1A

| ACTUAL | PREDICTED | |
|---|---|---|
| | INDOLENT | LETHAL |
| INDOLENT | 11 | 17 |
| LETHAL | 2 | 6 |

ODDS RATIO=1.94

CTSH/FGFR1/PMP22

| ACTUAL | PREDICTED | |
|---|---|---|
| | INDOLENT | LETHAL |
| INDOLENT | 12 | 16 |
| LETHAL | 3 | 5 |

ODDS RATIO=1.25

FGFR1/PMP22/SERPING1

| ACTUAL | PREDICTED | |
|---|---|---|
| | INDOLENT | LETHAL |
| INDOLENT | 16 | 12 |
| LETHAL | 2 | 4 |

ODDS RATIO=1.30

FGFR1/CDKN1A/B2M

| ACTUAL | PREDICTED | |
|---|---|---|
| | INDOLENT | LETHAL |
| INDOLENT | 20 | 8 |
| LETHAL | 5 | 3 |

ODDS RATIO=1.50

CLU/CTSH/PMP22

| ACTUAL | PREDICTED | |
|---|---|---|
| | INDOLENT | LETHAL |
| INDOLENT | 16 | 12 |
| LETHAL | 4 | 4 |

ODDS RATIO=1.33

CTSH/GPX3/PMP22

| ACTUAL | PREDICTED | |
|---|---|---|
| | INDOLENT | LETHAL |
| INDOLENT | 12 | 16 |
| LETHAL | 4 | 4 |

ODDS RATIO=1.25

# FIG. 8

FIG. 9A

FIG. 9B

FIG. 9C

TAYLOR ET AL. FGFR1

PMP22

CDNK1A

GLEASON 6 AND 3 + 4 (N = 95)
*P* VALUE = 0.34

GLEASON 6 AND 3 + 4 (N = 95)
*P* VALUE = 0.56

GLEASON 6 AND 3 + 4 (N = 95)
*P* VALUE = 0.02

## FIG. 10A

— LOW RISK
- - - HIGH RISK
+ CENSORED

HICCC TMA

FGFR1

PMP22

CDKN1A

GLEASON 6 AND 3 + 4 (N = 44)
*P* VALUE = 0.03

GLEASON 6 AND 3 + 4 (N = 44)
*P* VALUE = 0.53

GLEASON 6 AND 3 + 4 (N = 44)
*P* VALUE = 0.25

## FIG. 10B

BIOCHEMICAL RECURRENCE-FREE SURVIVAL

TIME (MONTHS)

FIG. 11A

FIG. 11B

FIG. 12

| Gene Name | Entrezgene [Homo sapiens] | Nucleotide Accession [Homo sapiens] | Nucleotide G [Homo sapiens] | Nucleotide Brief Description [Homo sapiens] |
|---|---|---|---|---|
| FGFR1 | 2260 | NM_001174067.1<br>NM_001174066.1<br>NM_001174065.1<br>NM_001174064.1<br>NM_001174063.1<br>NM_023110.2<br>NM_023105.2<br>NM_023106.2<br>NM_015850.3 | 291327496<br>291327494<br>291327492<br>291327490<br>291327488<br>105990521<br>105990517<br>105990516<br>105990515 | Transcript variant 14, mRNA [Homo sapiens]<br>Transcript variant 13, mRNA [Homo sapiens]<br>Transcript variant 12, mRNA [Homo sapiens]<br>Transcript variant 11, mRNA [Homo sapiens]<br>Transcript variant 10, mRNA [Homo sapiens]<br>Transcript variant 1, mRNA [Homo sapiens]<br>Transcript variant 3, mRNA [Homo sapiens]<br>Transcript variant 4, mRNA [Homo sapiens]<br>Transcript variant 2, mRNA [Homo sapiens] |
| PMP22 | 5376 | NM_153322.1<br>NM_153321.1<br>NM_000304.2 | 24430164<br>24430162<br>24430161 | Transcript variant 3, mRNA [Homo sapiens]<br>Transcript variant 2, mRNA [Homo sapiens]<br>Transcript variant 1, mRNA [Homo sapiens] |
| CDKN1A | 1026 | NM_001220778.1<br>NM_001220777.1<br>NM_078467.2<br>NM_000389.4 | 334085241<br>334085239<br>310832423<br>310832422 | Transcript variant 4, mRNA [Homo sapiens]<br>Transcript variant 5, mRNA [Homo sapiens]<br>Transcript variant 2, mRNA [Homo sapiens]<br>transcript variant 1, mRNA [Homo sapiens] |
| Gene Name | Entrezgene [Mus musculus] | Nucleotide Accession [Mus musculus] | Nucleotide GI [Mus musculus] | Nucleotide Brief Description [Mus musculus] |
| FGFR1 | 14182 | NM_001079908.1<br>NM_010206.2<br>NM_001079909.1 | 120952697<br>120952640<br>120952632 | transcript variant 2, mRNA [Mus musculus]<br>transcript variant 1, mRNA [Mus musculus]<br>transcript variant 3, mRNA [Mus musculus] |
| PMP22 | 18858 | NM_008885.2 | 118130612 | peripheral myelin protein 22 (Pmp22), mRNA [Mus musculus ] |
| CDKN1A | 12575 | NM_007669.4<br>NM_001111099.1 | 161760647<br>162287332 | cyclin-dependent kinase inhibitor 1A (P21) (Cdkn1a), transcript<br>cyclin-dependent kinase inhibitor 1A (P21) (Cdkn1a), transcript |

## FIG. 13

| | Protein Accession [Homo sapiens] | Protein GI [Homo sapiens] | Protein Brief Description [Homo sapiens] |
|---|---|---|---|
| | NP_001167536.1 | 291327493 | Isoform 2 precursor [Homo sapiens] |
| | NP_075598.2 | 105990522 | isoform 1 precursor [Homo sapiens] |
| | NP_056934.2 | 13186251 | isoform 2 precursor [Homo sapiens] |
| | NP_001167537.1 | 291327495 | isoform 3 precursor [Homo sapiens] |
| | NP_001167535.1 | 291327491 | isoform 11 precursor [Homo sapiens] |
| | NP_001167538.1 | 291327497 | isoform 14 precursor [Homo sapiens] |
| | NP_001167534.1 | 291327489 | isoform 10 precursor [Homo sapiens] |
| | NP_075594.1 | 13186236 | isoform 4 precursor [Homo sapiens] |
| | NP_075593.1 | 13186234 | isoform 3 precursor [Homo sapiens] |
| | NP_696996.1 | 24430163 | peripheral myelin protein 22 [Homo sapiens] |
| | NP_696997.1 | 24430165 | peripheral myelin protein 22 [Homo sapiens] |
| | NP_000295.1 | 4505907 | peripheral myelin protein 22 [Homo sapiens] |
| | NP_000380.1 | 11386203 | cyclin-dependent kinase inhibitor 1 [Homo sapiens] |
| | NP_510867.1 | 17978495 | cyclin-dependent kinase inhibitor 1 [Homo sapiens] |
| | NP_001207706.1 | 334085240 | cyclin-dependent kinase inhibitor 1 [Homo sapiens] |
| | NP_001207707.1 | 334085242 | cyclin-dependent kinase inhibitor 1 [Homo sapiens] |
| | Protein Accession [Mus musculus] | Protein GI [Mus musculus] | Protein Brief Description [Mus musculus] |
| | NP_001073377.1 | 120952698 | isoform 2 precursor [Mus musculus] |
| | NP_034336.2 | 120952641 | isoform 1 precursor [Mus musculus] |
| | NP_001073378.1 | 120952633 | isoform 3 precursor [Mus musculus] |
| | NP_032911.1 | 6679395 | peripheral myelin protein 22 [Mus musculus] |
| variant 1, mRNA[Mus m | NP_031695.1 | 6671726 | cyclin-dependent kinase inhibitor 1 [Homo sapiens] |
| variant 2, mRNA | NP_001104569.1 | 162287333 | cyclin-dependent kinase inhibitor 1 [Homo sapiens] |
| | | | cyclin-dependent kinase inhibitor 1 [Homo sapiens] |
| | | | cyclin-dependent kinase inhibitor 1 [Homo sapiens] |

# FIG. 13
CONTINUED

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2011082198 A
- WO 2006110264 A
- WO 03053223 A
- US 5631171 A
- US 5955377 A
- US 6019944 A

- US 6225047 B
- WO 9951773 A
- US 6329209 B
- WO 0056934 A
- US 5242828 A

### Non-patent literature cited in the description

- **L. A. MUCCI et al.** Testing a Multigene Signature of Prostate Cancer Death in the Swedish Watchful Waiting Cohort. *CANCER EPIDEMIOLOGY, BIOMARKERS & PREVENTION,* 01 July 2008, vol. 17 (7), 1682-1688
- **MARC OVERMYER.** Molecular predictor differentiates indolent, advanced prostate cancer. *Urology Times,* 01 May 2011, http://urologytimes.modernmedicine.com/urology-times/news/modernmedicine/modern-medicine-news/molecular-predictor-differentiates-indolent-a?page=full
- Expression of bFGF/FGFR-1 and vascular proliferation related to clinicopathologic features and tumor progress in localized prostate cancer. **KARSTEN GRAVDAL et al.** VIRCHOWS ARCHIV. SPRINGER, 01 January 2006, vol. 448, 68-74
- Identification of metastasis-associated genes in prostate cancer by genetic profiling of human prostate cancer cell lines. **TROJAN L et al.** ANTICANCER RESEARCH - INTERNATIONAL JOURNAL OF CANCER RESEARCH AND TREATMENT. INTERNATIONAL INSTITUTE OF ANTICANCER RESEARCH, vol. 25, 183-191
- Gene expression of PMP22 is an independent prognostic factor for disease-free and overall survival in breast cancer patients. **TONG DAN et al.** BMC CANCER. BIOMED CENTRAL, 15 December 2010, vol. 10, 682
- **SAMBROOK et al.** Molecular Cloning: A Laboratory Manual. Cold Spring Harbor Laboratory Press, 1989
- **AUSUBEL et al.** Current Protocols in Molecular Biology. Greene Publishing Associates, 1992
- **HARLOW ; LAN.** Antibodies: A Laboratory Manual. Cold Spring Harbor Laboratory Press, 1990
- Principles of Neural Science. McGraw-Hill, 2000

- **OUYANG X ; DEWEESE TL ; NELSON WG ; ABATE-SHEN C.** Loss-of-function of Nkx3.1 promotes increased oxidative damage in prostate carcinogenesis. *Cancer Res,* 2005, vol. 65, 6773-9
- **YU YP ; LANDSITTEL D ; JING L et al.** Gene expression alterations in prostate cancer predicting tumor aggression and preceding development of malignancy. *Journal of clinical oncology : official journal of the American Society of Clinical Oncology,* 2004, vol. 22, 2790-9
- **TAYLOR BS ; SCHULTZ N ; HIERONYMUS H et al.** Integrative genomic profiling of human prostate cancer. *Cancer cell,* 2010, vol. 18, 11-22
- **SBONER A ; DEMICHELIS F ; CALZA S et al.** Molecular sampling of prostate cancer: a dilemma for predicting disease progression. *BMC medical genomics,* 2010, vol. 3, 8
- **ROMERO ; ROTBART.** *Diagnostic Molecular Biology: Principles and Applications,* 401-406
- **BEAUCAGE, S. L. ; CARUTHERS, M. H.** *Tetrahedron Letts.,* 1981, vol. 22 (20), 1859-1862
- **NEEDHAM-VANDEVANTER, D. R. et al.** *Nucleic Acids Res.,* 1984, vol. 12, 6159-6168
- **PEARSON, J. D. ; REGNIER, F. E.** *J. Chrom.,* 1983, vol. 255, 137-149
- **NG ; ILAG.** *J. Cell Mol. Med.,* 2002, vol. 6, 329-340
- **M. R. COOPERBERG ; J. M. BROERING ; P. W. KANTOFF ; P. R. CARROLL.** Contemporary trends in low risk prostate cancer: risk assessment and treatment. *The Journal of urology,* 2007, vol. 178, S14
- **H. G. WELCH ; P. C. ALBERTSEN.** Prostate cancer diagnosis and treatment after the introduction of prostate-specific antigen screening: 1986-2005. *Journal of the National Cancer Institute,* 2009, vol. 101, 1325

- **J. R. PRENSNER ; M. A. RUBIN ; J. T. WEI ; A. M. CHINNAIYAN.** Beyond PSA: the next generation of prostate cancer biomarkers. *Science translational medicine,* 2012, vol. 4, 127rv3
- **D. F. GLEASON.** Histologic grading of prostate cancer: a perspective. *Human pathology,* 1992, vol. 23, 273
- **T. J. WILT ; R. MACDONALD ; I. RUTKS ; T. A. SHAMLIYAN ; B. C. TAYLOR ; R. L. KANE.** Systematic review: comparative effectiveness and harms of treatments for clinically localized prostate cancer. *Annals of internal medicine,* 2008, vol. 148, 435
- **T. J. DASKIVICH ; K. CHAMIE ; L. KWAN ; J. LABO ; R. PALVOLGYI ; A. DASH ; S. GREENFIELD ; M. S. LITWIN.** Overtreatment of men with low-risk prostate cancer and significant comorbidity. *Cancer,* 2011, vol. 117, 2058
- **H. G. WELCH ; W. C. BLACK.** Overdiagnosis in cancer. *Journal of the National Cancer Institute,* 2010, vol. 102, 605
- **B. B. CANTRELL ; D. P. DEKLERK ; J. C. EGGLESTON ; J. K. BOITNOTT ; P. C. WALSH.** Pathological factors that influence prognosis in stage A prostatic cancer: the influence of extent versus grade. *The Journal of urology,* 1981, vol. 125, 516
- **M. R. COOPERBERG ; P. R. CARROLL ; L. KLOTZ.** Active surveillance for prostate cancer: progress and promise. *Journal of clinical oncology : official journal of the American Society of Clinical Oncology,* 2011, vol. 29, 3669
- **J. H. HAYES ; D. A. OLLENDORF ; S. D. PEARSON ; M. J. BARRY ; P. W. KANTOFF ; S. T. STEWART ; V. BHATNAGAR ; C. J. SWEENEY ; J. E. STAHL ; P. M. MCMAHON.** Active surveillance compared with initial treatment for men with low-risk prostate cancer: a decision analysis. *JAMA : the journal of the American Medical Association,* 2010, vol. 304, 2373
- **J. J. TOSOIAN ; B. J. TROCK ; P. LANDIS ; Z. FENG ; J. I. EPSTEIN ; A. W. PARTIN ; P. C. WALSH ; H. B. CARTER.** Active surveillance program for prostate cancer: an update of the Johns Hopkins experience. *Journal of clinical oncology : official journal of the American Society of Clinical Oncology,* 2011, vol. 29, 2185
- **M. M. SHEN ; C. ABATE-SHEN.** Molecular genetics of prostate cancer: new prospects for old challenges. *Genes Dev,* 2010, vol. 24, 1967
- **B. S. TAYLOR ; N. SCHULTZ ; H. HIERONYMUS ; A. GOPALAN ; Y. XIAO ; B. S. CARVER ; V. K. ARORA ; P. KAUSHIK ; E. CERAMI ; B. REVA.** Integrative genomic profiling of human prostate cancer. *Cancer cell,* 2010, vol. 18, 11
- **M. NARITA ; S. W. LOWE.** Senescence comes of age. *Nature medicine,* 2005, vol. 11, 920
- **J. CAMPISI.** Senescent cells, tumor suppression, and organismal aging: good citizens, bad neighbors. *Cell,* 2005, vol. 120, 513
- **J. CAMPISI.** Aging, tumor suppression and cancer: high wire-act!. *Mechanisms of ageing and development,* 2005, vol. 126, 51
- **M. COLLADO ; M. A. BLASCO ; M. SERRANO.** Cellular senescence in cancer and aging. *Cell,* 2007, vol. 130, 223
- **J. CHOI ; I. SHENDRIK ; M. PEACOCKE ; D. PEEHL ; R. BUTTYAN ; E. F. IKEGUCHI ; A. E. KATZ ; M. C. BENSON.** Expression of senescence-associated beta-galactosidase in enlarged prostates from men with benign prostatic hyperplasia. *Urology,* 2000, vol. 56, 160
- **P. CASTRO ; D. GIRI ; D. LAMB ; M. ITTMANN.** Cellular senescence in the pathogenesis of benign prostatic hyperplasia. *The Prostate,* 2003, vol. 55, 30
- **Z. CHEN ; L. C. TROTMAN ; D. SHAFFER ; H. K. LIN ; Z. A. DOTAN ; M. NIKI ; J. A. KOUTCHER ; H. I. SCHER ; T. LUDWIG ; W. GERALD.** Crucial role of p53-dependent cellular senescence in suppression of Pten-deficient tumorigenesis. *Nature,* 2005, vol. 436, 725
- **J. P. DE MAGALHAES ; J. CURADO ; G. M. CHURCH.** Meta-analysis of age-related gene expression profiles identifies common signatures of aging. *Bioinformatics,* 2009, vol. 25, 875
- **P. WIRAPATI ; C. SOTIRIOU ; S. KUNKEL ; P. FARMER ; S. PRADERVAND ; B. HAIBE-KAINS ; C. DESMEDT ; M. IGNATIADIS ; T. SENGSTAG ; F. SCHUTZ.** Meta-analysis of gene expression profiles in breast cancer: toward a unified understanding of breast cancer subtyping and prognosis signatures. *Breast cancer research : BCR,* 2008, vol. 10, R65
- **J. CUZICK ; G. P. SWANSON ; G. FISHER ; A. R. BROTHMAN ; D. M. BERNEY ; J. E. REID ; D. MESHER ; V. O. SPEIGHTS ; E. STANKIEWICZ ; C. S. FOSTER.** Prognostic value of an RNA expression signature derived from cell cycle proliferation genes in patients with prostate cancer: a retrospective study. *The lancet oncology,* 2011, vol. 12, 245
- **Y. P. YU ; D. LANDSITTEL ; L. JING ; J. NELSON ; B. REN ; L. LIU ; C. MCDONALD ; R. THOMAS ; R. DHIR ; S. FINKELSTEIN.** Gene expression alterations in prostate cancer predicting tumor aggression and preceding development of malignancy. *Journal of clinical oncology : official journal of the American Society of Clinical Oncology,* 2004, vol. 22, 2790

- A. BHATTACHARJEE ; W. G. RICHARDS ; J. STAUNTON ; C. LI ; S. MONTI ; P. VASA ; C. LADD ; J. BEHESHTI ; R. BUENO ; M. GILLETTE. Classification of human lung carcinomas by mRNA expression profiling reveals distinct adenocarcinoma subclasses. *Proceedings of the National Academy of Sciences of the United States of America,* 2001, vol. 98, 13790
- Comprehensive molecular portraits of human breast tumours. *Nature,* 2012, vol. 490, 61
- R. BHATIA-GAUR ; A. A. DONJACOUR ; P. J. SCIAVOLINO ; M. KIM ; N. DESAI ; P. YOUNG ; C. R. NORTON ; T. GRIDLEY ; R. D. CARDIFF ; G. R. CUNHA. Roles for Nkx3.1 in prostate development and cancer. *Genes Dev,* 1999, vol. 13, 966
- M. J. KIM ; R. BHATIA-GAUR ; W. A. BANACH-PETROSKY ; N. DESAI ; Y. WANG ; S. W. HAYWARD ; G. R. CUNHA ; R. D. CARDIFF ; M. M. SHEN ; C. ABATE-SHEN. Nkx3.1 mutant mice recapitulate early stages of prostate carcinogenesis. *Cancer Res,* 2002, vol. 62, 2999
- C. ABATE-SHEN ; M. M. SHEN ; E. GELMANN. Integrating differentiation and cancer: the Nkx3.1 homeobox gene in prostate organogenesis and carcinogenesis. *Differentiation,* 2008, vol. 76, 717
- X. OUYANG ; T. L. DEWEESE ; W. G. NELSON ; C. ABATE-SHEN. Loss-of-function of Nkx3.1 promotes increased oxidative damage in prostate carcinogenesis. *Cancer Res,* 2005, vol. 65, 6773
- A. G. SOWALSKY ; H. YE ; G. J. BUBLEY ; S. P. BALK. Clonal progression of prostate cancers from Gleason grade 3 to grade 4. *Cancer Res,* 2013, vol. 73, 1050
- A. SBONER ; F. DEMICHELIS ; S. CALZA ; Y. PAWITAN ; S. R. SETLUR ; Y. HOSHIDA ; S. PERNER ; H. O. ADAMI ; K. FALL ; L. A. MUCCI. Molecular sampling of prostate cancer: a dilemma for predicting disease progression. *BMC medical genomics,* 2010, vol. 3, 8
- A. V. D'AMICO ; R. WHITTINGTON ; S. B. MALKOWICZ ; D. SCHULTZ ; K. BLANK ; G. A. BRODERICK ; J. E. TOMASZEWSKI ; A. A. RENSHAW ; I. KAPLAN ; C. J. BEARD. Biochemical outcome after radical prostatectomy, external beam radiation therapy, or interstitial radiation therapy for clinically localized prostate cancer. *JAMA : the journal of the American Medical Association,* 1998, vol. 280, 969
- P. MOTAMEDINIA ; J. L. RICHARD ; J. M. MCKIERNAN ; G. J. DECASTRO ; M. C. BENSON. Role of immediate confirmatory prostate biopsy to ensure accurate eligibility for active surveillance. *Urology,* 2012, vol. 80, 1070
- J. CAMPISI. Cancer and ageing: rival demons?. *Nature reviews. Cancer,* 2003, vol. 3, 339
- S. ROY ; R. P. SINGH ; C. AGARWAL ; S. SIRIWARDANA ; R. SCLAFANI ; R. AGARWAL. Downregulation of both p21/Cip1 and p27/Kip1 produces a more aggressive prostate cancer phenotype. *Cell Cycle,* 2008, vol. 7, 1828
- T. ABBAS ; A. DUTTA. p21 in cancer: intricate networks and multiple activities, Nature reviews. *Cancer,* 2009, vol. 9, 400
- V. D. ACEVEDO ; M. ITTMANN ; D. M. SPENCER. Paths of FGFR-driven tumorigenesis. *Cell Cycle,* 2009, vol. 8, 580
- N. TURNER ; R. GROSE. Fibroblast growth factor signalling: from development to cancer, Nature reviews. *Cancer,* 2010, vol. 10, 116
- F. YANG ; Y. ZHANG ; S. J. RESSLER ; M. M. ITTMANN ; G. E. AYALA ; T. D. DANG ; F. WANG ; D. R. ROWLEY. FGFR1 is Essential for Prostate Cancer Progression and Metastasis. *Cancer Res,* 2013
- J. EDWARDS ; N. S. KRISHNA ; C. J. WITTON ; J. M. BARTLETT. Gene amplifications associated with the development of hormone-resistant prostate cancer. *Clinical cancer research : an official journal of the American Association for Cancer Research,* 2003, vol. 9, 5271
- G. MEYER ; ZU HORSTE ; K. A. NAVE. Animal models of inherited neuropathies. *Current opinion in neurology,* 2006, vol. 19, 464
- K. ADLKOFER ; R. MARTINI ; A. AGUZZI ; J. ZIELASEK ; K. V. TOYKA ; U. SUTER. Hypermyelination and demyelinating peripheral neuropathy in Pmp22-deficient mice. *Nature genetics,* 1995, vol. 11, 274
- U. SUTER ; G. J. SNIPES. Peripheral myelin protein 22: facts and hypotheses. *Journal of neuroscience research,* 1995, vol. 40, 145
- Z. DING ; C. J. WU ; G. C. CHU ; Y. XIAO ; D. HO ; J. ZHANG ; S. R. PERRY ; E. S. LABROT ; X. WU ; R. LIS. SMAD4-dependent barrier constrains prostate cancer growth and metastatic progression. *Nature,* 2011, vol. 470, 269
- E. K. MARKERT ; H. MIZUNO ; A. VAZQUEZ ; A. J. LEVINE. Molecular classification of prostate cancer using curated expression signatures. *Proceedings of the National Academy of Sciences of the United States of America,* 2011, vol. 108, 21276
- S. A. TOMLINS ; S. M. AUBIN ; J. SIDDIQUI ; R. J. LONIGRO ; L. SEFTON-MILLER ; S. MIICK ; S. WILLIAMSEN ; P. HODGE ; J. MEINKE ; A. BLASE. Urine TMPRSS2:ERG fusion transcript stratifies prostate cancer risk in men with elevated serum PSA. *Science translational medicine,* 2011, vol. 3, 94ra72

- **D. OLMOS ; D. BREWER ; J. CLARK ; D. C. DANILA ; C. PARKER ; G. ATTARD ; M. FLEISHER ; A. H. REID ; E. CASTRO ; S. K. SANDHU.** Prognostic value of blood mRNA expression signatures in castration-resistant prostate cancer: a prospective, two-stage study. *The lancet oncology,* 2012, vol. 13, 1114
- **M. BRAIG ; S. LEE ; C. LODDENKEMPER ; C. RUDOLPH ; A. H. PETERS ; B. SCHLEGELBERGER ; H. STEIN ; B. DORKEN ; T. JENUWEIN ; C. A. SCHMITT.** Oncogene-induced senescence as an initial barrier in lymphoma development. *Nature,* 2005, vol. 436, 660
- **M. COLLADO ; J. GIL ; A. EFEYAN ; C. GUERRA ; A. J. SCHUHMACHER ; M. BARRADAS ; A. BENGURIA ; A. ZABALLOS ; J. M. FLORES ; M. BARBACID.** Tumour biology: senescence in premalignant tumours. *Nature,* 2005, vol. 436, 642
- **M. MALUMBRES ; I. PEREZ DE CASTRO ; M. I. HERNANDEZ ; M. JIMENEZ ; T. CORRAL ; A. PELLICER.** Cellular response to oncogenic ras involves induction of the Cdk4 and Cdk6 inhibitor p15(INK4b). *Mol Cell Biol,* 2000, vol. 20, 2915
- **A. SUBRAMANIAN ; P. TAMAYO ; V. K. MOOTHA ; S. MUKHERJEE ; B. L. EBERT ; M. A. GILLETTE ; A. PAULOVICH ; S. L. POMEROY ; T. R. GOLUB ; E. S. LANDER.** Gene set enrichment analysis: a knowledge-based approach for interpreting genome-wide expression profiles. *Proceedings of the National Academy of Sciences of the United States of America,* 2005, vol. 102, 15545
- **M. J. PENCINA ; R. B. D'AGOSTINO.** Overall C as a measure of discrimination in survival analysis: model specific population value and confidence interval estimation. *Statistics in medicine,* 2004, vol. 23, 2109
- **R. A. IRIZARRY ; B. HOBBS ; F. COLLIN ; Y. D. BEAZER-BARCLAY ; K. J. ANTONELLIS ; U. SCHERF ; T. P. SPEED.** Exploration, normalization, and summaries of high density oligonucleotide array probe level data. *Biostatistics,* 2003, vol. 4, 249
- **Z. WU ; R. IRIZARRY ; R. GENTLEMAN ; F. M. MURILLO ; F. SPENCER.** A Model Based Background Adjustment for Oligonucleotide Expression Arrays. Johns Hopkins University, 2004
- **V. K. MOOTHA ; C. M. LINDGREN ; K. F. ERIKSSON ; A. SUBRAMANIAN ; S. SIHAG ; J. LEHAR ; P. PUIGSERVER ; E. CARLSSON ; M. RIDDERSTRALE ; E. LAURILA.** PGC-1alpha-responsive genes involved in oxidative phosphorylation are coordinately downregulated in human diabetes. *Nature genetics,* 2003, vol. 34, 267
- **G. A. F. SEBER.** Multivariate Observations. John Wiley & Sons, Inc, 1984
- **C. W. KINKADE ; M. CASTILLO-MARTIN ; A. PUZIO-KUTER ; J. YAN ; T. H. FOSTER ; H. GAO ; Y. SUN ; X. OUYANG ; W. L. GERALD ; C. CORDON-CARDO.** Targeting AKT/mTOR and ERK MAPK signaling inhibits hormone-refractory prostate cancer in a preclinical mouse model. *The Journal of clinical investigation,* 2008, vol. 118, 3051